(19) **Europäisches Patentamt / European Patent Office / Office européen des brevets**

(11) **EP 4 173 638 A1**

(12) **EUROPEAN PATENT APPLICATION**
published in accordance with Art. 153(4) EPC

(43) Date of publication:
**03.05.2023 Bulletin 2023/18**

(21) Application number: **21834252.5**

(22) Date of filing: **29.06.2021**

(51) International Patent Classification (IPC):
**A61K 39/395** $^{(2006.01)}$ **C07K 16/28** $^{(2006.01)}$

(52) Cooperative Patent Classification (CPC):
**A61K 39/395; C07K 16/28**

(86) International application number:
**PCT/CN2021/102998**

(87) International publication number:
**WO 2022/002019 (06.01.2022 Gazette 2022/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.06.2020 CN 202010617663**

(71) Applicants:
• **Jiangsu Hengrui Pharmaceuticals Co., Ltd.**
  **Lianyungang, Jiangsu 222047 (CN)**
• **Shanghai Hengrui Pharmaceutical Co., Ltd.**
  **Shanghai 200245 (CN)**

(72) Inventors:
• **SUN, Le**
  **Shanghai 200245 (CN)**

• **YE, Xin**
  **Shanghai 200245 (CN)**
• **CHEN, Yuxiao**
  **Shanghai 200245 (CN)**
• **JIN, Xinsheng**
  **Shanghai 200245 (CN)**
• **TAO, Weikang**
  **Shanghai 200245 (CN)**

(74) Representative: **Potter Clarkson**
**Chapel Quarter**
**Mount Street**
**Nottingham NG1 6HQ (GB)**

Remarks:
The complete document including Reference Table(s) and the Sequence Listing(s) can be downloaded from the EPO website

(54) **ANTI-CD70 ANTIBODY AND APPLICATION THEREOF**

(57) The present invention relates to an anti-CD70 antibody and an application thereof. Specifically, the present disclosure relates to an anti-CD70 antibody, which comprises a light chain variable region and a heavy chain variable region of the antibody, and a use as a drug.

EP 4 173 638 A1

**Description**

**TECHNICAL FIELD**

The present disclosure relates to the field of biomedicine, and in particular relates to an antibody binding to CD70 and use thereof.

**BACKGROUND**

The statements herein merely provide background information related to the present disclosure and may not necessarily constitute the prior art.

CD70 is a cell surface antigen belonging to the tumor necrosis factor (TNF) family, and is a type II membrane protein containing 193 amino acids with a molecular weight of about 50 kD. CD70 interacts with its receptor CD27 *in vivo* as a homotrimer, and the intracellular domain of CD27 binds to TNF (tumor necrosis factor) receptor-associated factors (TRAFs), such as TRAF2 and TRAF5, to activate the NFκB and JNK pathways, ultimately leading to pro-survival and proliferation signals. CD27 signaling induced by CD70 leads to increased production and activation of regulatory T cells expressing CD27. CD70 also evades immune surveillance by inducing regulatory T cells, thereby promoting tumor growth. Under physiological conditions, CD70 is transiently expressed on activated T cells, B cells and dendritic cells, but rarely expressed on normal non-lymphoid tissues. However, CD70 is highly expressed in a variety of hematologic and solid tumors, such as B cell lymphoma, renal cancer and breast cancer, and has a negative correlation with prognosis. CD27 is co-expressed with CD70 in hematological tumors, and their binding results in cleavage of the extracellular domain of CD27, enabling the formation of soluble CD27 (sCD27) which can be used as a diagnostic biomarker.

To date, various anti-CD70 antibodies have been disclosed in several documents including WO2012123586A1, WO2006044643A3, WO2007038637A3 and WO2017138471A1. At present, there are still no effective anti-CD70 antibody drugs for clinical application, and novel effective anti-CD70 antibody drugs need to be developed.

**SUMMARY**

The present disclosure provides a novel anti-CD70 antibody. The anti-CD70 antibody described herein includes an anti-CD70 full-length antibody and an antigen-binding fragment thereof.

In some embodiments, the present disclosure provides an anti-CD70 antibody comprising a heavy chain variable region and a light chain variable region, wherein:

    i) the heavy chain variable region comprises a HCDR1 and a HCDR3 set forth in SEQ ID NO: 9 and SEQ ID NO: 11, respectively, and a HCDR2 set forth in SEQ ID NO: 10 or SEQ ID NO: 42; and the light chain variable region comprises a LCDR1 and a LCDR3 set forth in SEQ ID NO: 12 and SEQ ID NO: 14, respectively, and a LCDR2 set forth in SEQ ID NO: 13 or SEQ ID NO: 43;
    ii) the heavy chain variable region comprises a HCDR1 and a HCDR3 set forth in SEQ ID NO: 15 and SEQ ID NO: 17, respectively, and a HCDR2 set forth in SEQ ID NO: 16 or SEQ ID NO: 54; and the light chain variable region comprises a LCDR1, a LCDR2 and a LCDR3 set forth in SEQ ID NO: 18, SEQ ID NO: 19 and SEQ ID NO: 20 respectively; or
    iii) the heavy chain variable region comprises a HCDR1 and a HCDR3 set forth in SEQ ID NO: 21 and SEQ ID NO: 23, respectively, and a HCDR2 set forth in SEQ ID NO: 22 or SEQ ID NO: 71; and the light chain variable region comprises a LCDR1 and a LCDR3 set forth in SEQ ID NO: 24 and SEQ ID NO: 25, respectively, and a LCDR2 set forth in SEQ ID NO: 13 or SEQ ID NO: 43.

In some embodiments, the anti-CD70 antibody of the present disclosure comprises a heavy chain variable region and a light chain variable region, wherein,

    the heavy chain variable region comprises a HCDR1, a HCDR2 and a HCDR3 set forth in SEQ ID NO: 9, SEQ ID NO: 10 and SEQ ID NO: 11, respectively, and the light chain variable region comprises a LCDR1, a LCDR2 and a LCDR3 set forth in SEQ ID NO: 12, SEQ ID NO: 13 and SEQ ID NO: 14, respectively; or
    the heavy chain variable region comprises a HCDR1, a HCDR2 and a HCDR3 set forth in SEQ ID NO: 9, SEQ ID NO: 42 and SEQ ID NO: 11, respectively, and the light chain variable region comprises a LCDR1, a LCDR2 and a LCDR3 set forth in SEQ ID NO: 12, SEQ ID NO: 43 and SEQ ID NO: 14, respectively; or
    the heavy chain variable region comprises a HCDR1, a HCDR2 and a HCDR3 set forth in SEQ ID NO: 15, SEQ ID NO: 16 and SEQ ID NO: 17, respectively, and the light chain variable region comprises a LCDR1, a LCDR2 and a LCDR3 set forth in SEQ ID NO: 18, SEQ ID NO: 19 and SEQ ID NO: 20, respectively; or

the heavy chain variable region comprises a HCDR1, a HCDR2 and a HCDR3 set forth in SEQ ID NO: 15, SEQ ID NO: 54 and SEQ ID NO: 17, respectively, and the light chain variable region comprises a LCDR1, a LCDR2 and a LCDR3 set forth in SEQ ID NO: 18, SEQ ID NO: 19 and SEQ ID NO: 20, respectively; or

the heavy chain variable region comprises a HCDR1, a HCDR2 and a HCDR3 set forth in SEQ ID NO: 21, SEQ ID NO: 22 and SEQ ID NO: 23, respectively, and the light chain variable region comprises a LCDR1, a LCDR2 and a LCDR3 set forth in SEQ ID NO: 24, SEQ ID NO: 13 and SEQ ID NO: 25, respectively; or

the heavy chain variable region comprises a HCDR1, a HCDR2 and a HCDR3 set forth in SEQ ID NO: 21, SEQ ID NO: 71 and SEQ ID NO: 23, respectively, and the light chain variable region comprises a LCDR1, a LCDR2 and a LCDR3 set forth in SEQ ID NO: 24, SEQ ID NO: 43 and SEQ ID NO: 25, respectively.

In some embodiments, the anti-CD70 antibody described above is a murine antibody, a chimeric antibody or a humanized antibody; in some embodiments, the anti-CD70 antibody described above is a full-length antibody or an antigen-binding fragment thereof. In some embodiments, the antigen-binding fragment is selected from the group consisting of: a Fab, an F(ab')$_2$, an F(ab)$_2$, an Fd, an Fv, a dsFv, an scFv and a diabody thereof.

In some embodiments, the anti-CD70 antibody described above is a humanized antibody, wherein the humanized antibody comprises a framework region or a framework region variant of a human antibody, wherein the framework region variant has up to 11 amino acid back mutations relative to a light chain framework region and/or a heavy chain framework region of the human antibody.

In some embodiments, the antibody comprises a heavy chain framework region comprising one or more amino acid back mutations selected from the group consisting of 4M, 37I, 38K, 48I, 67A, 69L, 71A, 73R, 78A, 80L and 94T, and/or a light chain framework region comprising one or more amino acid back mutations selected from the group consisting of 5S and 70N; in some embodiments, the antibody comprises a light chain framework region comprising one or more amino acid back mutations selected from the group consisting of 38R, 43S, 69R, 70Q and 71Y, and/or a heavy chain framework region comprising one or more amino acid back mutations selected from the group consisting of 2I, 24T, 46K, 72E, and 82a N; in some embodiments, the antibody comprises a heavy chain framework region comprising one or more amino acid back mutations selected from the group consisting of 27D, 30P, 37L, 38K, 48I, 66K, 67A, 69L and 82a N, and/or a light chain framework region comprising a 49S amino acid back mutation; wherein sites of the mutations are numbered according to the Kabat numbering scheme, for example, "4M" indicates that a residue at position 4 (corresponding to Kabat numbering scheme) of the heavy chain variable region is "M".

In some embodiments, the anti-CD70 antibody comprises a heavy chain framework region variant of the human antibody comprising one or more amino acid back mutations selected from the group consisting of 4M, 37I, 38K, 48I, 67A, 69L, 71A, 73R, 78A, 80L and 94T relative to the heavy chain framework region of the human antibody, and/or the anti-CD70 antibody comprises a light chain framework region variant of the human antibody comprising one or more amino acid back mutations selected from the group consisting of 5S and 70N relative to the light chain framework region of the human antibody; in some embodiments, the anti-CD70 antibody comprises a light chain framework region variant of the human antibody comprising one or more amino acid back mutations selected from the group consisting of 38R, 43S, 69R, 70Q and 71Y relative to the light chain framework region of the human antibody, and/or the anti-CD70 antibody comprises a heavy chain framework region variant of the human antibody comprising one or more amino acid back mutations selected from the group consisting of 2I, 24T, 46K, 72E and 82a N relative to the heavy chain framework region of the human antibody; in some embodiments, the anti-CD70 antibody comprises a heavy chain framework region variant of the human antibody comprising one or more amino acid back mutations relative to the heavy chain framework region of the human antibody selected from the group consisting of 27D, 30P, 37L, 38K, 48I, 66K, 67A, 69L and 82a N, and/or the anti-CD70 antibody comprises a light chain framework region variant of the human antibody comprising a 49S amino acid back mutation relative to the light chain framework region of the human antibody; wherein sites of the mutations are numbered according to the Kabat numbering scheme, for example, "4M" indicates that a residue at position 4 (corresponding to the Kabat numbering scheme) of the heavy chain variable region is "M".

In some embodiments, the humanized antibody comprises a light chain variable region and a heavy chain variable region selected from the group consisting of a), b) and c) below:

the humanized antibody comprises:

a) a heavy chain variable region comprising a HCDR1 and a HCDR3 set forth in SEQ ID NO: 9 and SEQ ID NO: 11, respectively, and a HCDR2 set forth in SEQ ID NO: 10 or SEQ ID NO: 42; and a light chain variable region comprising a LCDR1 and a LCDR3 set forth in SEQ ID NO: 12 and SEQ ID NO: 14, respectively, and a LCDR2 set forth in SEQ ID NO: 13 or SEQ ID NO: 43; wherein the heavy chain variable region comprises a heavy chain framework region variant of the human antibody comprising one or more amino acid back mutations selected from the group consisting of 4M, 37I, 38K, 48I, 67A, 69L, 71A, 73R, 78A, 80L and 94T relative to the heavy chain framework region of the human antibody, and/or the light chain variable region comprises a light chain framework region variant of the human antibody comprising one or more amino acid back mutations selected from the group consisting of 5S and 70N relative to the light chain framework region of the human antibody; or

b) a heavy chain variable region comprising a HCDR1 and a HCDR3 set forth in SEQ ID NO: 15 and SEQ ID NO: 17, respectively, and a HCDR2 set forth in SEQ ID NO: 16 or SEQ ID NO: 54; and a light chain variable region comprising a LCDR1, a LCDR2 and a LCDR3 set forth in SEQ ID NO: 18, SEQ ID NO: 19 and SEQ ID NO: 20, respectively; wherein the light chain variable region comprises a light chain framework region variant of the human antibody comprising one or more amino acid back mutations selected from the group consisting of 38R, 43S, 69R, 70Q and 71Y relative to the light chain framework region of the human antibody, and/or the heavy chain variable region comprises a heavy chain framework region variant of the human antibody comprising one or more amino acid back mutations selected from the group consisting of 2I, 24T, 46K, 72E and 82a N relative to the heavy chain framework region of the human antibody; or

c) a heavy chain variable region comprising a HCDR1 and a HCDR3 set forth in SEQ ID NO: 21 and SEQ ID NO: 23, respectively, and a HCDR2 set forth in SEQ ID NO: 22 or SEQ ID NO: 71; and a light chain variable region comprising a LCDR1 and a LCDR3 set forth in SEQ ID NO: 24 and SEQ ID NO: 25, respectively, and a LCDR2 set forth in SEQ ID NO: 13 or SEQ ID NO: 43; wherein the heavy chain variable region comprises a heavy chain framework region variant of the human antibody comprising one or more amino acid back mutations selected from the group consisting of 27D, 30P, 37L, 38K, 48I, 66K, 67A, 69L and 82a N relative to the heavy chain framework region of the human antibody, and/or the light chain variable region comprises a light chain framework region variant of the human antibody comprising a 49S amino acid back mutation relative to the light chain framework region of the human antibody; wherein sites of the mutations are numbered according to the Kabat numbering scheme.

In some embodiments, the anti-CD70 antibody described above comprises a heavy chain variable region having one or more amino acid back mutations selected from the group consisting of 4M, 37I, 38K, 48I, 67A, 69L, 71A, 73R, 78A, 80L and 94T on the basis of SEQ ID NO: 26 or SEQ ID NO: 34, and a light chain variable region having one or more amino acid back mutations selected from the group consisting of 5S and 70N on the basis of SEQ ID NO: 32 or SEQ ID NO: 40; in some embodiments, the anti-CD70 antibody described above comprises a heavy chain variable region having one or more amino acid back mutations selected from the group consisting of 2I, 24T, 46K, 72E and 82a N on the basis of SEQ ID NO: 44 or SEQ ID NO: 51, and a light chain variable region having one or more amino acid back mutations selected from the group consisting of 38R, 43S, 69R, 70Q and 71Y on the basis of SEQ ID NO: 47; in some embodiments, the anti-CD70 antibody described above comprises a heavy chain variable region having one or more amino acid back mutations selected from the group consisting of 27D, 30P, 37L, 38K, 48I, 66K, 67A, 69L and 82a N on the basis of SEQ ID NO: 55 or SEQ ID NO: 63, and a light chain variable region having a 49S amino acid back mutation on the basis of SEQ ID NO: 61 or SEQ ID NO: 69; wherein sites of the mutations are numbered according to the Kabat numbering scheme.

It will be understood by those skilled in the art that when numbering schemes other than Kabat are used for the amino acid back mutation sites described above, amino acid residues that are functionally and/or structurally equivalent may be assigned different numbers but still correspond to the sites defined in the present disclosure.

In some embodiments, the anti-CD70 antibody described above comprises a heavy chain variable region and a light chain variable region, wherein:

d) the heavy chain variable region comprises an amino acid sequence having at least 90%, 91%, 92%, 93%, 94%, 95%, 96%, 97%, 98%, 99% or 100% sequence identity to SEQ ID NO: 3, 26, 27, 28, 29, 30, 31, 34, 35, 36, 37, 38 or 39, and/or the light chain variable region comprises an amino acid sequence having at least 90%, 91%, 92%, 93%, 94%, 95%, 96%, 97%, 98%, 99% or 100% sequence identity to SEQ ID NO: 4, 32, 33, 40 or 41;

e) the heavy chain variable region comprises an amino acid sequence having at least 90%, 91%, 92%, 93%, 94%, 95%, 96%, 97%, 98%, 99% or 100% sequence identity to SEQ ID NO: 5, 44, 45, 46, 51, 52 or 53, and/or the light chain variable region comprises an amino acid sequence having at least 90%, 91%, 92%, 93%, 94%, 95%, 96%, 97%, 98%, 99% or 100% sequence identity to SEQ ID NO: 6, 47, 48, 49 or 50; or

f) the heavy chain variable region comprises an amino acid sequence having at least 90%, 91%, 92%, 93%, 94%, 95%, 96%, 97%, 98%, 99%, or 100% sequence identity to SEQ ID NO: 7, 55, 56, 57, 58, 59, 60, 63, 64, 65, 66, 67 or 68, and/or the light chain variable region comprises an amino acid sequence having at least 90%, 91%, 92%, 93%, 94%, 95%, 96%, 97%, 98%, 99% or 100% sequence identity to SEQ ID NO: 8, 61, 62, 69 or 70.

In some embodiments, the anti-CD70 antibody described above comprises a combination of a heavy chain variable region and a light chain variable region as shown in Tables 1, 2, and 3 below:

Table 1. Combinations of light/heavy chain variable regions of a humanized antibody huB 1

|  | huB1VL1-1 (SEQ ID NO: 40) | huB1VL1-2 (SEQ ID NO: 41) | huBIVLI (SEQ ID NO: 32) | huB1VL2 (SEQ ID NO: 33) |
|---|---|---|---|---|
| huB!VHI (SEQ ID NO: 26) | huB1V001 | huB1V015 | huB1V025 | huB1V037 |
| huB1VH2 (SEQ ID NO: 27) | huB1V002 | huB1V016 | huB1V026 | huB1V038 |
| huB1VH3 (SEQ ID NO: 28) | huB1V003 | huB1V017 | huB1V027 | huB1V039 |
| huB!VH4 (SEQ ID NO: 29) | huB1V004 | huB1V018 | huB1V028 | huB1V040 |
| huB1VH5 (SEQ ID NO: 30) | huB!V005 | huB1V019 | huB!V029 | huB1V041 |
| huB1VH6 (SEQ ID NO: 31) | huB1V006 | huB1V007 | huB1V030 | huB!V042 |
| huB1VH1-1 (SEQ ID NO: 34) | huB1V009 | huB1V008 | huB!V031 | huB!V043 |
| huB1VH2-1 (SEQ ID NO: 35) | huB1V010 | huB1V020 | huB1V032 | huB1V044 |
| huB1VH3-1 (SEQ ID NO: 36) | huB1V011 | huB1V021 | huB!V033 | huB!V045 |
| huB1VH4-1 (SEQ ID NO: 37) | huB1V012 | huB1V022 | huB1V034 | huB1V046 |
| huB1VH5-1 (SEQ ID NO: 38) | huB!V013 | huB1V023 | huB1V035 | huB!V047 |
| huB1VH6-1 (SEQ ID NO: 39) | huB1V014 | huB1V024 | huB1V036 | huB1V048 |

Note: in the table, for example, "huB1V001" indicates that the heavy chain variable region of the antibody is SEQ ID NO: 26 in the same row as the antibody, and the light chain variable region of the antibody is SEQ ID NO: 40 in the same column as the antibody, and so on for others.

Table 2. Combinations of light and heavy chain variable regions of a humanized antibody huB7

|  | huB7VL1 (SEQ ID NO: 47) | huB7VL2 (SEQ ID NO: 48) | huB7VL3 (SEQ ID NO: 49) | huB7VL4 (SEQ ID NO: 50) |
|---|---|---|---|---|
| huB7VH1 (SEQ ID NO: 44) | huB7V001 | huB7V007 | huB7V013 | huB7V019 |
| huB7VH1-1 (SEQ ID NO: 51) | huB7V002 | huB7V008 | huB7V014 | huB7V020 |
| huB7VH2 (SEQ ID NO: 45) | huB7V003 | huB7V009 | huB7V015 | huB7V021 |
| huB7VH2-1 (SEQ ID NO: 52) | huB7V004 | huB7V010 | huB7V016 | huB7V022 |
| huB7VH3 (SEQ ID NO: 46) | huB7V005 | huB7V011 | huB7V017 | huB7V023 |

(continued)

| | huB7VL1 (SEQ ID NO: 47) | huB7VL2 (SEQ ID NO: 48) | huB7VL3 (SEQ ID NO: 49) | huB7VL4 (SEQ ID NO: 50) |
|---|---|---|---|---|
| huB7VH3-1 (SEQ ID NO: 53) | huB7V006 | huB7V012 | huB7V018 | huB7V024 |

Note: in the table, for example, "huB7V001" indicates that the heavy chain variable region of the antibody is SEQ ID NO: 44 in the same row as the antibody, and the light chain variable region of the antibody is SEQ ID NO: 47 in the same column as the antibody, and so on for others.

Table 3. Combinations of heavy and light chain variable regions of a humanized antibody huF4

| | huF4VL1-1 (SEQ ID NO:69) | huF4VL2-1 (SEQ ID NO:70) | huF4VL1 (SEQ ID NO:61) | huF4VL2 (SEQ ID NO:62) |
|---|---|---|---|---|
| huF4VH1 (SEQ ID NO: 55) | huF4V001 | huF4V014 | huF4V025 | huF4V037 |
| huF4VH2 (SEQ ID NO: 56) | huF4 V002 | huF4V015 | huF4V026 | huF4V038 |
| huF4VH3 (SEQ ID NO: 57) | huF4V003 | huF4V016 | huF4 V027 | huF4V039 |
| huF4VH4 (SEQ ID NO: 58) | huF 4 V004 | huF4V017 | huF4V028 | huF 4 V040 |
| huF4VH5 (SEQ ID NO: 59) | huF4V005 | huF4V018 | huF4V029 | huF4V041 |
| huF4VH6 SEQ ID NO: 60 | huF 4 V006 | huF4V019 | huF4V030 | huF4 V042 |
| huF4VH1-1 (SEQ ID NO:63) | huF 4 V007 | huF4 V020 | huF4V031 | huF4V043 |
| huF4VH2-1 (SEQ ID NO:64) | huF4V008 | huF4V021 | huF4V032 | huF 4 V044 |
| huF4VH3-1 (SEQ ID NO:65) | huF4V009 | huF4V011 | huF4V033 | huF4V045 |
| huF4VH4-1 (SEQ ID NO:66) | huF4V010 | huF4 V022 | huF4V034 | huF 4 V046 |
| huF4VH5-1 (SEQ ID NO:67) | huF4V012 | huF4 V023 | huF4V035 | huF4V047 |
| huF4VH6-1 (SEQ ID NO:68) | huF4V013 | huF4 V024 | huF4V036 | huF4V048 |

Note: in the table, for example, "huF4V001" indicates that the heavy chain variable region of the antibody is SEQ ID NO: 55 in the same row as the antibody, and the light chain variable region is SEQ ID NO: 69 in the same column as the antibody, and so on for others.

In some embodiments, the anti-CD70 antibody described above comprises a heavy chain variable region and a light chain variable region, wherein:

g) the heavy chain variable region comprises an amino acid sequence set forth in SEQ ID NO: 3; and the light chain variable region comprises an amino acid sequence set forth in SEQ ID NO: 4; or
h) the heavy chain variable region comprises an amino acid sequence set forth in SEQ ID NO: 26, 27, 28, 29, 30, 31, 34, 35, 36, 37, 38 or 39; and the light chain variable region comprises an amino acid sequence set forth in SEQ ID NO: 32, 33, 40 or 41; or

i) the heavy chain variable region comprises an amino acid sequence set forth in SEQ ID NO: 5; and the light chain variable region comprises an amino acid sequence set forth in SEQ ID NO: 6; or

j) the heavy chain variable region comprises an amino acid sequence set forth in SEQ ID NO: 44, 45, 46, 51, 52 or 53; and the light chain variable region comprises an amino acid sequence set forth in SEQ ID NO: 47, 48, 49 or 50; or

k) the heavy chain variable region comprises an amino acid sequence set forth in SEQ ID NO: 7; and the light chain variable region comprises an amino acid sequence set forth in SEQ ID NO: 8; or

l) the heavy chain variable region comprises an amino acid sequence set forth in SEQ ID NO: 55, 56, 57, 58, 59, 60, 63, 64, 65, 66, 67 or 68; and the light chain variable region comprises an amino acid sequence set forth in SEQ ID NO: 61, 62, 69 or 70.

In some embodiments, the anti-CD70 antibody described above comprises a heavy chain variable region and a light chain variable region as shown below, wherein:

n) the heavy chain variable region comprises an amino acid sequence set forth in SEQ ID NO: 35; and the light chain variable region comprises an amino acid sequence set forth in SEQ ID NO: 40; or

o) the heavy chain variable region comprises an amino acid sequence set forth in SEQ ID NO: 51; and the light chain variable region comprises an amino acid sequence set forth in SEQ ID NO: 47; or

p) the heavy chain variable region comprises an amino acid sequence set forth in SEQ ID NO: 65; and the light chain variable region comprises an amino acid sequence set forth in SEQ ID NO: 70.

In some embodiments, the anti-CD70 antibody described above comprises a heavy chain constant region and a light chain constant region of the antibody; wherein preferably, the heavy chain constant region is selected from the group consisting of constant regions of human IgG1, IgG2, IgG3 and IgG4 and conventional variants thereof, and the light chain constant region is selected from the group consisting of constant regions of human antibody $\kappa$ and $\lambda$ chains and conventional variants thereof; and more preferably, the antibody comprises a heavy chain constant region set forth in SEQ ID NO: 72 and a light chain constant region set forth in SEQ ID NO: 73.

In some embodiments, the anti-CD70 antibody described above comprises:

q) a heavy chain having at least 85%, 90%, 91%, 92%, 93%, 94%, 95%, 96%, 97%, 98%, 99% or 100% sequence identity to SEQ ID NO: 74 and/or a light chain having at least 85%, 90%, 91%, 92%, 93%, 94%, 95%, 96%, 97%, 98%, 99% or 100% sequence identity to SEQ ID NO: 75; or

r) a heavy chain having at least 85%, 90%, 91%, 92%, 93%, 94%, 95%, 96%, 97%, 98%, 99% or 100% sequence identity to SEQ ID NO: 76 and/or a light chain having at least 85%, 90%, 91%, 92%, 93%, 94%, 95%, 96%, 97%, 98%, 99% or 100% identity to SEQ ID NO: 77; or

s) a heavy chain having at least 85%, 90%, 91%, 92%, 93%, 94%, 95%, 96%, 97%, 98%, 99% or 100% sequence identity to SEQ ID NO: 78, and/or a light chain having at least 85%, 90%, 91%, 92%, 93%, 94%, 95%, 96%, 97%, 98%, 99% or 100% identity to SEQ ID NO: 79.

In some embodiments, the anti-CD70 antibody described above comprises:

t) a heavy chain set forth in SEQ ID NO: 74 and a light chain set forth in SEQ ID NO: 75; or

u) a heavy chain set forth in SEQ ID NO: 76 and a light chain set forth in SEQ ID NO: 77; or

v) a heavy chain set forth in SEQ ID NO: 78 and a light chain set forth in SEQ ID NO: 79.

In some embodiments, the present disclosure also provides an isolated anti-CD70 antibody, wherein the antibody competes for binding to a human CD70, a human CD70 epitope, a monkey CD70, or a monkey CD70 epitope with the anti-CD70 antibody according to any one of the above embodiments. In some embodiments, the antibody binds to the same epitope on human CD70 as the anti-CD70 antibody according to any one of the above embodiments.

In some embodiments, the anti-CD70 antibody according to any one of the above embodiments is a low-fucosylated antibody; in some embodiments, the low-fucosylated anti-CD70 antibody is an antibody with at least 80%, 85%, 90%, 91%, 92%, 93%, 94%, 95%, 96%, 97%, 98%, 99% or 100% of heavy chain not modified by fucosylation. In some embodiments, the low-fucosylated anti-CD70 antibody is an antibody with at least 95%, 96%, 97%, 98%, 99% or 100% of heavy chain not modified by fucosylation. In some embodiments, the anti-CD70 antibody described above is an IgG1 antibody with 100% of heavy chain not modified by fucosylation (also referred to as a non-fucosylated IgG1 antibody).

In some embodiments, the anti-CD70 antibody according to any one of the above embodiments has at least one of the following properties:

A. binding to human CD70 with a KD value of less than $1\times10^{-8}$ M, preferably less than $1\times10^{-9}$ M, or less than $1\times10^{-10}$ M, or less than $6\times10^{-11}$ M, or less than $5\times10^{-11}$ M, or less than $4\times10^{-11}$ M, or less than $3\times10^{-11}$ M, wherein the KD value is determined by surface plasmon resonance technology; for example, is assayed by the method described in Test Example 1 of the present disclosure;

B. being able to bind to both a human CD70 antigen and a monkey CD70 antigen, but not a mouse CD70 antigen;

C. being able to inhibit CD70-induced CD27 signaling, wherein preferably, the anti-CD70 antibody has maximum percentage inhibition (Imax (%)) of greater than or equal to 72%, 75%, 80%, 85%, 86%, 87%, 88%, 89%, 90%, 91%, 92%, 93%, 94%, 95%, 96%, 97%, 98%, 99% or 100%; more preferably, greater than or equal to 90%, 91%, 93% or 98%, for inhibiting IL-8 secretion from human CD27-expressing cells (e.g., HT1080/CD27 cells), wherein the IL-8 secretion is assayed by an Elisa method, for example by the method described in Test Example 5 of the present disclosure;

D. having one or more of the following effector functions: antibody-dependent cell-mediated cytotoxicity (ADCC), complement-dependent cytotoxicity (CDC) and antibody-dependent cell-mediated phagocytosis (ADCP) for human CD70-expressing cells; wherein preferably, the anti-CD70 antibody has a maximum lysis rate of greater than or equal to 70%, 74%, 75%, 76%, 77%, 78%, 79%, 80%, 81%, 82%, 83%, 84%, 85%, 86%, 87%, 88%, 89%, 90%, 95% or 100%; more preferably greater than or equal to 74%, 78%, 84% or 86%, for lysing human CD70-expressing cells (e.g., Raji cells) by CDC effector function; in some embodiments, the CDC effector function is assayed by the method described in Test Example 7 of the present disclosure; and

E. being able to be internalized by human CD70-expressing cells, wherein preferably, the cell internalization and lysis are performed at a maximum lysis rate of greater than or equal to 70%, 75%, 80%, 85%, 90%, 91%, 92%, 93%, 94%, 95%, 96%, 97%, 98%, 99% or 100%; more preferably greater than or equal to 96% or 97%; in some embodiments, the cell internalization is assayed by the method described in Test Example 10 of the present disclosure.

In some embodiments, the present disclosure also provides a nucleic acid molecule encoding the anti-CD70 antibody according to any one of the above embodiments.

In some embodiments, the present disclosure also provides a host cell comprising the nucleic acid molecule described above. The host cell may be selected from the group consisting of a prokaryotic cell and a eukaryotic cell, preferably a eukaryotic cell, more preferably a mammalian cell, preferably a mammalian cell not including a human cell, wherein the mammalian cell includes, but is not limited to CHO, 293, NSO and cells in which gene editing in a mammalian cell can alter the glycosylation modification of an antibody or an antigen-binding fragment thereof, thereby altering the ADCC function of the antibody or the antigen-binding fragment thereof, e.g. by knocking out genes such as *Fut8* or *GnT-III*.

In some embodiments, the present disclosure also provides a method for preparing the anti-CD70 antibody described above, which comprises the steps of culturing the host cell described above, and purifying and isolating the antibody.

In some embodiments, the present disclosure also provides an immunoconjugate comprising the anti-CD70 antibody according to any one of the above embodiments and an effector molecule conjugated to the anti-CD70 antibody; preferably, the effector molecule is selected from the group consisting of a radioisotope, an anti-tumor agent, an immunomodulator, a biological response modifier, a lectin, a cytotoxic drug, a chromophore, a fluorophore, a chemiluminescent compound, an enzyme, a metal ion, and any combination thereof.

In some embodiments, the present disclosure also provides a pharmaceutical composition comprising a therapeutically effective amount of the anti-CD70 antibody described above, or the nucleic acid molecule described above or the immunoconjugate described above, and one or more pharmaceutically acceptable carriers, diluents or excipients.

In some embodiments, the present disclosure also provides a method for immunodetection or determination of CD70, which comprises the step of making the anti-CD70 antibody according to any one of the above embodiments in contact with a subject or a sample from the subject.

In some embodiments, the present disclosure also provides a kit comprising the anti-CD70 antibody or the immunoconjugate according to any one of the above embodiments. In some embodiments, the present disclosure also provides a method for preventing or treating a disease or disorder, which comprises administering to a subj ect a therapeutically effective amount of the anti-CD70 antibody according to any one of the above embodiments, or the nucleic acid molecule described above, or the pharmaceutical composition described above, or the immunoconjugate described above.

In some embodiments, the present disclosure also provides use of the anti-CD70 antibody according to any one of the above embodiments, or the nucleic acid molecule described above, or the pharmaceutical composition described above, or the immunoconjugate described above, in the preparation of a medicament for preventing or treating a disease or disorder.

In some embodiments, the present disclosure provides the anti-CD70 antibody according to any one of the above embodiments, or the nucleic acid molecule described above, or the pharmaceutical composition described above, or the immunoconjugate described above, for use as a medicament for preventing or treating a disease or disorder.

In some embodiments, the disease or disorder according to any one of the above embodiments is a disease or disorder related to CD70. In some embodiments, the disease or disorder is a disease or disorder in which high CD70 expression is detrimental to a subject. In some embodiments, the disease or disorder is a tumor, an autoimmune disease or an infectious disease. In some embodiments, the tumor is selected from the group consisting of head and neck squamous cell carcinoma, head and neck cancer, brain cancer, neuroglioma, glioblastoma multiforme, neuroblastoma, central nervous system carcinoma, neuroendocrine tumor, throat cancer, nasopharyngeal cancer, esophageal cancer, thyroid cancer, malignant pleural mesothelioma, lung cancer, breast cancer, liver cancer, hepatobiliary cancer, pancreatic cancer, stomach cancer, gastrointestinal cancer, intestinal cancer, colon cancer, colorectal cancer, kidney cancer, clear cell renal cell carcinoma, ovarian cancer, endometrial cancer, cervical cancer, bladder cancer, prostate cancer, testicular cancer, skin cancer, melanoma, leukemia, lymphoma, bone cancer, chondrosarcoma, myeloma, multiple myeloma, myelodysplastic syndrome, Krukenberg tumor, myeloproliferative tumor, squamous cell carcinoma, Ewing's sarcoma, systemic light chain amyloidosis or Merkel cell carcinoma; in some other embodiments, the lymphoma is selected from the group consisting of Hodgkin's lymphoma, non-Hodgkin's lymphoma, diffuse large B-cell lymphoma, follicular lymphoma, primary mediastinal large B-cell lymphoma, mantle cell lymphoma, small lymphocytic lymphoma, large B-cell lymphoma rich in T-cells/histiocytes and lymphoplasmacytic lymphoma, the lung cancer is selected from the group consisting of non-small cell lung cancer and small cell lung cancer, and the leukemia is selected from the group consisting of chronic myeloid leukemia, acute myeloid leukemia, lymphocytic leukemia, lymphoblastic leukemia, acute lymphoblastic leukemia, chronic lymphocytic leukemia and myeloid cell leukemia. In some embodiments, the autoimmune disease is selected from the group consisting of rheumatoid arthritis, psoriasis, joint psoriasis, psoriasis, dermatitis, systemic scleroderma, systemic scleroderma and sclerosis, inflammatory bowel disease (IBD), Crohn's disease, ulcerative colitis, respiratory distress syndrome, meningitis, encephalitis, uveitis, glomerulonephritis, eczema, asthma, arteriosclerosis, leukocyte adhesion-deficient disease, multiple sclerosis, Raynaud's syndrome, Sjogren's syndrome, juvenile diabetes, Reiter's disease, Behcet's disease, immune complex nephritis, IgA nephropathy, IgM polyneuropathy, immune-mediated thrombocytopenic symptoms (such as acute idiopathic thrombocytopenic purpura and chronic idiopathic thrombocytopenic purpura), hemolytic anemia, myasthenia gravis, lupus nephritis, systemic lupus erythematosus, rheumatoid arthritis (RA), atopic dermatitis, pemphigus, Graves' disease, Hashimoto's thyroiditis, Wegener's granulomatosis, Omenn's syndrome, chronic renal failure, acute infectious mononucleosis, multiple sclerosis, HIV and herpes virus related diseases, severe acute respiratory syndrome, choreoretinitis and immunological diseases caused by virus infection (such as diseases caused or mediated by B cell infection by Epstein-Barr virus (EBV)). In some embodiments, the disease or disorder is: acute myeloid leukemia, myelodysplastic syndrome, nasopharyngeal cancer, non-Hodgkin's lymphoma, renal cell carcinoma, metastatic renal cell carcinoma, rheumatoid arthritis, and psoriasis.

In some embodiments, the therapeutically effective amount described above means that a unit dose of the pharmaceutical composition comprises 0.1 mg to 3000 mg of the anti-CD70 antibody described above, or the nucleic acid molecule described above, or the immunoconjugate described above, or the pharmaceutical composition described above. In some embodiments, the treatment further comprises administering to the subject a therapeutically effective amount of a second therapeutic agent.

**BRIEF DESCRIPTION OF THE DRAWINGS**

FIGs. 1A and 1B show experimental results of the binding of anti-CD70 antibodies to CD70-positive 786-O cells; in which FIG. 1A is a graph showing experimental results of the binding of anti-CD70 antibodies to CD70-positive 786-O cells, and FIG. 1B is a graph showing experimental results of the binding of non-fucosylated anti-CD70 antibodies to CD70-positive 786-O cells.

FIGs. 2A and 2B show experimental results of the binding of anti-CD70 antibodies to CD70-positive Raji cells, in which FIG. 2A is a graph showing experimental results of the binding of anti-CD70 antibodies to CD70-positive Raji cells, and FIG. 2B is a graph showing experimental results of the binding of non-fucosylated anti-CD70 antibodies to CD70-positive Raji cells.

FIG. 3 shows experimental results of the binding of huB7002 to human, monkey and mouse CD70 proteins by ELISA.

FIG. 4 shows experimental results of the binding of huB1010 to human, monkey and mouse CD70 proteins by ELISA.

FIG. 5 shows experimental results of the binding of huF4011 to human, monkey and mouse CD70 proteins by ELISA.

FIG. 6 shows experimental results of the blocking of the binding of CD27 to CD70-positive cells by anti-CD70 antibodies.

FIG. 7 shows experimental results of the blocking of the binding of CD27 to CD70-positive cells by non-fucosylated anti-CD70 antibodies.

FIG. 8 shows experimental results of the inhibition of IL-8 secretion from HT1080/CD27 cells by anti-CD70 antibodies.

FIG. 9 shows experimental results of the inhibition of IL-8 secretion from HT1080/CD27 cells by non-fucosylated anti-CD70 antibodies.

FIG. 10 shows experimental results of *in vitro* ADCC (NK92) of anti-CD70 antibodies on 786-O cells.

FIG. 11 shows experimental results of *in vitro* ADCC (PBMC) of anti-CD70 antibodies on 786-O cells.

FIG. 12 shows experimental results of *in vitro* CDC of anti-CD70 antibodies on Raji cells.

FIG. 13 shows experimental results of *in vitro* CDC of non-fucosylated anti-CD70 antibodies on Raji cells.

FIGs. 14A and 14B show experimental results *of in vitro* ADCP of anti-CD70 antibodies on 786-O and Raji cells, in which FIG. 14A is a graph showing experimental results of ADCP of anti-CD70 antibodies on 786-O cells, and FIG. 14B is a graph showing experimental results of ADCP of anti-CD70 antibodies on Raji cells.

FIG. 15 shows experimental results of *in vitro* inhibition of anti-CD70 antibodies on Treg cells.

FIG. 16 shows experimental results of internalization of 786-O cells on anti-CD70 antibodies.

FIG. 17 shows experimental results of *in vivo* pharmacodynamics of anti-CD70 antibodies in a mouse Raji model.

FIG. 18 shows experimental results of *in vivo* pharmacodynamics of non-fucosylated anti-CD70 antibodies in a mouse Raji model.

## Detailed Description of the Invention

Terms (definition)

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure belongs. The three-letter and single-letter codes for amino acids used in the present disclosure are as described in J. biol. chem, 243, p3558 (1968).

The term "antibody" herein is used in the broadest sense and encompasses a variety of antibody structures, including but not limited to monoclonal antibodies, polyclonal antibodies, multispecific antibodies (e.g., bispecific antibodies), full-length antibodies or antigen-binding fragments thereof (also known as antigen-binding moieties) so long as they exhibit the desired antigen-binding activity. A natural full-length antibody is an immunoglobulin (Ig) that comprises at least two heavy chains and two light chains interconnected by disulfide bonds. The heavy chain constant regions of immunoglobulins differ in their amino acid composition and arrangement, and thus in their antigenicity. Accordingly, immunoglobulins can be divided into five classes, otherwise called isotypes of immunoglobulins, namely IgM, IgD, IgG, IgA and IgE, with their corresponding heavy chains being $\mu$ chain, $\delta$ chain, $\gamma$ chain, $\alpha$ chain and $\varepsilon$ chain, respectively. Ig of the same class can be divided into different subclasses according to differences in the amino acid composition of the hinge regions and the number and positions of disulfide bonds of the heavy chains; for example, IgG can be divided into IgG1, IgG2, IgG3 and IgG4. Light chains are classified into $\kappa$ or $\lambda$ chains according to differences in the constant regions. Each of the five classes of Ig may have a $\kappa$ chain or $\lambda$ chain.

In the heavy and light chains of the antibody, the sequences of about 110 amino acids near the N-terminus vary considerably and thus are referred to as variable regions (abbreviated as Fv regions); the remaining amino acid sequences near the C-terminus are relatively stable and thus are referred to as constant regions. Each heavy chain consists of a heavy chain variable region (abbreviated as VH) and a heavy chain constant region (abbreviated as CH). The heavy chain constant region comprises three regions (domains), i.e., CH1, CH2 and CH3. Each light chain consists of a light chain variable region (abbreviated as VL) and a light chain constant region (abbreviated as CL). The heavy chain variable region and the light chain variable region comprise hypervariable regions (also referred to as complementarity determining regions, abbreviated as CDRs or HVRs) and framework regions (abbreviated as FRs) whose sequences are relatively conserved. Each VL and VH consist of 3 CDRs and 4 FRs arranged from the amino terminus to the carboxyl terminus in the following order: FR1-CDR1-FR2-CDR2-FR3-CDR3-FR4. The 3 CDRs of the light chain refer to LCDR1, LCDR2 and LCDR3, and the 3 CDRs of the heavy chain refer to HCDR1, HCDR2 and HCDR3.

The antibody of the present disclosure includes a murine antibody, a chimeric antibody and a humanized antibody.

The term "murine antibody" herein refers to a murine monoclonal antibody to an antigen (e.g., human CD70) prepared according to the knowledge and skills in the art. For example, a test subject is injected with a CD70 antigen, and then hybridoma of antibodies expressing the desired sequence or functional properties is isolated. In a preferred embodiment of the present disclosure, the murine anti-CD70 antibody or the antigen-binding fragment thereof may further comprise a light chain constant region of a murine $\kappa$ or $\lambda$ chain or a variant thereof, or further comprise a heavy chain constant region of a murine IgG1, IgG2, or IgG3 or a variant thereof.

The term "chimeric antibody" refers to an antibody obtained by fusing a variable region of a murine antibody to a constant region of a human antibody, which can reduce an immune response induced by the murine antibody. Generally, the chimeric antibody is constructed by firstly establishing hybridoma secreting murine specific monoclonal antibody, then cloning a variable region gene from murine hybridoma cells, cloning a constant region gene of human antibody as required, connecting the murine variable region gene and the human constant region gene into a chimeric gene, inserting the chimeric gene into an expression vector, and finally expressing chimeric antibody molecules in a eukaryotic system or prokaryotic system. In a preferred embodiment of the present disclosure, the light chain of the chimeric antibody further comprises a light chain constant region of a human κ or λ chain or a variant thereof. The antibody heavy chain of the CD70 chimeric antibody further comprises a heavy chain constant region of human IgG1, IgG2, IgG3 or IgG4 or a variant thereof, preferably a heavy chain constant region of human IgG1, IgG2 or IgG4, or an IgG1, IgG2 or IgG4 variant using an amino acid mutation (e.g., an L234A and/or L235A mutation, and/or an S228P mutation, 265A and/or 297A).

The term "humanized antibody", also known as a CDR-grafted antibody, refers to an antibody produced by grafting murine CDR sequences into a human antibody variable region framework, i.e., a different type of human germline antibody framework sequence. Such an antibody can overcome the heterogeneous reaction induced by the chimeric antibody because of carrying a large amount of mouse protein components. Such framework sequences can be obtained from public DNA databases or published references that include germline antibody gene sequences. For example, germline DNA sequences of genes of the human heavy and light chain variable regions can be found in the "VBase" human species sequence database, as well as in Kabat, E. A. et al., 1991 Sequences of Proteins of Immunological Interest, 5th edition. To avoid the decrease in activity caused by the decrease in immunogenicity of the antibody, the framework region sequence in human antibody variable region can be subjected to minimum reverse mutation or back mutation to maintain or enhance activity. The humanized antibody of the present disclosure also includes humanized antibodies which were further subjected to CDR affinity maturation mutation by yeast display.

In one embodiment of the present disclosure, the antibody or the antigen-binding fragment thereof may further comprise a light chain constant region of a human or murine κ and λ chain or a variant thereof, or may further comprise a heavy chain constant region of a human or murine IgG1, IgG2, IgG3 or IgG4 or a variant thereof; may comprise a heavy chain constant region of human IgG1, IgG2 or IgG4, or an IgG1, IgG2 or IgG4 variant using an amino acid mutation (e.g., L234A and/or L235A mutation, and/or S228P mutation, 265A and/or 297A).

The "conventional variant" of the human antibody heavy chain constant region and the human antibody light chain constant region described herein refers to a variant of the heavy chain constant region or light chain constant region derived from human that has been disclosed in the prior art and does not change the structure and function of the antibody variable region. Exemplary variants include IgG1, IgG2, IgG3 or IgG4 heavy chain constant region variants with site-directed modifications and amino acid substitutions in the heavy chain constant region. Specific substitutions are, for example, YTE mutation, L234A and/or L235A mutation, or S228P mutation, 265A (e.g., D265A) and/or 297A (e.g., N297A), and/or mutations to obtain a knob-into-hole structure (so that the antibody heavy chain has a combination of knob-Fc and hole-Fc) known in the art. These mutations have been confirmed to make the antibody have new properties, but do not change the function of the antibody variable region.

The "human antibody" (HuMAb), "human-derived antibody", "fully human antibody" and "completely human antibody" are used interchangeably and can be either a human derived antibody or an antibody obtained from a transgenic organism that is "engineered" to produce specific human antibodies in response to antigenic challenge and can be produced by any method known in the art. In certain techniques, elements of the human heavy and light chain gene loci are introduced into cell strains of organisms derived from embryonic stem cell lines in which endogenous heavy and light chain gene loci are subjected to targeted disruption. The transgenic organism can synthesize human antibodies specific to human antigens, and the organism can be used to produce human antibody-secreting hybridomas. A human antibody can also be an antibody in which the heavy and light chains are encoded by nucleotide sequences derived from one or more human DNA sources. Completely human antibodies can also be constructed by gene or chromosome transfection methods and phage display techniques, or by *in vitro* activated B cells, all of which are known in the art.

The terms "full-length antibody", "intact antibody", "complete antibody" and "whole antibody" are used interchangeably herein to refer to an antibody in its substantially intact form, as distinguished from an antigen-binding fragment defined below. The term especially refers to antibodies in which the light and heavy chains comprise constant regions.

The "antibody" of the present disclosure includes "full-length antibodies" and antigen-binding fragments thereof.

In some embodiments, the full length antibodies of the present disclosure include full length antibodies formed by linking the light and heavy chain variable regions of the combination in Tables 1, 2 and 3 to the light and heavy chain constant regions, respectively. Those skilled in the art can select different antibody-derived light chain constant regions and heavy chain constant regions according to actual needs, for example, human antibody-derived light chain constant regions and heavy chain constant regions. Meanwhile, the different combinations of the light chain variable region and heavy chain variable region in Tables 1, 2 and 3 may form a single-chain antibody (scFv), a Fab or other antigen-binding fragment forms containing an scFv or a Fab.

The term "antigen-binding fragment" or "functional fragment" or "antigen-binding moiety" refers to one or more fragments of an intact antibody that retain the ability to specifically bind to an antigen (e.g., CD70). It is shown that a fragment of a full-length antibody can be used to perform the antigen-binding function of the antibody. Illustratively, examples of the binding fragment included in the term "antigen-binding fragment" include (i) a Fab fragment, a monovalent fragment consisting of VL, VH, CL and CH1 domains; (ii) an F(ab')$_2$ fragment, a bivalent fragment comprising two Fab fragments linked by a disulfide bridge in the hinge region; (iii) an Fd fragment, consisting of VH and CH1 domains; (iv) an Fv fragment, consisting of VH and VL domains of one arm of the antibody; (V) a dsFv, a stable antigen-binding fragment formed by VH and VL via interchain disulfide bonds therebetween; (vi) a diabody, a bispecific antibody and a multi-specific antibody, comprising such fragments as an scFv, a dsFv and a Fab. Furthermore, although the two domains of the Fv fragment, VL and VH, are encoded by separate genes, these two domains can be linked by a recombinant method using an artificial peptide linker that enables them to be formed as a single protein chain, wherein the VL and VH pair to form a monovalent molecule, referred to as single-chain Fv (scFv) (see, e.g., Bird et al. (1988) Science 242: 423-426; and Huston et al. (1988) Proc. Natl. Acad. Sci USA 85:5879-5883). Such single-chain antibodies are also included in the term "antigen-binding fragment" of an antibody. Such antibody fragments are obtained by conventional techniques known to those skilled in the art, and screened for utility in the same manner as for intact antibodies. Antigen-binding moieties may be produced by a recombinant DNA technique or by enzymatic or chemical cleavage of intact immunoglobulins. Antibodies may be of different isotypes, e.g., IgG (e.g., subtype IgG1, IgG2, IgG3 or IgG4), IgA1, IgA2, IgD, IgE or IgM antibody.

The term "amino acid difference" or "amino acid mutation" refers to the presence of amino acid changes or mutations in the variant protein or polypeptide compared with the original protein or polypeptide, including occurrence of 1, 2, 3 or more amino acid insertions, deletions or substitutions on the sequence of the original protein or polypeptide.

The term "antibody framework" or "FR" refers to a portion of a variable domain VL or VH, which serves as a framework for the antigen-binding loops (CDRs) of the variable domain. It is essentially a variable domain without CDRs.

The term "complementarity determining region", "CDR" or "hypervariable region" refers to one of the 6 hypervariable regions within the variable domain of an antibody which primarily contribute to antigen binding. Generally, there are three CDRs (HCDR1, HCDR2 and HCDR3) in each heavy chain variable region and three CDRs (LCDR1, LCDR2 and LCDR3) in each light chain variable region. Any one of a variety of well-known schemes can be used to determine the amino acid sequence boundaries of the CDRs, including the "Kabat" numbering scheme (see Kabat et al., (1991), "Sequences of Proteins of Immunological Interest", 5th edition, Public Health Service, National Institutes of Health, Bethesda, MD), the "Chothia" numbering scheme (see Al-Lazikani et al., (1997) JMB 273: 927-948), the ImMunoGenTics (IMGT) numbering scheme (Lefranc M. P., Immunologist, 7, 132-136 (1999); Lefranc, M. P., et al., Dev. Comp. Immunol., 27, 55-77 (2003)), etc. For example, for the classical format, according to the Kabat scheme, the CDR amino acid residues in the heavy chain variable domain (VH) are numbered as 31-35 (HCDR1), 50-65 (HCDR2) and 95-102 (HCDR3); the CDR amino acid residues in the light chain variable domain (VL) are numbered as 24-34 (LCDR1), 50-56 (LCDR2) and 89-97 (LCDR3). According to the Chothia scheme, the CDR amino acids in VH are numbered as 26-32 (HCDR1), 52-56 (HCDR2) and 95-102 (HCDR3); and amino acid residues in VL are numbered as 26-32 (LCDR1), 50-52 (LCDR2) and 91-96 (LCDR3). According to the CDR definitions by combining both the Kabat scheme and the Chothia scheme, the CDR is composed of amino acid residues 26-35 (HCDR1), 50-65 (HCDR2) and 95-102 (HCDR3) in the human VH and amino acid residues 24-34 (LCDR1), 50-56 (LCDR2) and 89-97 (LCDR3) in the human VL. According to the IMGT scheme, the CDR amino acid residues in VH are roughly numbered as 26-35 (CDR1), 51-57 (CDR2) and 93-102 (CDR3), and the CDR amino acid residues in VL are roughly numbered as 27-32 (CDR1), 50-52 (CDR2) and 89-97 (CDR3). According to the IMGT scheme, the CDRs of the antibody can be determined by using the program IMGT/DomainGap Align. According to the AbM scheme, the CDR amino acids in VH are numbered as 26-32 (HCDR1), 50-58 (HCDR2) and 95-102 (HCDR3); and amino acid residues in VL are numbered as 24-34 (LCDR1), 50-56 (LCDR2) and 89-97 (LCDR3). Unless otherwise stated, the sequences of variable regions and CDRs of the antibody of the present disclosure correspond to the "Kabat" numbering scheme.

The term "epitope" or "antigenic determinant" refers to a site on an antigen (e.g., a specific site on an CD70 molecule) to which an antibody specifically binds. Epitopes generally comprise at least 3, 4, 5, 6, 7, 8, 9, 10, 11, 12, 13, 14 or 15 contiguous or non-contiguous amino acids in a unique spatial conformation. See, e.g., Epitope Mapping Protocols in Methods in Molecular Biology, volume 66, G.E. Morris, Ed. (1996).

The terms "specific binding", "selective binding", "selectively bind to" and "specifically bind to" refer to the binding of an antibody to an epitope on a predetermined antigen. Generally, the antibody binds with an affinity (KD) of less than about $10^{-8}$ M, e.g., less than about $10^{-9}$ M, $10^{-10}$ M, $10^{-11}$ M, $10^{-12}$ M, or less.

The term "KD" refers to the dissociation equilibrium constant for specific antibody-antigen interaction. Generally, the antibody of the present disclosure bind to CD70 with a dissociation equilibrium constant (KD) of less than about $10^{-8}$ M, e.g., less than about $10^{-9}$ M or $10^{-10}$ M, and the KD value are determined in a Biacore T200 instrument by the surface plasmon resonance (SPR) technique.

The term "compete", when used in a case where antigen-binding proteins (e.g., neutralizing antigen-binding proteins or neutralizing antibodies) compete for the same epitope, refers to the competition between the antigen-binding proteins, which is determined by the following assays in which a test antigen-binding protein (e.g., an antibody or an immunologically functional fragment thereof) prevents or inhibits (e.g., reduces) specific binding of a reference antigen-binding protein (e.g., a ligand or a reference antibody) to a common antigen (e.g., CD70 antigen or a fragment thereof). Numerous types of competitive binding assays are available for determining whether an antigen-binding protein competes with another, such as: solid phase direct or indirect radioimmunoassay (RIA), solid phase direct or indirect enzyme immunoassay (EIA), and sandwich competition assay (see, e.g., Stahli et al., 1983, Methods in Enzymology 9: 242-253); solid phase direct biotin-avidin EIA (see, e.g., Kirkland et al., 1986, J. Immunol. 137: 3614-3619), solid phase direct labeled assay, and solid phase direct labeled sandwich assay (see, e.g., Harlow and Lane, 1988, Antibodies: A Laboratory Manual, Cold Spring Harbor Press); solid phase direct labeled RIA with I-125 label (see, e.g., Morel et al., 1988, Molec. Immunol. 25: 7-15); solid phase direct biotin-avidin EIA (see, e.g., Cheung, et al., 1990, Virology 176: 546-552) and direct labeled RIA (Moldenhauer et al., 1990, Scand. J. Immunol. 32: 77-82). Generally, the assay relates to use of a purified antigen binding to a solid surface or a cell bearing any of an unlabeled assayed antigen-binding protein and a labeled reference antigen-binding protein. Competitive inhibition is determined by measuring the amount of label bound to the solid surface or the cell in the presence of the assayed antigen-binding protein. Generally, the assayed antigen-binding protein exists in an excessive amount. Antigen-binding proteins identified by the competitive assay (competitive antigen-binding proteins) include: an antigen-binding protein binding to the same epitope as a reference antigen-binding protein, and an antigen-binding protein binding to an adjacent epitope sufficiently close to a binding epitope of the reference antigen-binding protein, and the two epitopes spatially interfere with each other to prevent the binding. Other detailed information regarding the method for assaying competitive binding is provided in the examples herein. Generally, when the competitive antigen-binding protein exists in an excessive amount, the specific binding of the reference antigen-binding protein to the common antigen will be inhibited (e.g., reduced) by at least 40%-45%, 45%-50%, 50%-55%, 55%-60%, 60%-65%, 65%-70%, 70%-75% or 75% or more. In certain instances, the binding is inhibited by at least 80%-85%, 85%-90%, 90%-95%, 95%-97% or 97% or more.

The term "nucleic acid molecule" used herein refers to a DNA molecule and an RNA molecule. The nucleic acid molecule may be single-stranded or double-stranded, and is preferably a double-stranded DNA, a single-stranded mRNA or a modified mRNA. A nucleic acid is "operably linked" when it is placed into a functional relationship with another nucleic acid sequence. For example, a promoter or enhancer is operably linked to a coding sequence if it affects the transcription of the coding sequence.

The amino acid sequence "identity" refers to the percentage of amino acid residues in a first sequence that are identical to those in a second sequence in aligning the amino acid sequences (when necessary, gaps are introduced to achieve maximum percent sequence identity, and any conservative substitution is not considered as part of the sequence identity). For the purpose of determining percent amino acid sequence identity, alignments can be achieved in a variety of ways that are well-known in the art, for example, using software such as BLAST, BLAST-2, ALIGN, ALIGN-2 or Megalign (DNASTAR). Those skilled in the art can determine parameters suitable for measuring alignment, including any algorithms required to achieve maximum alignment of the full length of the aligned sequences.

The term "fucosylation", "fucosylated" or "fucosylation modification" refers to the presence of a fucose residue within an oligosaccharide attached to a peptide chain of an antibody. Fucosylation is a general process of post-translational modification of glycoproteins, representative enzyme genes related to core fucosylation include *GMD* (GDP-mannose 4,6-dehydratase) gene and *Fut8* (fut8, FUT8, alpha-1,6-fucosyltransferase) gene, and the core fucosylation level can be effectively regulated by inhibiting the expression of the two genes or constructing a *Fut8*-knockout CHO host cell (YAMANE-OHNUKI et al., "Establishment of FUT8knockout Chinese hamster ovary cells: an ideal host cell line for producing completely defucosylated antibodies with enhanced antibody-dependent cellular cytotoxicity.", BIOTECHNOLOGY AND BIOENGINEERING, 2004, p614-622). Generally, the fucosylated antibody comprises $\alpha$-1,6-fucose at a core *N*-acetylglucosamine (GlcNAc) residue (e.g., position Asn297 of human IgG1 Fc (corresponding to the EU numbering scheme)) in the *N*-oligosaccharides of the Fc region.

"Low-fucosylated" antibody refers to an antibody in which the carbohydrate structure attached to the Fc region has a low level of fucosylation modification; "non-fucosylated" or "afucosylated" antibody refers to an antibody in which the carbohydrate structure attached to the Fc region lacks fucosylation modification. The level of fucosylation of an antibody can be determined by determining all oligosaccharides by methods known in the art to determine the percentage of fucosylated oligosaccharides. Methods known in the art for determining fucosylation include, but are not limited to, gel electrophoresis, liquid chromatography, mass spectrometry, and the like. For example, the level of fucosylation of an antibody is determined by hydrophilic interaction chromatography (or hydrophilic interaction liquid chromatography, HILIC), for example by denaturing a sample with peptide-N-glycanase F to cleave N-linked glycans, and then analyzing N-linked glycans for fucose content. In the present disclosure, in some embodiments, the low-fucosylated antibody of the present disclosure is an antibody with at least 80% of heavy chain is not modified by fucosylation, e.g., with at least 80%-95%, 90%-95%, 95%-100%, 85%, 90%, 91%, 92%, 93%, 94%, 95%, 96%, 97%, 98%, 99% or 100% of heavy chain not modified by fucosylation. In the present disclosure, "non-fucosylated" refers to an antibody with 100% of heavy chain not modified by fucosylation, unless otherwise stated.

Low-fucosylated or non-fucosylated antibodies can be prepared by methods well known in the art. For example, they can be prepared by addition, removal or deletion of one or more carbohydrate moieties present in the antibody, for example, by cleavage of the fucose residue of the antibody using fucosidase (see Tarentino et al., (1975) Biochem. 14: 5516). Antibodies with reduced fucosylation can be prepared by changing the level of glycosylation by altering the composition of glycosylation, for example, by modifying the glycan moiety attached to each Fc fragment at the N297 residue (Natsume et al., (2009) Drug Des. Devel. Ther. 3: 7). They can also be prepared without altering the antibody sequence, for example, by expressing a low-fucosylated or non-fucosylated antibody by cells that alter the glycosylation pattern of the antibody, including, for example, glycosylation engineered cells that have been genetically engineered (see, e.g., Hse et al., (1997) J. Biol. Chem. 272: 9062-9070; Yang et al., (2015) Nature Biotechnology 33, 842-844). Various glycosylation engineered cells have been disclosed in the art, for example, cell lines Ms704, Ms705 and Ms709 lacking fucose transferase gene (FUT8, ($\alpha$-(1,6)fucosyltransferase)) (see Yamane-Ohnuki et al., (2004) Biotechnol. Bioeng. 87: 614; US Patent No. 20040110704), a CHO cell line Lec13 with reduced ability to attach fucose to Asn (297)-linked sugars (see WO 03/035835), a rat myeloma cell line YB2/0 with little or no activity of adding fucose to N-acetylglucosamine, (which binds to the Fc region of antibodies) (see EP 1176195), and plant cells for the production of antibodies with modified glycosylation patterns (see US Patent No. US20120276086). In addition, cells carrying recombinant genes encoding an enzyme that uses GDP-6-deoxy-D-lyxo-4-hexose as a substrate, such as GDP-6-deoxy-D-lyxo-4-hexose reductase (RMD), can also produce low-fucosylated or non-fucosylated antibodies (see US Patent No. US8642292). In some specific embodiments of the present disclosure, the non-fucosylated antibodies are prepared, for example, by the methods described in Example 5.

"Antibody-dependent cell-mediated cytotoxicity" or "ADCC" refers to a cell-mediated response in which nonspecific cytotoxic cells expressing FcRs (e.g., natural killer (NK) cells, neutrophils, and macrophages) recognize bound antibody on a target cell, resulting in lysis of the target cell. Primary cells and NK cells that regulate ADCC express FcγRIII only, whereas monocytes express FcγRI, FcγRII and FcγRIII. *In vivo* and *in vitro* ADCC assays may be performed to assess ADCC activity of a molecule of interest, such as those described by Clynes et al. (PNAS USA 95: 652-656 (1998)), and in US Patent Nos. US5500362 and US5821337, and the like. In the present disclosure, in some examples, the ADCC is assayed by the method in Test Example 6 of the present disclosure.

"Antibody-dependent cellular phagocytosis" or "ADCP" refers to the mechanism by which antibody-coated target cells or virions are eliminated by internalization of phagocytic cells (e.g., macrophages, neutrophils, and dendritic cells). Internalized antibody-coated target cells or virions are contained in vesicles called phagosomes, which are subsequently fused to one or more lysosomes to form phagolysosomes. ADCP can be assessed by an *in vitro* cytotoxicity assay using macrophages as effector cells and videomicroscopy (e.g., van Bij et al., Journal of Hepatology Vol. 53, No. 4, October 2010, pp677-685). In the present disclosure, in some examples, the ADCP is assayed by the method in Test Example 8 of the present disclosure.

"Complement-dependent cytotoxicity" or "CDC" refers to cytotoxicity in which complement is involved, i.e., a lytic effect on the target cell by a membrane attack complex that is formed by the activation of the classical pathway of complement after binding of an antibody to the corresponding antigen on a cell or virion to form a complex. CDC can be assessed by an *in vitro* assay (e.g., assay on CDC using normal human serum as a source of complement) or in a series of C1q concentrations. A decrease in CDC activity (e.g., a decrease in CDC activity due to the introduction of a second mutation in a polypeptide or antibody) can be determined by comparing the CDC activity of the polypeptide or antibody to the CDC activity of a parent polypeptide or antibody that does not have the second mutation in the same assay. An assay such as that described by Romeuf et al (Romeuf et al., Br J Haematol. 2008 Mar; 140(6): 635-43) can be performed to assess the ability of an antibody to induce CDC. In the present disclosure, in some examples, the CDC is assayed by the method in Test Example 7 of the present disclosure. The term "conjugate", "drug conjugate" or "immunoconjugate" refer to a novel drug formed by linking a stable linker unit to a biologically active drug. For example, an "antibody-drug conjugate" (ADC) is a drug formed by linker a monoclonal antibody or an antibody fragment to a biologically active drug via a stable linker unit. The antibody may be conjugated to the drug directly or via a linker. The average number of drug modules per antibody may range, for example, from about 0 to about 20 drug modules per antibody, in some embodiments, from 1 to about 10 drug modules per antibody, and in some embodiments, from 1 to about 8 drug modules per antibody. In the composition of the mixture of the antibody-drug conjugates of the present disclosure, the mean drug loading per antibody is about 2 to about 5 or about 3 to about 4.

In some embodiments, provided is an immunoconjugate. In some embodiments, the immunoconjugate disclosed herein may be an antibody attached to an effector molecule, wherein the antibody may be an antibody comprising a heavy chain and a light chain. In some embodiments, the antibody may be an antibody fragment, such as a Fab, an Fab', an F(ab')$_2$, an scFv, a dsFv, a ds-scFv, a dimer, a minibody, a diabody, a bispecific antibody fragment, a multimer, and any combination thereof.

In embodiments described herein, the effector molecule may be a radioisotope, an anti-tumor agent, an immunomodulator, a biological response modifier, a lectin, a cytotoxic drug, a chromophore, a fluorophore, a chemiluminescent compound, an enzyme, a metal ion, and any combination thereof.

The antibody or the antibody fragment described herein may be conjugated to the effector molecule by any means. For example, the antibody or the antibody fragment may be chemically or recombinantly attached to the toxin.

Chemical means for preparing fusions or conjugates are known in the art and can be used to prepare immunoconjugates. The method for conjugating the antibody or the antibody fragment and the toxin must be capable of linking the antibody to the toxin without interfering with the ability of the antibody or the antibody fragment to bind to the target molecule.

The term "cytotoxic drug" refers to a substance that inhibits or prevents cell functions and/or causes cell death or cell destruction, including toxins, chemotherapeutic drugs and other compounds that can be used for killing tumor cells.

The term "toxin" refers to any substance capable of exerting a deleterious effect on the growth or proliferation of cells and may be small molecule toxins from bacteria, fungi, plants or animals and derivatives thereof, including camptothecin derivatives (such as exatecan, and maytansinoids and derivatives thereof (CN101573384) (such as DM1, DM3, DM4, and auristatin F (AF) and derivatives thereof (such as MMAF, MMAE, 3024 (WO 2016/127790 A1, compound 7)))), diphtheria toxin, exotoxin, ricin A chain, abrin A chain, modeccin, α-sarcin, *Aleutites fordii* toxic protein, dianthin toxic protein, *Phytolaca americana* toxic protein (PAPI, PAPII and PAP-S), *Momordica charantia* inhibitor, curcin, crotin, sapaonaria officinalis inhibitor, gelonin, mitogellin, restrictocin, phenomycin, enomycin and trichothecenes.

The term "chemotherapeutic agent" refers to a chemical compound that can be used to treat tumors. The definition also includes anti-hormonal agents that act to modulate, reduce, block or inhibit the effects of hormones that can promote cancer growth, and are often in the form of systematic or systemic treatment. They may themselves be hormones. Examples of chemotherapeutic agents include alkylating agents such as thiotepa and cyclosphamide (CYTOXAN™); alkylsulfonates such as busulfan, improsulfan and piposulfan; aziridine such as benaodopa, carboquone, meturedopa and uredopa; aziridine and melamineamine including altretamine, triethylenemelamine, triethylenephosphoramide, triethylenethiophosphoramide and trimethylolomelamine; nitrogen mustards such as chlorambucil, chlornaphazine, cholophosphamide, estramustine, ifosfamide, mechlorethamine and nitromin hydrochloride; melphalan, novembichin, phenesterine, prednimustine, trofosfamide, and uracil mustard; nitrosureas such as carmustine, chlorozotocin, fotemustine, lomustine, nimustine, and ranimustine; antibiotics such as aclacinomycin, actinomycin, authramycin, azaserine, bleomycin, cactinomycin, calicheamicin, carabicin, chromomycin, carzinophilin, chromomycin, actinomycin D, daunorubicin, detorubicin, 6-diazo-5-oxo-L-norleucine, doxorubicin, epirubicin, esorubicin, idarubicin, marcellomycin, mitomycin, mycophenolic acid, nogalamycin, olivomycin, peplomycin, potfiromycin, puromycin, quelamycin, rodorubicin, and streptonigrin; streptozocin, tuberculocidin, ubenimex, zinostatin, and zorubicin; antimetabolites such as methotrexate and 5-fluorouracil (5-FU); folic acid analogs such as denopterin, methotrexate, pteropterin, and trimetrexate; pterine analogs such as fludarabine, 6-mercaptopterin, thiopterin and thioguanterin; pyrimidine analogs such as ancitabine, azacitidine, 6-azauridine, carmofur, cytarabine, dideoxyuridine, doxitluridine, enocitabine, floxuridine, and 5-FU; androgens such as calusterone, dromostanolong propionate, epitiostanol, mepitiostane, and testolactone; antiadrenergics such as aminoglutethimide, mitotane, and trilostane; folic acid supplements such as frolinic acid; acetogluconolactone; aldophosphamideglycoside; aminolevulinic acid; amsacrine; bestrabucil; biasntrene; edatraxate; defofamine; colchicine; diaziquone; elfomithine; elliptinium acetate; etoglucid; gallium nitrate; hydroxyurea; lentinan; lonidamine; mitoguazone; mitoxantrone; mopidamol; nitracrine; pintostatin; phenamet; pirarubicin; podophyllinic acid; 2-ethylhydrazide; procarbazine; PSK®; razoxane; sizofiran; spirogermanium; tenuazonic acid; triaziquone; 2,2',2"-trichlorrotriethylamine; uretha; vindesine; dacarbazine; mannomustine; mitobronitol; mitolactol; pipobroman; gacytosine; arabinoside ("Ara-C"); cyclophosphamide; thiotepa; taxane such as paclitaxel (TAXOL®, Bristol-Myers Squibb Oncology, Princeton, NJ) and docetaxel (TAXOTERE®, Rhone-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunorubicin; aminopterin; xeloda, and ibandronate; CPT-11; topoisomerase inhibitor RFS2000; difluoromethylornithine (DMFO); retinoic acid; esperamicins; capecitabine; and pharmaceutically acceptable salts, acids or derivatives of any one of the above substances. The definition also includes anti-hormonal agents that can modulate or inhibit the effect of hormones on tumors, such as anti-estrogen agents including tamoxifen, raloxifene, the aromatase inhibitor 4(5)-imidazole, 4-hydroxytamoxifene, trioxifene, keoxifene, LY117018, onapristone, and toremifene (Fareston); and anti-androgen agents such as flutamide, nilutamide, bicalutamide, leuprolide and goserelin; and pharmaceutically acceptable salts, acids or derivatives of any one of the above substances.

In one embodiment, both the antibody and toxin are proteins and can be conjugated using techniques well known in the art. There are hundreds of cross-linking agents disclosed in the art that can conjugate two proteins. The cross-linking agent is generally selected based on reactive functional groups available or inserted on the antibody or toxin. Alternatively, if no reactive groups are present, a photo-activatable cross-linking agent may be used. In some cases, it may be desirable to include a spacer between the antibody and the toxin. Cross-linking agents known in the art include homobifunctional agents: glutaraldehyde, dimethyl adipimidate and bis(diazobenzidine), and heterobifunctional agents: *m*-maleimidobenzoyl-*N*-hydroxysuccinimide and sulfo-*m*-maleimidobenzoyl-*N*-hydroxysuccinimide.

Cross-linking agents that can be used to conjugate an effector molecule to an antibody fragment include, for example, TPCH (S-(2-thiopyridyl)-L-cysteine hydrazide) and TPMPH (S-(2-thiopyridyl)mercapto-propionhydrazide). TPCH and TPMPH react on the carbohydrate moiety of the glycoprotein that had previously been oxidized by mild periodate treatment, thereby forming a hydrazone bond between the hydrazide moiety of the crosslinking agent and the aldehyde generated by periodate. The heterobifunctional cross-linking agents GMBS (N-(y-maleimidobutyryloxy)-succinimide) and SMCC (succinimidyl 4-(N-maleimido-methyl)cyclohexane) are reacted with a primary amine, thereby introducing a maleimido group onto the component. This maleimido group may then react with a sulfhydryl group on another component which may be introduced by a cross-linking agent, thereby forming a stable thioether bond between the components. If steric hindrance between the components interferes with the activity of either component, a cross-linking agent may be used to introduce a long spacer between the components, such as N-succinimidyl 3-(2-pyridyldithio)propionate (SPDP). Thus, there are many suitable cross-linking agents that may be used and selected individually depending on their effect on the yield of the optimal immunoconjugate.

The term "expression vector" refers to a nucleic acid molecule capable of transporting another nucleic acid to which it has been linked. In one embodiment, the vector is a "plasmid" that refers to a circular double-stranded DNA loop into which additional DNA segments can be ligated. In another embodiment, the vector is a viral vector in which additional DNA segments may be ligated into the viral genome. The vectors disclosed herein are capable of autonomous replication in a host cell into which they have been introduced (e.g., bacterial vectors having a bacterial origin of replication and episomal mammalian vectors) or being integrated into the genome of a host cell upon introduction into the host cell and thereby replicated along with the host genome (e.g., non-episomal mammalian vectors).

Methods for producing and purifying antibodies and antigen-binding fragments are well known in the art, for example, those described in chapters 5-8 and 15 of "Antibodies: A Laboratory Manual", Cold Spring Harbor Press. For example, mice can be immunized with human CD70 or a fragment thereof, and the obtained antibodies can be renatured and purified, and amino acid sequencing can be performed by using conventional methods. Likewise, antigen-binding fragments can be prepared by conventional methods. The antibody or the antigen-binding fragment described herein is genetically engineered to contain one or more additional human FRs in the non-human CDRs. Human FR germline sequences can be obtained at the website http://imgt.cines.fr of ImMunoGeneTics (IMGT) or from the immunoglobulin journal, 2001ISBN012441351, by comparing the IMGT human antibody variable region germline gene database with the MOE software.

The term "host cell" refers to a cell into which an expression vector has been introduced. Host cells may include bacterial, microbial, plant or animal cells. Bacteria susceptible to transformation include members of the *Enterobacteriaceae* family, such as strains of *Escherichia coli* or *Salmonella*; members of the *Bacillaceae* family, such as *Bacillus subtilis*; *Pneumococcus*; *Streptococcus* and *Haemophilus influenzae.* Suitable microorganisms include *Saccharomyces cerevisiae* and *Pichia pastoris.* Suitable animal host cell lines include CHO (Chinese hamster ovary cell lines), 293 and NS0 cells. To obtain non-fucosylated antibodies, host cells with *Glul* and *Fut8* gene knocked out can be used, including but not limited to CHOK1 cells with *Glul* and *Fut8* gene knockout.

The engineered antibody or antigen-binding fragment of the present disclosure can be prepared and purified by conventional methods. For example, cDNA sequences encoding the heavy and light chains can be cloned and recombined into a GS expression vector. Recombinant immunoglobulin expression vectors can be stably transfected into CHO cells. As a more recommended prior art, mammalian expression systems may result in glycosylation of antibodies, particularly at the highly conserved N-terminal site of the Fc region. Stable clones are obtained by expression of the antibody that specifically binds to human CD70. Positive clones are expanded in a serum-free medium of a bioreactor to produce antibodies. The culture with the secreted antibody can be purified using conventional techniques. For example, purification is carried out on an A or G Sepharose FF column containing an adjusted buffer. Non-specifically bound fractions are washed away. The bound antibody is eluted using pH gradient method, and the antibody fragments are detected by SDS-PAGE and collected. The antibody can be filtered and concentrated by conventional methods. Soluble mixtures and polymers can also be removed by conventional methods, such as molecular sieves and ion exchange. The resulting product needs to be immediately frozen, e.g., at -70 °C, or lyophilized.

"Administrating", "giving" and "treating", when applied to animals, humans, experimental subjects, cells, tissues, organs or biological fluids, refer to contact of an exogenous drug, a therapeutic agent, a diagnostic agent or a composition with the animals, humans, subjects, cells, tissues, organs or biological fluids. "Administrating", "giving" and "treating" can refer to, for example, therapeutic, pharmacokinetic, diagnostic, research and experimental methods. The treatment of the cells comprises making the reagent in contact with the cells and making the reagent in contact with fluid, where the fluid is in contact with the cells. "Administrating", "giving" and "treating" also refer to treating, e.g., cells by reagents, diagnosis, binding compositions or by another cell *in vitro* and *ex vivo.* "Treating", when applied to humans, veterinary or research subjects, refers to therapeutic treatment, preventive or prophylactic measures, and research and diagnostic applications.

"Treatment" refers to administering a therapeutic agent, such as a composition comprising any one of the conjugation compounds of the present disclosure, either internally or externally to a patient with one or more symptoms of a disease on which the therapeutic agent is known to have a therapeutic effect. Generally, the therapeutic agent is administered in an amount effective to alleviate one or more symptoms of the disease in the patient or population being treated to induce regression of such symptoms or inhibiting the development of such symptoms to any clinically measurable degree. The amount of therapeutic agent effective to alleviate the symptoms of any particular disease (also referred to as a "therapeutically effective amount") may vary depending on a variety of factors, such as the disease state, age, and weight of the patient, and the ability of the drug to produce a desired therapeutic effect in the patient. Whether a symptom of a disease has been alleviated can be evaluated by any clinical testing methods commonly used by doctors or other health care professionals to evaluate the severity or progression of the symptom. Although the embodiments of the present disclosure (for example, treatment methods or products) may not be effective in alleviating the symptoms of each disease of interest, they shall reduce the symptoms of a disease of interest in a statistically significant number of patients, as determined according to any statistical testing methods known in the art, such as Student t-test, chi-square test, Mann and Whitney's U test, Kruskal-Wallis test (H test), Jonckheere-Terpstra test and Wilcoxon test.

"Conservative modification" or "conservative replacement or substitution" refers to replacement of amino acids in a protein with other amino acids having similar characteristics (e.g., charge, side-chain size, hydrophobicity/hydrophilicity, or backbone conformation and rigidity), so that changes can be frequently made without changing the biological activity of the protein. Those skilled in the art know that, generally speaking, a single amino acid replacement in a non-essential region of a polypeptide does not substantially change the biological activity (see, e.g., Watson et al. (1987) Molecular Biology of the Gene, The Benj amin/Cummings Pub. Co., p224, (4th edition)). In addition, the replacement of amino acids with similar structure or function is unlikely to disrupt the biological activity. Exemplary conservative substitutions are as follows:

Table 4. Exemplary amino acid conservative substitutions

| Original residue | Conservative substitution |
| --- | --- |
| Ala (A) | Gly; Ser |
| Arg (R) | Lys; His |
| Asn (N) | Gln; His; Asp |
| Asp (D) | Glu; Asn |
| Cys (C) | Ser; Ala; Val |
| Gln (Q) | Asn; Glu |
| Glu (E) | Asp; Gln |
| Gly (G) | Ala |
| His (H) | Asn; Gln |
| Ile (I) | Leu; Val |
| Leu (L) | Ile; Val |
| Lys (K) | Arg; His |
| Met (M) | Leu; Ile; Tyr |
| Phe (F) | Tyr; Met; Leu |
| Pro (P) | Ala |
| Ser (S) | Thr |
| Thr (T) | Ser |
| Trp (W) | Tyr; Phe |
| Tyr (Y) | Trp; Phe |
| Val (V) | Ile; Leu |

"Effective amount" or "effective dose" refers to the amount of a drug, a compound or a pharmaceutical composition necessary to obtain any one or more beneficial or desired therapeutic results. For preventive use, the beneficial or desired results include elimination or reduction of risk, reduction of severity or delay of the onset of a disorder, including the biochemistry, histology and/or behavioral symptoms of the disorder, complications thereof and intermediate pathological phenotypes that appear during the progression of the disorder. For therapeutic applications, the beneficial or desired results include clinical results, such as reducing the incidence of various disorders related to the target antigen of the present disclosure or alleviating one or more symptoms of the disorder, reducing the dosage of other agents required to treat the disorder, enhancing the therapeutic effect of another agent, and/or delaying the progression of disorders of the patient related to the target antigen of the present disclosure.

"Exogenous" refers to substances produced outside organisms, cells or human bodies according to circumstances. "Endogenous" refers to substances produced inside cells, organisms or human bodies according to circumstances.

"Homology" refers to sequence similarity between two polynucleotide sequences or between two polypeptides. When positions in both compared sequences are occupied by the same base or amino acid monomer subunit, e.g., if each position of two DNA molecules is occupied by adenine, then the molecules are homologous at that position.

The homology percentage between two sequences is a function of the number of matching or homologous positions shared by the two sequences divided by the number of positions compared $\times$ 100%. For example, in the optimal alignment of sequences, if 6 out of 10 positions of two sequences are matched or homologous, the two sequences are 60% homologous, and if 95 out of 100 positions of two sequences are matched or homologous, the two sequences are 95% homologous. Generally, two sequences, when aligned, are compared to give the maximum percent homology. For example, the comparison may be made by the BLAST algorithm, wherein the parameters of the algorithm are selected to give the maximum match between the reference sequences over the entire length of each sequence. The following references relate to the BLAST algorithm often used for sequence analysis: the BLAST algorithms: Altschul, S.F. et al., (1990) J. Mol. Biol., 215: 403-410; Gish, W., et al., (1993) Nature Genet., 3: 266-272; Madden, T.L. et al., (1996) Meth. Enzymol., 266: 131-141; Altschul, S.F. et al., (1997) Nucleic Acids Res., 25: 3389-3402; Zhang, J. et al., (1997) Genome Res., 7: 649-656. Other conventional BLAST algorithms, such as one provided by NCBI BLAST, are also well known to those skilled in the art.

As used herein, the expressions "cell", "cell line" and "cell culture" are used interchangeably, and all such designations include their progenies. Therefore, the words "transformant" and "transformed cell" include primary test cells and cultures derived therefrom, regardless of the number of transfers. It should also be understood that all progenies may not be precisely identical in DNA content due to deliberate or unintentional mutations. Mutant progeny with the same function or biological activity as screened in the original transformed cells is included. When referring to different designations, they will become clear through the context.

"Polymerase chain reaction" or "PCR" used herein refers to a procedure or technique in which a trace amount of a specific moiety of nucleic acid, RNA and/or DNA is amplified as described in, for example, US Patent No. 4,683,195. Generally speaking, it is necessary to obtain sequence information from the end or outside of the target region, so that oligonucleotide primers can be designed; these primers are the identical or similar in terms of sequence to the corresponding strand of the template to be amplified. The 5'-terminal nucleotide of 2 primers may coincide with the end of the material to be amplified. PCR can be used to amplify specific RNA sequences, specific DNA sequences from total genomic DNA and cDNA sequences transcribed from total cellular RNA, phage, plasmid sequences, or the like. See generally Mullis, et al., (1987) Cold Spring Harbor Symp. Quant. Biol. 51: 263; Erlich ed. (1989) PCR TECHNOLOGY (Stockton Press, N.Y). The PCR used herein is considered to be an example, but not the only one, of a nucleic acid polymerase reaction method for amplifying a nucleic acid test sample, and the method comprises using known nucleic acids as primers and nucleic acid polymerases to amplify or produce a specific moiety of the nucleic acid.

"Isolated" refers to a purified state, and in this case means that the designated molecule is substantially free of other biomolecules, such as nucleic acids, proteins, lipids, carbohydrates, or other materials (such as cell debris and growth medium). Generally, the term "isolated" does not mean the complete absence of such substances or the absence of water, buffers or salts, unless they are present in amounts that will significantly interfere with the experimental or therapeutic use of the compounds described herein.

The term "optional" or "optionally" means that the event or circumstance subsequently described may, but not necessarily, occur, and that the description includes instances where the event or circumstance occurs or does not occur.

The term "pharmaceutical composition" refers to a mixture containing one or more of the compounds described herein or physiologically/pharmaceutically acceptable salts or prodrugs thereof, and other chemical components, and the other components are, for example, physiologically/pharmaceutically acceptable carriers and excipients. The pharmaceutical composition is intended to promote the administration to an organism, which facilitates the absorption of the active ingredient, thereby exerting biological activities.

The term "pharmaceutically acceptable carrier" refers to any inactive substance suitable for use in formulations for delivery of drugs (e.g., the anti-CD70 antibody or the antigen-binding fragment described herein). The carrier can be an anti-adhesive agent, binder, coating, disintegrant, filler or diluent, preservative (such as antioxidant, antibacterial or antifungal agent), sweetener, absorption delaying agent, wetting agent, emulsifier, buffer, etc. Examples of suitable pharmaceutically acceptable carriers include water, ethanol, polyol (such as glycerol, propanediol and polyethylene glycol), dextrose, vegetable oil (such as olive oil), saline, buffer, buffered saline, and isotonic agents such as sugar, polyol, sorbitol and sodium chloride.

In addition, the present disclosure includes an agent for treating a disease related to target antigen (e.g., CD70)-positive cells, and the agent comprises the anti-CD70 antibody or the antigen-binding fragment thereof of the present disclosure as an active ingredient. The active ingredient is administered to a subject in a therapeutically effective amount to treat a disease related to CD70-positive cells in the subject. The therapeutically effective amount means that a unit dose of the composition comprises 0.1 mg to 3000 mg of the antibody specifically binding to human CD70 described above.

The disease or disorder related to CD70 in the present disclosure is not limited as long as it is a disease or disorder related to CD70. For example, in some embodiments, the molecules of the present disclosure are useful for some of the following diseases or disorders that express CD70: for example, rheumatoid arthritis, autoimmune demyelinating diseases (e.g., multiple sclerosis or allergic encephalomyelitis), endocrine ophthalmopathy, uveoretinitis, systemic lupus erythematosus, myasthenia gravis, Grave's disease, glomerulonephritis, autoimmune hepatological disease, inflammatory bowel diseases (e.g., Crohn's disease, ulcerative colitis, or celiac disease), anaphylaxis, allergic reaction, Sjogren syndrome, type I diabetes, primary biliary cirrhosis, Wegener's granulomatosis, fibromyalgia, polymyositis, dermatomyositis, multiple endocrine deficiencies, Schmidt syndrome, autoimmune uveitis, Addison's disease, adrenalitis, thyroiditis, Hashimoto's thyroiditis, autoimmune thyroid disease, pernicious anemia, gastric atrophy, chronic hepatitis, lupus-like hepatitis, atherosclerosis, subacute cutaneous lupus erythematosus, hypoparathyroidism, Dressier syndrome, autoimmune thrombocytopenia, idiopathic thrombocytopenic purpura, hemolytic anemia, pemphigus vulgaris, pemphigus, dermatitis herpetiformis, alopecia areata, pemphigoid, scleroderma, progressive systemic sclerosis, CREST syndrome (calcium deposition, Raynaud's phenomenon, esophageal motility disorder, scleroderma and telangiectasia), male and female autoimmune infertility, ankylosing spondylitis, ulcerative colitis, mixed connective tissue disease, polyateritis nodosa, systemic necrotizing vasculitis, atopic dermatitis, atopic rhinitis, Goodpasture's syndrome, Chagas's disease, sarcoidosis, rheumatic fever, asthma, recurrent spontaneous abortion, antiphospholipid syndrome, farmer's lung, erythema multiforme, post-cardiotomy syndrome, Gushing syndrome, autoimmune chronic active hepatitis, bird breeder's lung, toxic epidermal necrolysis syndrome, Alport syndrome, alveolitis, allergic alveolitis, fibrotic alveolitis, interstitial lung disease, erythema nodosum, pyoderma gangrenosum, transfusion reaction, Takayasu arteritis, polymyalgia rheumatica, temporal arteritis, schistosomiasis, giant cell arteritis, ascariasis, aspergillosis, Samter's syndrome, eczema, lymphomatoid granulomatosis, Behcet's disease, Caplan's syndrome, kawasaki disease, dengue fever, encephalomyelitis, endocarditis, endomyocardial fibrosis, endophthalmitis, erythema elevatum et diutinum, psoriasis, erythroblastosis fetalis, eosinophilic fasciitis, Shulman's syndrome, Felty's syndrome, filariasis, cyclitis, chronic cyclitis, heterochromic cyclitis, Fuch's cyclitis, IgA nephropathy, Henoch-Schonlein purpura, Graft versus host disease, graft rejective reaction, cardiomyopathy, Eaton-Lambert syndrome, relapsing polychondritis, cryoglobulinemia, Evan syndrome, and autoimmune gonadal failure, disorders of B lymphocytes (e.g., systemic lupus erythematosus, Goodpasture's syndrome, rheumatoid arthritis, and type I diabetes), disorders of Th1 lymphocytes (e.g., rheumatoid arthritis, multiple sclerosis, psoriasis, Sjorgren syndrome, Hashimoto's thyroiditis, Grave's disease, primary biliary cirrhosis, Wegener's granulomatosis, tuberculosis, or graft versus host disease), or disorders of Th2 lymphocytes (e.g., atopic dermatitis, systemic lupus erythematosus, atopic asthma, rhinoconjunctivitis, allergic rhinitis, Omen syndrome, systemic sclerosis, or chronic graft versus host disease), Churg-Strauss syndrome, microscopic polyangiitis, and Takayasu arteritis. In some other embodiments, the molecules of the present disclosure are useful for some of the following diseases (e.g., cancer) that express CD70, including renal cancer (e.g., renal cell carcinoma), breast cancer, brain tumor, chronic or acute leukemia (including acute myeloid leukemia, chronic myeloid leukemia, acute lymphocytic leukemia, chronic lymphocytic leukemia), lymphoma (e.g., Hodgkin's lymphoma and non-Hodgkin's lymphoma, lymphocytic lymphoma, primary CNS lymphoma, T-cell lymphoma), nasopharyngeal cancer, melanoma (e.g., metastatic malignant melanoma), prostate cancer, colon cancer, lung cancer, bone cancer, pancreatic cancer, skin cancer, cancer of the head or neck, cutaneous or intraocular malignant melanoma, uterine cancer, ovarian cancer, rectal cancer, cancer of the anal region, gastric cancer, testicular cancer, uterine cancer, fallopian tube cancer, endometrial cancer, cervical cancer, vaginal cancer, vulvar cancer, esophageal cancer, cancer of the small intestine, cancer of the endocrine system, thyroid cancer, parathyroid cancer, adrenal cancer, soft tissue sarcoma, urethral carcinoma, cancer of the penis, solid tumors of childhood, bladder cancer, cancer of the kidney or ureter, cancer of the renal pelvis, central nervous system (CNS) tumor, tumor angiogenesis, tumors of the spinal cord axis, brain stem glioma, pituitary adenoma, Kaposi's sarcoma, epidermal cancer, squamous cell carcinoma, and mesothelioma. In some other embodiments, the molecules of the present disclosure have good effect on tumor cells characterized by the presence of tumor cells expressing CD70, including, for example, renal cell carcinoma (RCC) such as clear cell RCC, glioblastoma, breast cancer, brain tumor, nasopharangeal cancer, non-Hodgkin's lymphoma (NHL), acute lymphocytic leukemia (ALL), chronic lymphocytic leukemia (CLL), Burkitt's lymphoma, anaplastic large cell lymphoma (ALCL), multiple myeloma, cutaneous T cell lymphoma, nodular small cell lymphoma, lymphocytic lymphoma, peripheral T cell lymphoma, Lennert's lymphoma, immunoblast lymphoma, T cell leukemia/lymphoma (ATLL), adult T cell leukemia (T-ALL), central blastic/central cytolytic (cb/cc) follicular lymphomas cancers, diffuselarge cell lymphoma of B lineage, angioimmunoblastic lymphadenopathy (AILD)-like T cell lymphoma, HIV-associated body cavity based lymphoma, embryonic carcinoma, undifferentiated carcinoma of the nasopharynx (e.g., Schmincke tumor), Castleman's disease, Kaposi's sarcoma, multiple myeloma, Waldenstrom macroglobulinemia, mantle cell lymphoma and other B-cell lymphomas.

In addition, the present disclosure relates to methods for immunodetection or determination of the target antigen (e.g., CD70), reagents for immunodetection or determination of the target antigen (e.g., CD70), methods for immunodetection or determination of cells expressing the target antigen (e.g., CD70) and diagnostic agents for diagnosing diseases related to positive cells of the target antigen (e.g., CD70), which includes the antibody or the antibody fragment of the present disclosure as an active ingredient, which specifically recognizes the target antigen (e.g., human CD70) and binds to the amino acid sequence of the extracellular region or a three-dimensional structure thereof.

In the present disclosure, the method for detection or determination of the amount of the target antigen (e.g., CD70) may be any known method, for example, immunodetection or determination methods.

The immunodetection or determination methods are methods for detecting or determining the amount of antibody or antigen using labeled antigens or antibodies. Examples of immunodetection or determination methods include radioimmunoassay (RIA), enzyme immunoassay (EIA or ELISA), fluorescence immunoassay (FIA), luminescence immunoassay, western blotting, physicochemical methods, etc.

Diseases related to CD70-positive cells (e.g., cells highly expressing CD70) can be diagnosed by detecting or determining cells expressing CD70 with the antibody or the antibody fragment of the present disclosure.

In order to detect cells expressing the polypeptide, known immunodetection methods can be used, preferably immunoprecipitation, fluorescent cell staining, immunohistochemical staining, etc. In addition, fluorescent antibody staining method utilizing the FMAT8100HTS system (Applied Biosystem) can be used.

In the present disclosure, there is no particular limitation for the living sample used for detection or determination of the target antigen (e.g., CD70), as long as it has the possibility of comprising cells expressing the target antigen (e.g., CD70), such as tissue cells, blood, plasma, serum, pancreatic juice, urine, feces, tissue fluid or culture. According to the required diagnostic method, the diagnostic agent containing the monoclonal antibody or the antibody fragment thereof of the present disclosure can also contain reagents for performing antigen-antibody reaction or reagents for detecting the reaction. The reagents for performing the antigen-antibody reaction include buffers, salts, etc. The reagents for detection include reagents commonly used in immunodetection or determination methods, for example labeled second antibodies that recognize the monoclonal antibody, the antibody fragment thereof or the conjugate thereof, and substrates corresponding to the label, etc.

The details of one or more embodiments of the present disclosure are set forth in the specification above. Although any methods and materials similar or identical to those described herein can be used in the practice or testing of the present disclosure, the preferred methods and materials are described below. Other features, objects and advantages of the present disclosure will be apparent from the specification and the claims. In the specification and claims, singular forms include plural referents unless otherwise indicated clearly in the context. Unless defined otherwise, all technical and scientific terms used herein have the general meaning as commonly understood by those of ordinary skilled in the art to which the present disclosure belongs. All the patents and publications cited in the specification are incorporated by reference. The following examples are set forth in order to more fully illustrate the preferred embodiments of the present disclosure. These examples should not be construed in any way as limiting the scope of the present disclosure.

## DETAILED DESCRIPTION

The present disclosure is further described below with reference to the following examples and test examples, which, however, do not limit the present disclosure. In the present disclosure, the experimental methods in the examples and test examples in which specific conditions are not specified are generally performed under conventional conditions such as Antibodies: A Laboratory Manual and Molecular Cloning: A Laboratory Manual by Cold Spring Harbor Laboratory, or under conditions recommended by the manufacturer of the raw material or the goods. The reagents without specific origins indicated are available from the market.

## Examples

### Example 1: Preparation of CD70 Antigen

With UniProt CD70 antigen (human CD70 protein, Uniprot number: P32970) used as a template of CD70, the amino acid sequences of the antigen and the protein for detection used in the present disclosure were designed, and optionally, different tags such as His tag or Fc can be fused on the basis of the CD70 protein. The resulting fragments were separately cloned into a pTT5 vector (Biovector, CAT#102762), transiently expressed in 293 cells, and purified to obtain the antigen and the protein for detection of the present disclosure.

The sequence of a His-tagged CD70 protein extracellular domain (abbreviated as His-TNC-CD70) was used as a detection reagent;

HHHHHH*ACGCAAAPDIKDLLSRLEELEGLVSSLREQ*QRFAQAQQQLPLESLGWDV
AELQLNHTGPQQDPRLYWQGGPALGRSFLHGPELDKGQLRIHRDGIYMVHIQV
TLAICSSTTASRHHPTTLAVGICSPASRSISLLRLSFHQGCTIASQRLTPLARGDTL
CTNLTGTLLPSRNTDETFFGVQWVRP                          SEQ ID NO: 1

Note: the underlined part is the 6×His tag, the italicized part is the TNC tag and the rest is the CD70 protein extracellular domain.

The sequence of a fusion protein of the CD70 protein extracellular domain and Human-IgG1-Fc (abbreviated as CD70-Fc) was used as an immunogen;

QRFAQAQQQLPLESLGWDVAELQLNHTGPQQDPRLYWQGGPALGRSFLHGPEL

DKGQLRIHRDGIYMVHIQVTLAICSSTTASRHHPTTLAVGICSPASRSISLLRLSFH
QGCTIASQRLTPLARGDTLCTNLTGTLLPSRNTDETFFGVQWVRPEPKSSDKTHT
CPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAP
IEKTISKAKGQPREPQVYTLPPSRDELTKNQVSLTCLVKGFYPSDIAVEWESNGQ
PENNYKTTPPVLDSDGSFFLYSKLTVDKSRWQQGNVFSCSVMHEALHNHYTQK
SLSLSPGK                                           SEQ ID NO: 2

Note: the underlined part is the Human-IgG1-Fc, and the non-underlined part is the CD70 protein extracellular domain.

**Example 2: Purification of CD70-Related Recombinant Proteins**

*1. Purification of His-tagged recombinant protein:*

The cell expression supernatant sample was centrifuged at high speed to remove impurities, and buffer-exchanged with PBS, followed by the addition of imidazole to make a final concentration of 5 mM. A nickel column was equilibrated with a PBS solution containing 5 mM imidazole and washed with 2-5 column volumes. The cell supernatant sample after buffer exchange was loaded on the Ni Sepharose excel column (GE, 17-3712-02). The column was washed with a PBS solution containing 5 mM imidazole until $A_{280}$ reading dropped to baseline. The chromatographic column was then washed with a mixture of PBS and 10 mM imidazole to remove non-specifically bound impure proteins, and the effluent was collected. The target protein was eluted with a PBS solution containing 300 mM imidazole and the elution peaks were collected. The collected eluate was concentrated and further purified using a gel chromatographic column Superdex200 (GE, 28-9893-35) with PBS as mobile phase. The polymer peaks were removed and the elution peak was collected. The obtained protein was identified by electrophoresis, peptide mapping and LC-MS, and then aliquoted for later use if it was determined to be correct. His-tagged His-TNC-CD70 was obtained for use as a detection reagent for the antibodies of the present disclosure.

*2. Purification of CD70-Fc fusion protein:*

The cell expression supernatant sample was centrifuged at high speed to remove impurities, and the supernatant was subjected to MabSelect Sure (GE, 17-5438-01) affinity chromatography. The MabSelect Sure chromatographic column was firstly regenerated with 0.2 M NaOH, then washed with pure water, and equilibrated with PBS. After the supernatant was bound, the column was washed with PBS until the A280 reading dropped to baseline. The target protein was eluted with 0.1 M acetate buffer at pH 3.5 and neutralized with 1 M Tris-HCl. The elution sample was properly concentrated and further purified using the gel chromatographic column Superdex200 (GE, 28-9893-35) equilibrated with PBS, and the target protein was concentrated to the appropriate concentration in the receiver tube where the target protein was collected. This method was used to purify a CD70-Fc fusion protein, and can also be used to purify the antibody proteins of the present disclosure.

**Example 3: Screening of Murine Anti-CD70 Phage Library Antibodies**

*1. Immunization of mice*

Anti-human CD70 antibodies were generated by immunizing mice. Laboratory Balb/c white mice, female, 6-8 weeks of age (Beijing Vital River Laboratory Animal Technology Co., Ltd., animal production license number: SCXK (Beijing) 2012-0001). Housing environment: SPF grade. The purchased mice were fed in a laboratory environment for 1 week, in a 12/12 hour light/dark cycle, at a temperature of 20-25 °C, with humidity at 40-60%. The acclimatized mice were immunized according to the following scheme. The mice were cross-immunized with a protein antigen (CD70-Fc, SEQ ID NO: 2) and a cell antigen (CHO-S cell expressing human full-length CD70 (Invitrogen, R80007)), wherein TiterMax® Gold Adjuvant (Sigma Cat No. T2684) and Thermo Imject® Alum (Thermo Cat No. 77161) adjuvant were alternatively used for the protein antigen. The ratio of the protein antigen to the adjuvant (TiterMax® Gold Adjuvant) was 1:1, the ratio of the protein antigen to the adjuvant (Thermo Imject® Alum) was 3: 1, and the doses were as follows: 50 $\mu$g/mouse/time (protein antigen immunization) and $1\times10^7$ cells/mouse/time (cell antigen immunization). The protein antigen was used for inoculation after being emulsified, and the cell antigen was used for inoculation after being resuspended in a phosphate buffer solution. The inoculation was performed on days 0, 14, 28, 42, 56 and 70, and blood was collected on days 21, 49 and 82. The antibody titer in the mouse serum was determined by ELISA. Mice with high antibody titers in serum and titers approaching a plateau were selected and their spleens were taken for establishing an immune repertoire.

*2. Construction of murine phage single-chain antibody library*

Spleen cells of mice were taken, from which total RNA was extracted using Trizol (Invitrogen Cat No. 15596-018). Reverse transcription was performed using PrimeScript™ II 1st Strand cDNA Synthesis Kit (Takara Cat No. 6210A) to obtain cDNA. Primers for constructing the library were designed and synthesized according to the IMGT database (Genewiz). A single-chain antibody fragment was obtained by three rounds of PCR reactions. LA Tag (Takara Cat No. RR02MB) was used for all PCR reactions. In the first round of PCR, cDNA was used as a template, and sequences of a heavy chain variable region and a light chain variable region were obtained by respective amplification; in the second round of PCR, the products from the first round of PCR were used as templates, the Sfi 1 enzymatic digestion site sequence was introduced at the 5' end of the heavy chain variable region and the 3' end of the light chain variable region, and the ligation sequence was introduced at the 3' end of the heavy chain variable region and the 5' end of the light chain variable region; in the third round of PCR, the heavy chain variable region and the light chain variable region of the products from the second round of PCR were used as templates, and over lapextension PCR was performed to obtain the single-chain antibody fragment with the heavy chain variable region in the front and the light chain variable region in the back.

The single-chain antibody fragment and a modified library construction vector pCantab5E (Amersham Biosciences/GE Cat No. 27-9400-01) were digested by Sfi1 (NEB Cat No. R0123L), and the fragments were purified and extracted using E.Z.N.A.® Gel Extraction Kit (Omega Cat No. D2500-02) after electrophoresis. Then, they were ligated with T4 DNA ligase (NEB Cat No. M0202L) at 16 °C for 16-18 h, purified and extracted with the above kit, and finally eluted with deionized water. 1 $\mu$g of the ligation product was mixed with 1 tube of electrotransformation competent TG1 (Lucigen Cat No. 60502-2) and the mixture was electrotransformed with an electroporator (Bio Rad Micropulser). After the transformation was repeated 20 times, the cells were plated and cultured in an inverted state at 37 °C for 16-18 h. All colonies were scraped off and mixed together, added with glycerol at a final concentration of 15%, and stored at -80 °C for later use.

*3. Acquisition of positive monoclonal sequence of anti-CD70 by screening phage single-chain antibody library*

After the phage single-chain antibody library was packaged and concentrated, two rounds of panning were performed firstly. The phage library ($1\times10^{12}$-$1\times10^{13}$/pfu) was suspended in 1 mL of 2% MPBS (PBS containing 2% of skim milk powder), and then 100 $\mu$L of Dynabeads® M-280 Streptavidin (Invitrogen, Cat No. 11206D) was added. The tube was placed on a rotating plate and flipped up and down repeatedly, and then blocked at room temperature for 1 h. The tube was then placed on a magnetic rack and left to stand for 2 min, the Dynabeads were removed, and then the phage library was transferred to a new tube. 2 $\mu$g/mL biotin-labeled His-TNC-CD70 was added to the blocked phage library, and the tube was placed on the rotating plate and flipped up and down repeatedly for 1 h. Meanwhile, 100 $\mu$L of Dynabeads were suspended in 1 mL of 2% MPBS, and the tube was placed on a rotating plate and flipped up and down repeatedly, and blocked at room temperature for 1 h. The tube was then placed on a magnetic rack and left to stand for 2 min, and the blocking solution was pipetted off. The blocked Dynabeads were added to the mixed solution of the phage library and His-TNC-CD70, and the tube containing the resulting mixture was placed on a rotating plate and flipped up and down repeatedly for 15 min. The tube was then placed on a magnetic rack and left to stand for 2 min, and the mixed solution was pipetted off. Dynabeads were eluted with 1 mL of PBST (PBS containing 0.1% Tween-20), and 0.5 mL of 1 mg/mL trypsin (Sigma Cat No. T1426-250MG) was added. The tube was flipped up and down repeatedly and incubated on a rotating plate for 15 min, and elution was performed. The eluted phages were directly used to infect *E. coli* TG1 in the logarithmic growth phase, determined for the titer and amplified and concentrated for the next round of panning. In the second round of panning of the phage library ($1\times10^{11}$-$1\times10^{12}$/pfu), the concentration of biotin-labeled human His-TNC-CD70 was reduced to 1 $\mu$g/mL, and the number of PBST washes was increased to 15. The eluted phages were used to infect *E. coli* TG1, which was then plated, and single clones were randomly picked for phage ELISA.

The clones were seeded into a 96-well deep-well plate (Nunc Cat No. 260251) and incubated at 37 °C for 16-18 h. A small number of the incubated clones were seeded into another 96-well deep-well plate until the $OD_{600}$ reached about 0.5, and M13K07 helper phages (NEB, Cat No. N0315S) were then added for packaging. The mixture was centrifuged at 4000 g for 10 min to remove cell lysate and the culture solution was pipetted out for ELISA assay of human CD70 binding. The positive clone strains were promptly cryopreserved and sent to a sequencing company for sequencing. The obtained amino acid sequences corresponding to the DNA sequences of the positive clones B1, B7 and F4 are as follows:

> Heavy chain variable region sequence of B1 (B1 mVH):

*QVQMQQSGAELVKPGASVMMSCKASGYTFT*<u>TYNIH</u>*WIKQTPGQGLEWIG*<u>DIYPGN GDASYNQKFRD</u>*RATLTADRSSSTAYLQLSSLTSEDSAIYYCAT*<u>FSRFDGWFAY</u>*WGQG TLVTVSA*

SEQ ID NO: 3

> Light chain variable region sequence of B1 (B1 mVL):

*DIVMSQSPASLAVSLGQRATISC*<u>RASRSVSTSGYSYMH</u>*WYQQKPGQPPKLLIY*<u>LASN LES</u>*GVPARFSGSGSGTNFTLNIHPVEEEDAATYYC*<u>QHSRELPYT</u>*FGGGTKLEIK*

SEQ ID NO: 4

> Heavy chain variable region sequence of B7 (B7 mVH):

*QIQLVQSGPELKKPGETVKISCKTSGYTFT*<u>NYGMN</u>*WVRQAPGKGLKWMG*<u>WINTYT GEPTYADDFKG</u>*RFAFSLETSAGTAYLQINNLENEDTATYFCAR*<u>GDSFTTEILRNWYF DV</u>*WGAGTT*

SEQ ID NO: 5

> Light chain variable region sequence of B7 (B7 mVL):

*DIQMTQTPASLSASVGETVTITC*<u>GASENIYGALN</u>*WYQRKQGKSPQLLIY*<u>GATNLADG</u> *MSSRFSGSGSGRQYSLKISSLHPDDVATYYC*<u>QNVLSTPWT</u>*FGGGTKLEMK*

SEQ ID NO: 6

> Heavy chain variable region sequence of F4 (F4 mVH):

*QVQLKQSGAELVKPGASVKMSCKASGDTFP*<u>RYNMH</u>*WLKQTPGQGLEWIG*<u>AIFPGN GETSYNQNFKG</u>*KATLTADKSSSTAYMQLNSLTSEDSAVYYCAR*<u>NSYYDYAWFTY</u>*WG QGTLVTVSA*

SEQ ID NO: 7

> Light chain variable region sequence of F4 (F4 mVL):

*DIVMTQSPASLDVSLGQRATISC*<u>RASKSVSSSGYSFMH</u>*WYQQKPGQPPKLLIS*<u>LASN LES</u>*GVPARFSGSGSGTDFTLTIYPVEEEDAATYYC*<u>QHSREFPPT</u>*FGSGTKL*EIK

SEQ ID NO: 8

Note: in the above sequences, the regions are arranged in the following order: FR1-CDR1-FR2-CDR2-FR3-CDR3-FR4, the underlined part is a CDR sequence determined according to the Kabat numbering scheme, and the italicized part is an FR sequence.

The CDR sequences of the heavy chain and light chains of murine antibodies B1, B7 and F4 are shown in Table 5 below:

Table 5. CDR sequences of heavy chain and light chains of antibody

| Antibod y | Heavy chain | | Light chain | |
|---|---|---|---|---|
| B1 | HCDR 1 | TYNIH SEQ ID NO: 9 | LCDR 1 | RASRSVSTSGYSYM H SEQ ID NO: 12 |
| | HCDR 2 | DIYPGNGDASYNQKFR D SEQ ID NO: 10 | LCDR 2 | LASNLES SEQ ID NO: 13 |
| | HCDR 3 | FSRFDGWFAY SEQ ID NO: 11 | LCDR 3 | QHSRELPYT SEQ ID NO: 14 |
| B7 | HCDR 1 | NYGMN SEQ ID NO: 15 | LCDR 1 | GASENIYGALN SEQ ID NO: 18 |
| | HCDR 2 | WINTYTGEPTYADDFK G SEQ ID NO: 16 | LCDR 2 | GATNLAD SEQ ID NO: 19 |
| | HCDR 3 | GDSFTTEILRNWYFDV SEQ ID NO: 17 | LCDR 3 | QNVLSTPWT SEQ ID NO: 20 |
| F4 | HCDR 1 | RYNMH SEQ ID NO: 21 | LCDR 1 | RASKSVSSSGYSFMH SEQ ID NO: 24 |
| | HCDR 2 | AIFPGNGETSYNQNFKG SEQ ID NO: 22 | LCDR 2 | LASNLES SEQ ID NO: 13 |
| | HCDR 3 | NSYYDYAWFTY SEQ ID NO: 23 | LCDR 3 | QHSREFPPT SEQ ID NO: 25 |
| Note: the CDRs in the table are CDRs determined according to the Kabat numbering scheme | | | | |

#### 4. ELISA assay on the binding of phage display single-chain antibodies to human CD70 protein

Human His-TNC-CD70 protein was diluted to a concentration of 2 $\mu$g/mL with a PBS buffer at pH 7.4, added to a 96-well microplate (Corning, Cat No. CLS3590-100EA) at a volume of 100 $\mu$L/well, and placed in a refrigerator at 4 °C for 16-18 h. After the liquid was discarded, a blocking solution, 5% skim milk powder (Sangon Biotech, product No. A600669-0250) diluted with PBS, was added at 200 $\mu$L/well, and the plate was incubated at 37 °C for 2 h for blocking. After the blocking was completed, the blocking solution was discarded, and the plate was washed 3 times with a PBST buffer (pH 7.4, PBS containing 0.1% tween-20), followed by the addition of phage culture diluted at a 1:1 ratio with 2% MPBS (pH 7.4, PBS containing 2% skim milk powder) at 100 $\mu$L/well. The plate was incubated at 37 °C for 1 h in an incubator. After the incubation was completed, the plate was washed 6 times with PBST, followed by the addition of anti-M13 antibody (HRP) secondary antibody (SB, Cat. No. 11973-MM05T-H) diluted with 2% MPBS at 100 $\mu$L/well. The plate was incubated at 37 °C for 1 h. The plate was washed 6 times with PBST, followed by the addition of TMB chromogenic substrate (KPL, Cat No. 52-00-03) at 50 $\mu$L/well. The plate was incubated at room temperature for 5-10 min, and then 1 M $H_2SO_4$ was added at 50 $\mu$L/well to stop the reaction. The absorbance values were read at a wavelength of 450 nm using a VERSAmax microplate reader (Molecular Devices). The experimental results are shown in the table below.

Table 6. Experimental results of the binding of single-chain antibodies to human CD70 antigen by ELISA

| Antibody | Reading at $OD_{450}$ nm of microplate reader |
|---|---|
| B1 | 1.16 |
| F4 | 1.09 |
| B7 | 1.97 |

#### Example 4: Humanization of Anti-CD70 Murine Antibodies

By comparing the IMGT human antibody heavy and light chain variable region germline gene database with the MOE software, heavy and light chain variable region germline genes with high homology to B1, B7 and F4 were selected as templates, and CDRs of the three murine antibodies were grafted into corresponding humanized templates to form variable region sequences in the following order: FR1-CDR1-FR2-CDR2-FR3-CDR3-FR4. Illustratively, the amino acid residues of CDRs in the specific examples below were determined and annotated by the Kabat numbering scheme.

#### 1. Humanization of murine antibody B1

For the murine antibody B1, humanized light chain templates were IGKV4-1*01 and IGKJ4*01, and humanized heavy chain templates were IGHV1-46*01 and IGHJ1*01. CDRs of the murine antibody B1 were grafted into their humanized templates, and amino acids of the FR portions of the humanized antibody were subjected to back mutation engineering, wherein the FR portion of the light chain included one or more back mutations of 5S or 70N (wherein the positions of the back mutation sites were determined according to the Kabat numbering scheme), and the FR portion of the heavy chain included one or more back mutations of 4M, 37I, 38K, 48I, 67A, 69L, 71A, 73R, 78A, 80L and 94T (wherein the positions of the back mutation sites were determined according to the Kabat numbering scheme). The back mutation design for the humanized antibodies of the antibody B1 is shown in Table 7 below:

Table 7. Back mutation design for humanized antibodies of B1

| VL | | VH | |
|---|---|---|---|
| huB1VL1 | Graft | huB1VH1 | Graft |
| huB1VL2 | Graft + T5S, D70N | huB1VH2 | Graft + L4M, R94T |
| | | huB1VH3 | Graft + L4M, R71A, T73R, R94T |
| | | huB1VH4 | Graft + L4M, M69L, R71A, T73R, V78A, R94T |
| | | huB1VH5 | Graft + L4M, M48I, V67A, M69L, R71A, T73R, V78A, M80L, R94T |

(continued)

| VL | | VH | |
|---|---|---|---|
| | | huB1VH6 | Graft + L4M, V37I, R38K, M48I, V67A, M69L, R71A, T73R, V78A, M80L, R94T |
| Note: Graft indicates that CDRs of the murine antibody were grafted into the human germline FR regions; wherein the positions of the back mutation sites were determined according to the Kabat numbering scheme, for example, "T5S" indicates that T at position 5 was mutated to S according to the Kabat numbering scheme. | | | |

Light chain variable region/heavy chain variable region sequences of humanized antibodies of B1 are as follows:

> huB1VH1 (huB1VH-graft)

*EVQLVQSGAEVKKPGASVKVSCKASGYTFT*TYNIH*WVRQAPGQGLEWMG*DIYPGN
GDASYNQKFRD*RVTMTRDTSTSTVYMELSSLRSEDTAVYYCAR*FSRFDGWFAY*WGQ
GTLVTVSS*

SEQ ID NO: 26

> huB1VH2

*EVQMVQSGAEVKKPGASVKVSCKASGYTFT*TYNIH*WVRQAPGQGLEWMG*DIYPG
NGDASYNQKFRD*RVTMTRDTSTSTVYMELSSLRSEDTAVYYCA**T**FSRFDGWFAY*WG
QGTLVTVSS*

SEQ ID NO: 27

> huB1VH3

*EVQMVQSGAEVKKPGASVKVSCKASGYTFT*TYNIH*WVRQAPGQGLEWMG*DIYPG
NGDASYNQKFRD*RVTMTAD**R**STSTVYMELSSLRSEDTAVYYCA**T**FSRFDGWFAY*W
GQGTLVTVSS*

SEQ ID NO: 28

> huB1VH4

*EVQMVQSGAEVKKPGASVKVSCKASGYTFT*TYNIH*WVRQAPGQGLEWMG*DIYPG
NGDASYNQKFRD*RVT**L**TAD**R**STSTAYMELSSLRSEDTAVYYCA**T**FSRFDGWFAY*WG
QGTLVTVSS*

SEQ ID NO: 29

> huB1VH5

*EVQMVQSGAEVKKPGASVKVSCKASGYTFT*<u>TYNIH</u>*WVRQAPGQGLEWIG*<u>DIYPGN</u>
<u>GDASYNQKFRD</u>*RATLTADRSTSTAYLELSSLRSEDTAVYYCAT*<u>FSRFDGWFAY</u>*WGQ*
*GTLVTVSS*

SEQ ID NO: 30

> huB1VH6

*EVQMVQSGAEVKKPGASVKVSCKASGYTFT*<u>TYNIH</u>*WIKQAPGQGLEWIG*<u>DIYPGN</u>
<u>GDASYNQKFRD</u>*RATLTADRSTSTAYLELSSLRSEDTAVYYCAT*<u>FSRFDGWFAY</u>*WGQ*
*GTLVTVSS*

SEQ ID NO: 31

> huB1VL1 (huB1VL-graft)

*DIVMTQSPDSLAVSLGERATINC*<u>RASRSVSTSGYSYMH</u>*WYQQKPGQPPKLLIY*<u>LAS</u>
<u>NLES</u>*GVPDRFSGSGSGTDFTLTISSLQAEDVAVYYC*<u>QHSRELPYT</u>*FGGGTKVEIK*

SEQ ID NO: 32

> huB1VL2

*DIVMSQSPDSLAVSLGERATINC*<u>RASRSVSTSGYSYMH</u>*WYQQKPGQPPKLLIY*<u>LAS</u>
<u>NLES</u>*GVPDRFSGSGSGTNFTLTISSLQAEDVAVYYC*<u>QHSRELPYT</u>*FGGGTKVEIK*

SEQ ID NO: 33

In addition, individual amino acids of CDR portions of the light chain variable region and the heavy chain variable region were modified, wherein the amino acid sequence of HCDR2 was modified from <u>DIYPGNGDASYNQKFRD</u> (set forth in SEQ ID NO: 10) to <u>DIYPGTGDASYNQKFRD</u> (set forth in SEQ ID NO: 42), and the amino acid sequence of LCDR2 was modified from <u>LASNLES</u> (set forth in SEQ ID NO: 13) to: <u>LADNLES</u> (set forth in SEQ ID NO: 43). The sequences of the light chain variable region/heavy chain variable region of the humanized antibodies of B1 after modification are as follows:

> huB1VH1-1

*EVQLVQSGAEVKKPGASVKVSCKASGYTFT*<u>TYNIH</u>*WVRQAPGQGLEWMG*<u>DIYPGT</u>
<u>GDASYNQKFRD</u>*RVTMTRDTSTSTVYMELSSLRSEDTAVYYCAR*<u>FSRFDGWFAY</u>*WGQ*
*GTLVTVSS*

SEQ ID NO: 34

> huB1VH2-1

*EVQMVQSGAEVKKPGASVKVSCKASGYTFT<u>TYNIH</u>WVRQAPGQGLEWMG<u>DIYPGT</u>*
*<u>GDASYNQKFRD</u>RVTMTRDTSTSTVYMELSSLRSEDTAVYYCA<u>T</u><u>FSRFDGWFAY</u>WGQ*
*GTLVTVSS*

SEQ ID NO: 35

> huB1VH3-1

*EVQMVQSGAEVKKPGASVKVSCKASGYTFT<u>TYNIH</u>WVRQAPGQGLEWMG<u>DIYPGT</u>*
*<u>GDASYNQKFRD</u>RVTMTADRSTSTVYMELSSLRSEDTAVYYCA<u>T</u><u>FSRFDGWFAY</u>WG*
*QGTLVTVSS*

SEQ ID NO: 36

> huB1VH4-1

*EVQMVQSGAEVKKPGASVKVSCKASGYTFT<u>TYNIH</u>WVRQAPGQGLEWMG<u>DIYPGT</u>*
*<u>GDASYNQKFRD</u>RVTLTADRSTSTAYMELSSLRSEDTAVYYCA<u>T</u><u>FSRFDGWFAY</u>WGQ*
*GTLVTVSS*

SEQ ID NO: 37

> huB1VH5-1

*EVQMVQSGAEVKKPGASVKVSCKASGYTFT<u>TYNIH</u>WVRQAPGQGLEWIG<u>DIYPGT</u>*

*<u>GDASYNQKFRD</u>RATLTADRSTSTAYLELSSLRSEDTAVYYCA<u>T</u><u>FSRFDGWFAY</u>WGQ*
*GTLVTVSS*

SEQ ID NO: 38

> huB1VH6-1

*EVQMVQSGAEVKKPGASVKVSCKASGYTFT<u>TYNIH</u>WIKQAPGQGLEWIG<u>DIYPGT</u>*
*<u>GDASYNQKFRD</u>RATLTADRSTSTAYLELSSLRSEDTAVYYCA<u>T</u><u>FSRFDGWFAY</u>WGQ*
*GTLVTVSS*

SEQ ID NO: 39

> huB1VL1-1

*DIVMTQSPDSLAVSLGERATINC<u>RASRSVSTSGYSYMH</u>WYQQKPGQPPKLLIY<u>LAD</u>*
*<u>NLES</u>GVPDRFSGSGSGTDFTLTISSLQAEDVAVYYC<u>QHSRELPYT</u>FGGGTKVEIK*

SEQ ID NO: 40

> huB1VL2-1

*DIVMSQSPDSLAVSLGERATINC*<u>RASRSVSTSGYSYMH</u>*WYQQKPGQPPKLLIY*<u>LA**D**</u>
<u>NLES</u>*GVPDRFSGSGSGTNFTLTISSLQAEDVAVYYC*<u>QHSRELPYT</u>*FGGGTKVEIK*

SEQ ID NO: 41

In the above sequences, the regions are arranged in the following order: FR1-CDR1-FR2-CDR2-FR3-CDR3-FR4, the italicized part is an FR sequence, and the underlined part is a CDR sequence determined according to the Kabat numbering scheme.

### 2. Selection of humanized framework of antibody library huB7

For the murine antibody B7, humanized light chain templates were IGKV27-1*01 and IGKJ4*01, and humanized heavy chain templates were IGHV7-4-1*02 and IGHJ6*01. CDRs of the murine antibody B7 were grafted into their humanized templates, and amino acids of the FR portions of the humanized antibody were subjected to back mutation engineering, wherein the FR portion of the light chain included one or more back mutations of 38R, 43S, 69R, 70Q and 71Y (wherein the positions of the back mutation sites were determined according to the Kabat numbering scheme), and the FR portion of the heavy chain included one or more back mutations of 2I, 24T, 46K, 72E and 82a N (wherein the positions of the back mutation sites were determined according to the Kabat numbering scheme). The back mutation design for variable regions of humanized antibodies of the antibody B7 is shown in Table 8 below:

Table 8. Back mutation design for humanized antibodies of B7

| VL | | VH | |
|---|---|---|---|
| huB7VL1 | Graft | huB7VH1 | Graft |
| huB7VL2 | Graft + T69R, F71Y | huB7VH2 | Graft + V2I, A24T, D72E |
| huB7VL3 | Graft + V43S, T69R, F71Y | huB7VH3 | Graft + V2I, A24T, E46K, D72E, S82a N |
| huB7VL4 | Graft + Q38R, V43S, T69R, D70Q, F71Y | | |

Note: Grafted indicates that CDRs of the murine antibody were grafted into the human germline FR regions; wherein the positions of the mutation sites were determined according to the Kabat numbering scheme, for example, "S82a N" indicates that S at position 82a (also called 82A) was mutated to N according to the Kabat numbering scheme.

Light/heavy chain variable region sequences of humanized antibodies of B7 are as follows:

> huB7VH1 (huB7VH-graft)

*EVQLVQSGSELKKPGASVKVSCKASGYTFT*<u>NYGMN</u>*WVRQAPGQGLEWMG*<u>WINTY</u>
<u>TGEPTYADDFKG</u>*RFVFSLDTSVSTAYLQISSLKAEDTAVYYCAR*<u>GDSFTTEILRNWYF</u>
<u>DV</u>*WGQGTTVTVSS*

SEQ ID NO: 44

> huB7VH2

*EI QLVQSGSELKKPGASVKVSCKT SGYTFT*<u>NYGMN</u>*WVRQAPGQGLEWMG*<u>WINTY</u>
<u>TGEPTYADDFKG</u>*RFVFSL**E**TSVSTAYLQISSLKAEDTAVYYCAR*<u>GDSFTTEILRNWYF</u>
<u>DV</u>*WGQGTTVTVSS*

SEQ ID NO: 45

> huB7VH3

*EIQLVQSGSELKKPGASVKVSCK**T**SGYTFT*<u>NYGMN</u>*WVRQAPGQGL**K**WMG*<u>WINTY</u>
<u>TGEPTYADDFKG</u>*RFVFSL**E**TSVSTAYLQINSLKAEDTAVYYCAR*<u>GDSFTTEILRNWYF</u>
<u>DV</u>*WGQGTTVTVSS*

SEQ ID NO: 46

> huB7VL1 (huB7VL-graft)

*DIQMTQSPSSLSASVGDRVTITC*<u>GASENIYGALN</u>*WYQQKPGKVPKLLIY*<u>GATNLAD</u>
*GVPSRFSGSGSGTDFTLTISSLQPEDVATYYC*<u>QNVLSTPWT</u>*FGGGTKVEIK*

SEQ ID NO: 47

> huB7VL2

*DIQMTQSPSSLSASVGDRVTITC*<u>GASENIYGALN</u>*WYQQKPGKVPKLLIY*<u>GATNLAD</u>
*GVPSRFSGSGSG**RD**YTLTISSLQPEDVATYYC*<u>QNVLSTPWT</u>*FGGGTKVEIK*

SEQ ID NO: 48

> huB7VL3

*DIQMTQSPSSLSASVGDRVTITC*<u>GASENIYGALN</u>*WYQQKPGK**S**PKLLIY*<u>GATNLAD</u>
*GVPSRFSGSGSG**RD**YTLTISSLQPEDVATYYC*<u>QNVLSTPWT</u>*FGGGTKVEIK*

SEQ ID NO: 49

> huB7VL4

*DIQMTQSPSSLSASVGDRVTITC*<u>GASENIYGALN</u>*WYQ**R**KPGK**S**PKLLIY*<u>GATNLAD</u>*G
VPSRFSGSGSG**RQ**YTLTISSLQPEDVATYYC*<u>QNVLSTPWT</u>*FGGGTKVEIK*

SEQ ID NO: 50

In addition, individual amino acids of CDR portions of the heavy chain variable region were modified, wherein the amino acid sequence of HCDR2 was modified from the original <u>WINTYTGEPTYADDFKG</u> (set forth in SEQ ID NO: 16) to: WINTYTGEPTYAD**E**FKG (set forth in SEQ ID NO: 54), the heavy chain variable region sequence of the humanized antibodies of B7 after modification is as follows:

> huB7VH1-1

*EVQLVQSGSELKKPGASVKVSCKASGYTFT*<u>NYGMN</u>*WVRQAPGQGLEWMG*<u>WINTY</u>
<u>TGEPTYAD**E**FKG</u>*RFVFSLDTSVSTAYLQISSLKAEDTAVYYCAR*<u>GDSFTTEILRNWYF</u>
<u>DV</u>*WGQGTTVTVSS*

SEQ ID NO: 51

> huB7VH2-1

*EIQLVQSGSELKKPGASVKVSCK**T**SGYTFT*<u>NYGMN</u>*WVRQAPGQGLEWMG*<u>WINTY TGEPTYAD**E**FK**G**</u>*RFVFSL**E**TSVSTAYLQISSLKAEDTAVYYCAR*<u>GDSFTTEILRNWYF DV</u>*WGQGTTVTVSS*

<div align="right">SEQ ID NO: 52</div>

> huB7VH3-1

*EI**Q**LVQSGSELKKPGASVKVSCK**T**SGYTFT*<u>NYGMN</u>*WVRQAPGQGL**K**WMG*<u>WI NTYTGEPTYAD**E**FK**G**</u>*RFVFSL**E**TSVSTAYLQIN*SLKAEDTAVYYCAR<u>GDSFTTEILRN WYFDV</u>*WGQGTTVTVSS*

<div align="right">SEQ ID NO: 53</div>

In the above sequences, the regions are arranged in the following order: FR1-CDR1-FR2-CDR2-FR3-CDR3-FR4, the italicized part is an FR sequence, and the underlined part is a CDR sequence determined according to the Kabat numbering scheme.

### 3. Selection of humanized framework of antibody library huF4

For the murine antibody F4, humanized light chain templates were IGKV4-1*01 and IGKJ2*01, and humanized heavy chain templates were IGHV1-69*08 and IGHJ1*01. CDRs of the murine antibody F4 were grafted into their humanized templates, and amino acids of the FR portions of the humanized antibody were subjected to back mutation engineering, wherein the FR portion of the light chain included the 49S back mutation (wherein the position of the back mutation site was determined according to the Kabat numbering scheme), and the FR portion of the heavy chain included one or more back mutations of 27D, 30P, 37L, 38K, 48I, 66K, 67A, 69L and 82a N (wherein the positions of the back mutation sites were determined according to the Kabat numbering scheme). The back mutation design for variable regions of the humanized antibodies of the antibody F4 is shown in Table 9 below:

<div align="center">Table 9. Back mutation design for humanized antibodies of F4</div>

| VL | | VH | |
|---|---|---|---|
| huF4VL 1 | Graft | huF4VH1 | Graft |
| huF4VL2 | Graft + Y49S | huF4VH2 | Graft + G27D, S30P |
| | | huF4VH3 | Graft + G27D, S30P, I69L |
| | | huF4VH4 | Graft + G27D, S30P, V37L, R38K, I69L |
| | | huF4VH5 | Graft + G27D, S30P, V37L, R38K, R66K, I69L, S82a N |
| | | huF4VH6 | Graft + G27D, S30P, V37L, R38K, M48I, R66K, V67A, I69L, S82a N |
| Note: Grafted indicates that CDRs of the murine antibody were grafted into the human germline FR regions; wherein the positions of the back mutation sites were determined according to the Kabat numbering scheme, for example, "S82a N" indicates that S at position 82a (also called 82A) was mutated to N according to the Kabat numbering scheme. | | | |

> huF4VH1 (huF4VH-graft)

*EVQLVQSGAEVKKPGSSVKVSCKASGGTFS*<u>RYNMH</u>*WVRQAPGQGLEWMG*<u>AIFPG</u>
<u>NGETSYNQNFKG</u>*RVTITADKSTSTAYMELSSLRSEDTAVYYCAR*<u>NSYYDYAWFTY</u>*W*
*GQGTLVTVSS*

SEQ ID NO: 55

> huF4VH2

*EVQLVQSGAEVKKPGSSVKVSCKASG**D**TF**P**<u>RYNMH</u>WVRQAPGQGLEWMG*<u>AIFPG</u>
<u>NGETSYNQNFKG</u>*RVTITADKSTSTAYMELSSLRSEDTAVYYCAR*<u>NSYYDYAWFTY</u>*W*
*GQGTLVTVSS*

SEQ ID NO: 56

> huF4VH3

*EVQLVQSGAEVKKPGSSVKVSCKASG**D**TF**P**<u>RYNMH</u>WVRQAPGQGLEWMG*<u>AIFPG</u>
<u>NGETSYNQNFKG</u>*RVT**L**TADKSTSTAYMELSSLRSEDTAVYYCAR*<u>NSYYDYAWFTY</u>*W*
*GQGTLVTVSS*

SEQ ID NO: 57

> huF4VH4

*EVQLVQSGAEVKKPGSSVKVSCKASG**D**TF**P**<u>RYNMH</u>W**LK**QAPGQGLEWMG*<u>AIFPG</u>
<u>NGETSYNQNFKG</u>*RVT**L**TADKSTSTAYMELSSLRSEDTAVYYCAR*<u>NSYYDYAWFTY</u>*W*
*GQGTLVTVSS*

SEQ ID NO: 58

> huF4VH5

*EVQLVQSGAEVKKPGSSVKVSCKASG**D**TF**P**<u>RYNMH</u>W**LK**QAPGQGLEWMG*<u>AIFPG</u>
<u>NGETSYNQNFKG</u>**K**VT**L**TADKSTSTAYMEL**N**SLRSEDTAVYYCAR*<u>NSYYDYAWFTY</u>*W*
*GQGTLVTVSS*

SEQ ID NO: 59

> huF4VH6

*EVQLVQSGAEVKKPGSSVKVSCKASG**D**TF**P**<u>RYNMH</u>W**LK**QAPGQGLEW**I**G*<u>AIFPGN</u>
<u>GETSYNQNFKG</u>**KA**T**L**TADKSTSTAYMEL**N**SLRSEDTAVYYCAR*<u>NSYYDYAWFTY</u>*W*
*GQGTLVTVSS*

SEQ ID NO: 60

> huF4VL1 (huF4VL-graft)

*DIVMTQSPDSLAVSLGERATINC*<u>RASKSVSSSGYSFMH</u>*WYQQKPGQPPKLLIY*<u>LAS</u> <u>NLES</u>*GVPDRFSGSGSGTDFTLTISSLQAEDVAVYYC*<u>QHSREFPPT</u>*FGQGTKLEIK*

SEQ ID NO: 61

> huF4VL2

*DIVMTQSPDSLAVSLGERATINC*<u>RASKSVSSSGYSFMH</u>*WYQQKPGQPPKLLIS*<u>LAS</u> <u>NLES</u>*GVPDRFSGSGSGTDFTLTISSLQAEDVAVYYC*<u>QHSREFPPT</u>*FGQGTKLEIK*

SEQ ID NO: 62

In addition, individual amino acids of CDR portions of the light chain variable region and the heavy chain variable region were modified, wherein the amino acid sequence of HCDR2 was modified from the original <u>AIFPGNGETSYN-QNFKG</u> (SEQ ID NO: 22) to: AIFPG**T**GETSYNQNFKG (set forth in SEQ ID NO: 71), and the amino acid sequence of LCDR2 was modified from the original <u>LASNLES</u> (SEQ ID NO: 13) to: LA**D**NLES (set forth in SEQ ID NO: 43).

> huF4VH1-1

*EVQLVQSGAEVKKPGSSVKVSCKASGGTFS*<u>RYNMH</u>*WVRQAPGQGLEWMG*<u>AIFPG**T**</u> <u>GETSYNQNFKG</u>*RVTITADKSTSTAYMELSSLRSEDTAVYYCAR*<u>NSYYDYAWFTY</u>*WG QGTLVTVSS*

SEQ ID NO: 63

> huF4VH2-1

*EVQLVQSGAEVKKPGSSVKVSCKASG**D**TF**P**RYNMH*WVRQAPGQGLEWMG*AIFPG **T**GETSYNQNFKG*RVTITADKSTSTAYMELSSLRSEDTAVYYCAR*<u>NSYYDYAWFTY</u>*W GQGTLVTVSS*

SEQ ID NO: 64

> huF4VH3-1

*EVQLVQSGAEVKKPGSSVKVSCKASG**D**TF**P**RYNMH*WVRQAPGQGLEWMG*AIFPG **T**GETSYNQNFKG*RVT**L**TADKSTSTAYMELSSLRSEDTAVYYCAR*<u>NSYYDYAWFTY</u>*W GQGTLVTVSS*

SEQ ID NO: 65

> huF4VH4-1

*EVQLVQSGAEVKKPGSSVKVSCKASGD TF P*<u>RYNMH</u>*W LK QAPGQGLEWMG*<u>AIFPG</u>
<u>TGETSYNQNFKG</u>*RVTL TADKSTSTAYMELSSLRSEDTAVYYCAR*<u>NSYYDYAWFTY</u>*W
GQGTLVTVSS*

<div align="right">SEQ ID NO: 66</div>

> huF4VH5-1

*EVQLVQSGAEVKKPGSSVKVSCKASGD TF P*<u>RYNMH</u>*W LK QAPGQGLEWMG*<u>AIFPG</u>
<u>TGETSYNQNFKG</u>*KVTL TADKSTSTAYMELNSLRSEDTAVYYCAR*<u>NSYYDYAWFTY</u>*W
GQGTLVTVSS*

<div align="right">SEQ ID NO: 67</div>

> huF4VH6-1

*EVQLVQSGAEVKKPGSSVKVSCKASGD TF P*<u>RYNMH</u>*W LK QAPGQGLEW IG*<u>AIFPGT</u>
<u>GETSYNQNFKG</u>*KA TL TADKSTSTAYMELNSLRSEDTAVYYCAR*<u>NSYYDYAWFTY</u>*W
GQGTLVTVSS*

<div align="right">SEQ ID NO: 68</div>

> huF4VL1-1

*DIVMTQSPDSLAVSLGERATINC*<u>RASKSVSSSGYSFMH</u>*WYQQKPGQPPKLLIY*<u>LAD</u>
<u>NLES</u>*GVPDRFSGSGSGTDFTLTISSLQAEDVAVYYC*<u>QHSREFPPT</u>*FGQGTKLEIK*

<div align="right">SEQ ID NO: 69</div>

> huF4VL2-1

*DIVMTQSPDSLAVSLGERATINC*<u>RASKSVSSSGYSFMH</u>*WYQQKPGQPPKLLIS*<u>LAD</u>
<u>NLES</u>*GVPDRFSGSGSGTDFTLTISSLQAEDVAVYYC*<u>QHSREFPPT</u>*FGQGTKLEIK*

<div align="center">SEQ ID NO: 70</div>

In the above sequences, the regions are arranged in the following order: FR1-CDR1-FR2-CDR2-FR3-CDR3-FR4, the italicized part is an FR sequence, and the underlined part is a CDR sequence determined according to the Kabat numbering scheme.

### 4. Construction and expression of IgG1 form of anti-CD70 humanized antibodies

Primers were designed for constructing VH/VK gene fragments of the antibodies by PCR, which were subjected to homologous recombination with an expression vector pTT5 (with a signal peptide and a constant region gene (CH1-FC/CL) fragment, constructed in the laboratory), thus constructing an expression vector VH-CH1-FC-pTT5/VK-CL-pTT5 for full-length antibodies. The heavy chain constant region of the antibody may be selected from the group consisting of the heavy chain constant regions of human IgG1, IgG2, IgG3, IgG4 and variants thereof, and the light chain constant region of the antibody may be selected from the group consisting of the light chain constant regions of human x and λ chains and variants thereof. Illustratively, in the following examples, the heavy chain constant region of the antibody is selected from the heavy chain constant region of human IgG1 set forth in SEQ ID NO: 72, and the light chain constant region of the antibody is selected from the human light chain constant region set forth in SEQ ID NO: 73.

Heavy chain constant region sequence of human IgG1:

ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC
PPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQKSL
SLSPGK                                                         SEQ ID NO: 72

Human light chain constant region sequence:

RTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC

SEQ ID NO: 73

The carboxyl terminus of the heavy chain variable region of the above murine antibodies B1, B7 and F4 obtained by screening was linked to the amino terminus of the human heavy chain constant region set forth in SEQ ID NO: 72, meanwhile, the carboxyl terminus of the light chain variable region of the murine antibodies was linked to the amino terminus of the human light chain constant region set forth in SEQ ID NO: 73, so that corresponding chimeric antibodies can be obtained. The chimeric antibodies of B 1, B7 and F4 were expressed as CHB1, CHB7 and CHF4, respectively.

The carboxyl terminus of the heavy chain variable region of the above humanized antibodies of B 1, B7 and F4 constructed was linked to the amino terminus of the human heavy chain constant region set forth in SEQ ID NO: 72 to form a full-length heavy chain of the antibodies, and the carboxyl terminus of the light chain variable region of the humanized antibodies was linked to the amino terminus of the human heavy chain constant region set forth in SEQ ID NO: 73 to form a full-length light chain of the antibodies, so that the humanized antibodies shown in Tables 10-12 below can be obtained:

Table 10. Humanized antibodies of B1

| Variable region | huB1VL1-1 | huB 1VL2-1 | huB1VL1 | huB 1 VL2 |
|---|---|---|---|---|
| huB1VH1 | huB1001 | huB1015 | huB1025 | huB1037 |
| huB1VH2 | huB1002 | huB1016 | huB1026 | huB1038 |
| huB1VH3 | huB1003 | huB1017 | huB1027 | huB1039 |
| huB1VH4 | huB1004 | huB1018 | huB1028 | huB1040 |
| huB1VH5 | huB1005 | huB1019 | huB1029 | huB1041 |
| huB1VH6 | huB1006 | huB1007 | huB1030 | huB1042 |
| huB1VH1-1 | huB1009 | huB1008 | huB1031 | huB1043 |
| huB1VH2-1 | huB1010 | huB1020 | huB1032 | huB1044 |
| huB1VH3-1 | huB1011 | huB1021 | huB1033 | huB1045 |
| huB1VH4-1 | huB1012 | huB1022 | huB1034 | huB1046 |
| huB1VH5-1 | huB1013 | huB1023 | huB1035 | huB1047 |

(continued)

| Variable region | huB1VL1-1 | huB 1VL2-1 | huB1VL1 | huB 1 VL2 |
|---|---|---|---|---|
| huB1VH6-1 | huB1014 | huB1024 | huB1036 | huB1048 |

Note: in the table, "huB 1001" indicates a humanized antibody in which the heavy chain variable region is huB1VH1 (SEQ ID NO: 26), the light chain variable region is huB1VL1-1 (SEQ ID NO: 40), and the heavy chain constant region is set forth in SEQ ID NO: 72, the light chain constant region is set forth in SEQ ID NO: 73, and so on for others.

Table 11. Humanized antibodies of B7

| Variable region | huB7VL 1 | huB7VL2 | huB7VL3 | huB7VL4 |
|---|---|---|---|---|
| huB7VH1 | huB7001 | huB7007 | huB7013 | huB7019 |
| huB7VH1-1 | huB7002 | huB7008 | huB7014 | huB7020 |
| huB7VH2 | huB7003 | huB7009 | huB7015 | huB7021 |
| huB7VH2-1 | huB7004 | huB7010 | huB7016 | huB7022 |
| huB7VH3 | huB7005 | huB7011 | huB7017 | huB7023 |
| huB7VH3-1 | huB7006 | huB7012 | huB7018 | huB7024 |

Note: in the table, "huB7001" indicates a humanized antibody in which the heavy chain variable region is huB7VH1 (SEQ ID NO: 44), the light chain variable region is huB7VL 1 (SEQ ID NO: 47), and the heavy chain constant region is set forth in SEQ ID NO: 72, the light chain constant region is set forth in SEQ ID NO: 73, and so on for others.

Table 12. Humanized antibodies of F4

| Variable region | huF4VL1-1 | huF4VL2-1 | huF4VL 1 | huF4VL2 |
|---|---|---|---|---|
| huF4VH1 | huF4001 | huF4014 | huF4025 | huF4037 |
| huF4VH2 | huF4002 | huF4015 | huF4026 | huF4038 |
| huF4VH3 | huF4003 | huF4016 | huF4027 | huF4039 |
| huF4VH4 | huF4004 | huF4017 | huF4028 | huF4040 |
| huF4VH5 | huF4005 | huF4018 | huF4029 | huF4041 |
| huF4VH6 | huF4006 | huF4019 | huF4030 | huF4042 |
| huF4VH1-1 | huF4007 | huF4020 | huF4031 | huF4043 |
| huF4VH2-1 | huF4008 | huF4021 | huF4032 | huF4044 |
| huF4VH3-1 | huF4009 | huF4011 | huF4033 | huF4045 |
| huF4VH4-1 | huF4010 | huF4022 | huF4034 | huF4046 |
| huF4VH5-1 | huF4012 | huF4023 | huF4035 | huF4047 |
| huF4VH6-1 | huF4013 | huF4024 | huF4036 | huF4048 |

Note: in the table, "huF4001" indicates a humanized antibody in which the heavy chain variable region is huF4VH1 (SEQ ID NO: 55), the light chain variable region is huF4VL1-1 (SEQ ID NO: 69), and the heavy chain constant region is set forth in SEQ ID NO: 72, the light chain constant region is set forth in SEQ ID NO: 73, and so on for others. Exemplary light/heavy chain full-length sequences of humanized antibodies are as follows:

Heavy chain sequence of huB1010:

EVQMVQSGAEVKKPGASVKVSCKASGYTFTTYNIHWVRQAPGQGLEWMGDI
YPGTGDASYNQKFRDRVTMTRDTSTSTVYMELSSLRSEDTAVYYCATFSRFDG
WFAYWGQGTLVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKV
DKKVEPKSCDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPGK                                    SEQ ID NO: 74

Light chain sequence of huB1010:

DIVMTQSPDSLAVSLGERATINCRASRSVSTSGYSYMHWYQQKPGQPPKLLIYL
ADNLESGVPDRFSGSGSGTDFTLTISSLQAEDVAVYYCQHSRELPYTFGGGTKVE
IKRTVAAPSVFIFPPSDEQLKSGTASVVCLLNNFYPREAKVQWKVDNALQSGNS

QESVTEQDSKDSTYSLSSTLTLSKADYEKHKVYACEVTHQGLSSPVTKSFNRGE
C                                                        SEQ ID NO: 75

Heavy chain sequence of huB7002:

EVQLVQSGSELKKPGASVKVSCKASGYTFTNYGMNWVRQAPGQGLEWMGWI
NTYTGEPTYADEFKGRFVFSLDTSVSTAYLQISSLKAEDTAVYYCARGDSFTTEIL
RNWYFDVWGQGTTVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSN
TKVDKKVEPKSCDKTHTCPPCPAPELLGGPSVFLFPPKPKDTLMISRTPEVTCVV
VDVSHEDPEVKFNWYVDGVEVHNAKTKPREEQYNSTYRVVSVLTVLHQDWL
NGKEYKCKVSNKALPAPIEKTISKAKGQPREPQVYTLPPSRDELTKNQVSLTCLV
KGFYPSDIAVEWESNGQPENNYKTTPPVLDSDGSFFLYSKLTVDKSRWQQGNVF
SCSVMHEALHNHYTQKSLSLSPGK                                SEQ ID NO: 76

Light chain sequence of huB7002:

DIQMTQSPSSLSASVGDRVTITCGASENIYGALNWYQQKPGKVPKLLIYGATNL
ADGVPSRFSGSGSGTDFTLTISSLQPEDVATYYCQNVLSTPWTFGGGTKVEIKRT
VAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC

                                                        SEQ ID NO: 77

Heavy chain sequence of huF4011:

EVQLVQSGAEVKKPGSSVKVSCKASGDTFPRYNMHWVRQAPGQGLEWMGAIF
PGTGETSYNQNFKGRVTLTADKSTSTAYMELSSLRSEDTAVYYCARNSYYDYAW
FTYWGQGTLVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVD
KKVEPKSCDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPGK                                   SEQ ID NO: 78

Light chain sequence huF4011:

DIVMTQSPDSLAVSLGERATINCRASKSVSSSGYSFMHWYQQKPGQPPKLLISLA
DNLESGVPDRFSGSGSGTDFTLTISSLQAEDVAVYYCQHSREFPPTFGQGTKLEIK
RTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC
                                                        SEQ ID NO: 79

Note: in the above full-length sequences of the antibodies, the underlined part is an antibody variable region sequence, and the non-underlined part is an antibody constant region sequence.
In addition, the light and heavy chain sequences of the positive control antibody 41D12 (see WO2012123586) in the examples of the present disclosure are as follows:

Heavy chain sequence of 41D12:

EVQLVESGGGLVQPGGSLRLSCAASGFTFSVYYMNWVRQAPGKGLEWVSDIN
NEGGTTYYADSVKGRFTISRDNSKNSLYLQMNSLRAEDTAVYYCARDAGYSNH
VPIFDSWGQGTLVTVSSASTKGPSVFPLAPSSKSTSGGTAALGCLVKDYFPEPVT
VSWNSGALTSGVHTFPAVLQSSGLYSLSSVVTVPSSSLGTQTYICNVNHKPSNTK
VDKKVEPKSCDKTHTCPPCPAPELLGGPSVFLFPPKPKDTLMISRTPEVTCVVVD
VSHEDPEVKFNWYVDGVEVHNAKTKPREEQYNSTYRVVSVLTVLHQDWLNG
KEYKCKVSNKALPAPIEKTISKAKGQPREPQVYTLPPSRDELTKNQVSLTCLVKG
FYPSDIAVEWESNGQPENNYKTTPPVLDSDGSFFLYSKLTVDKSRWQQGNVFSC
SVMHEALHNHYTQKSLSLSPGK
                                                        SEQ ID NO: 80

Light chain sequence of 41D12:

QAVVTQEPSLTVSPGGTVTLTCGLKSGSVTSDNFPTWYQQTPGQAPRLLIYNTN
TRHSGVPDRFSGSILGNKAALTITGAQADDEAEYFCALFISNPSVEFGGGTQLTV
LGQPKAAPSVTLFPPSSEELQANKATLVCLISDFYPGAVTVAWKADSSPVKAGVE
TTTPSKQSNNKYAASSYLSLTPEQWKSHRSYSCQVTHEGSTVEKTVAPTECS

SEQ ID NO: 81

Other protein sequences used in the examples of the present disclosure:
The sequence of a fusion protein of the monkey CD70 protein extracellular domain and
Human-IgG1-Fc (abbreviated as human CD70-Fc):

QRLSRAQQQLPLESLGWDIAELQLNHTGPQQDPRLYWQGGPALGRSFLHGPEL
DKGQLRIRRDGIYMVHIQVTLAICSSTSTSRHHHPTTLAVGICSPASRSISLLRLSF
HQGCTIASQRLTPLARGDTLCTNLTGTLLPSRNTDETFFGVQWVRP<u>EPKSSDKT</u>
<u>HTCPPCPAPELLGGPSVFLFPPKPKDTLMISRTPEVTCVVVDVSHEDPEVKFNWY</u>
<u>VDGVEVHNAKTKPREEQYNSTYRVVSVLTVLHQDWLNGKEYKCKVSNKALPA</u>
<u>PIEKTISKAKGQPREPQVYTLPPSRDELTKNQVSLTCLVKGFYPSDIAVEWESNG</u>
<u>QPENNYKTTPPVLDSDGSFFLYSKLTVDKSRWQQGNVFSCSVMHEALHNHYTQ</u>
<u>KSLSLSPGK</u>

SEQ ID NO: 82

Note: the underlined part is the Human-IgG1-Fc, and the non-underlined part is the monkey CD70 protein extracellular domain.

The sequence of a fusion protein of the monkey CD70 protein extracellular domain and Human-IgG1-Fc (abbreviated as monkey CD70-Fc):

SKQQQRLLEHPEPHTAELQLNLTVPRKDPTLRWGAGPALGRSFTHGPELEEGHL
RIHQDGLYRLHIQVTLANCSSPGSTLQHRATLAVGICSPAAHGISLLRGRFGQDC
TVALQRLTYLVHGDVLCTNLTLPLLPSRNADETFFGVQWICP<u>EPKSSDKTHTCPP</u>
<u>CPAPELLGGPSVFLFPPKPKDTLMISRTPEVTCVVVDVSHEDPEVKFNWYVDGV</u>
<u>EVHNAKTKPREEQYNSTYRVVSVLTVLHQDWLNGKEYKCKVSNKALPAPIEKT</u>
<u>ISKAKGQPREPQVYTLPPSRDELTKNQVSLTCLVKGFYPSDIAVEWESNGQPENN</u>
<u>YKTTPPVLDSDGSFFLYSKLTVDKSRWQQGNVFSCSVMHEALHNHYTQKSLSL</u>
<u>SPGK</u>

SEQ ID NO: 83

Note: the underlined part is the Human-IgG1-Fc, and the non-underlined part is the murine CD70 protein extracellular domain.

The sequence of a fusion protein of human CD27 and Human-IgG1-Fc (abbreviated as CD27-Fc):

ATPAPKSCPERHYWAQGKLCCQMCEPGTFLVKDCDQHRKAAQCDPCIPGVSFS
PDHHTRPHCESCRHCNSGLLVRNCTITANAECACRNGWQCRDKECTECDPLPN
PSLTARSSQALSPHPQPTHLPYVSEMLEARTAGHMQTLADFRQLPARTLSTHWPP
QRSLCSSDFIR*GGGGSGGGGS*EPKSSDKTHTCPPCPAPELLGGPSVFLFPPKPKDT
LMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRD
ELTKNQVSLTCLVKGFYPSDIAVEWESNGQPENNYKTTPPVLDSDGSFFLYSKLT
VDKSRWQQGNVFSCSVMHEALHNHYTQKSLSLSPGK                SEQ ID NO: 84

Note: the underlined part is the Human-IgG1-Fc, the non-underlined part is the human CD27, and the italicized part is the linking sequence.

## Example 5: Preparation of Non-Fucosylated Humanized Antibodies

A double-gene vector encoding amino acid sequences of the heavy chain and light chain of the CD70 antibody was constructed, and used to stably transfect CHOK1 cells (ECACC, Cat# 85051005-1VL, Lot# 12G006) with *Glul* and *Fut8* genes knocked out by electroporation using an electrotransfer (BioRad). The cells after electroporation were left to stand under an ice bath for 5 min, transferred to a preheated CD CHO medium (CAT#10743-029, Gibco) containing 1% SP4 (CAT# BESP1076E, Lonza) + 0.5% Anti-Clumping agent (CAT# 01-0057DG, Gibco) and gently mixed, and cultured in a cell shaker (36.5 °C, 6.0% $CO_2$, 120 rpm, 80% relative humidity).
24 h after the transfection, cells were counted and subjected to a minipool plating to be seeded into a 96-well cell culture plate at 2000 cells/well, followed by the addition of 100 $\mu$L of CD CHO medium containing 0.5% ACF supplement (CAT# 3820, STEMCELL Technologies) + 0.5% Anti-Clumping agent to each well to initiate minipool screening. The cell formation was observed on day 14 after plating, and the expression level of the protein in the supernatant was determined by an Octet-based method after the cell coverage rate was more than 30%. The cell populations with better expression level were transferred from the 96-well plate to a 24-well plate, cultured in a 1 mL of CD CHO culture medium containing 25 $\mu$M MSX (CAT# M5379-500MG, SIGMA) + 0.5% Anti-Clumping agent, screened for 14 days under pressurized conditions, and determined for the expression level of the protein in supernatant of the minipool cell populations. The cell populations with better expression level were transferred to a 125 mL of cell culture flask and cultured in a shaker (culture conditions in the shaker: 36.5 °C, 6.0% $CO_2$, 80% relative humidity, 120 rpm). After culturing for 2-3 days, the cells were sampled and counted, and passaged by a dilution method until the cells were in the exponential growth phase, then the cell strains were subjected to Fed-Batch culture. After about 14 days of culture, the supernatant sample was purified by Protein A. The purified sample was subjected to SEC and CE-SDS purity assays and glycoform assay. Finally, the non-fucosylated humanized antibodies were obtained. In the following test examples, non-fucosylated humanized antibodies were indicated with an "(afuc)" suffix, for example: huB7002 (afuc), huB1010 (afuc), huF4011 (afuc), and 41D12 (afuc) represented non-fucosylated antibodies of huB7002, huB1010, huF4011 and 41D12, respectively.

## Test Example 1: Experiment on Anti-CD70 Antibody Affinity by Biacore

The affinity of the chimeric and humanized antibodies of the present disclosure for the human CD70 antigen was tested using a Biacore instrument. The procedures are as follows:
The human Fc capture molecules were covalently coupled to a CM5 biosensor chip (CAT# BR-1005-30, GE) according to the method described in the instructions of the human Fc capture kit (CAT# BR-1008-39, GE) for affinity capture of the antibodies to be tested. Then, a human His-TNC-CD70 antigen (set forth in SEQ ID NO: 1) flowed through the surface of the chip, and the reaction signal was detected in real time using a Biacore T200 instrument to obtain association and dissociation curves. After the dissociation was completed for each cycle, the biochip was washed and regenerated with a regeneration solution configured in the human antibody capture kit (GE). Data were fitted using a 1:1 model. The experimental results are shown in Table 13.

Table 13. Experimental results of the affinity of anti-CD70 antibodies for human CD70 antigen

| Antibody | ka (1/Ms) | kd (1/s) | KD (M) |
|---|---|---|---|
| CHB1 | 2.26E+06 | 7.54E-05 | 3.34E-11 |
| CHF4 | 2.23E+06 | 8.37E-05 | 3.75E-11 |
| CHB7 | 5.66E+06 | 1.70E-04 | 3.00E-11 |
| huF4025 | 2.15E+06 | 8.68E-05 | 4.03E-11 |
| huF4026 | 2.44E+06 | 9.56E-05 | 3.92E-11 |
| huF4027 | 2.73E+06 | 9.13E-05 | 3.35E-11 |
| huF4028 | 2.56E+06 | 1.06E-04 | 4.12E-11 |
| huF4009 | 2.51E+06 | 1.14E-04 | 4.55E-11 |
| huF4011 | 3.12E+06 | 1.45E-04 | 4.65E-11 |
| huB1025 | 3.33E+06 | 1.54E-04 | 4.62E-11 |
| huB1026 | 1.91E+06 | 9.62E-05 | 5.03E-11 |
| huB1037 | 3.93E+06 | 1.60E-04 | 4.07E-11 |
| huB1038 | 1.80E+06 | 1.01E-04 | 5.61E-11 |
| huB1010 | 3.67E+06 | 1.30E-04 | 3.55E-11 |
| huB7002 | 6.09E+06 | 1.74E-04 | 2.86E-11 |
| huB7004 | 5.58E+06 | 1.57E-04 | 2.82E-11 |
| huB7006 | 6.41E+06 | 1.58E-04 | 2.46E-11 |
| huB7008 | 4.87E+06 | 1.69E-04 | 3.47E-11 |
| huB7010 | 4.36E+06 | 1.72E-04 | 3.96E-11 |
| huB7012 | 5.99E+06 | 1.54E-04 | 2.57E-11 |
| huB7014 | 4.92E+06 | 1.82E-04 | 3.71E-11 |
| huB7016 | 4.43E+06 | 1.85E-04 | 4.18E-11 |
| huB7018 | 4.12E+06 | 1.91E-04 | 4.64E-11 |
| huB7020 | 5.76E+06 | 1.59E-04 | 2.76E-11 |
| huB7022 | 4.41E+06 | 1.95E-04 | 4.42E-11 |
| huB7024 | 4.41E+06 | 2.05E-04 | 4.64E-11 |

The experimental results showed that the anti-CD70 antibodies of the present disclosure have high affinity for the human CD70 antigen.

**Test Example 2: Experiment on Binding of Anti-CD70 Antibodies to CD70-Positive Cells**

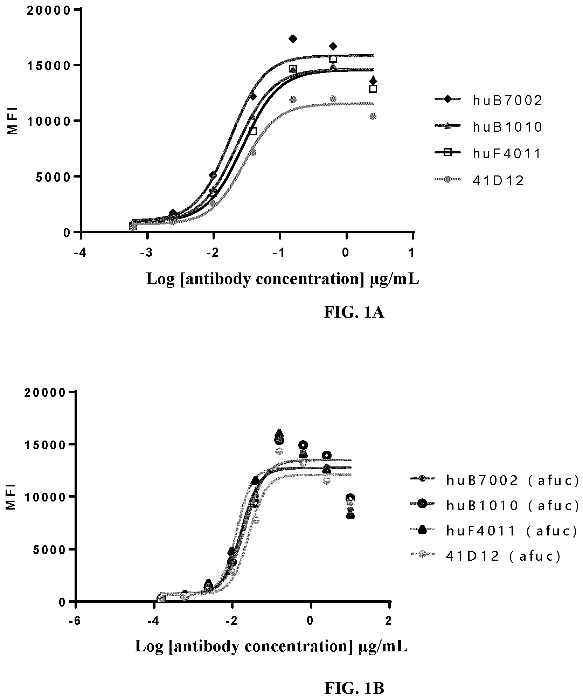

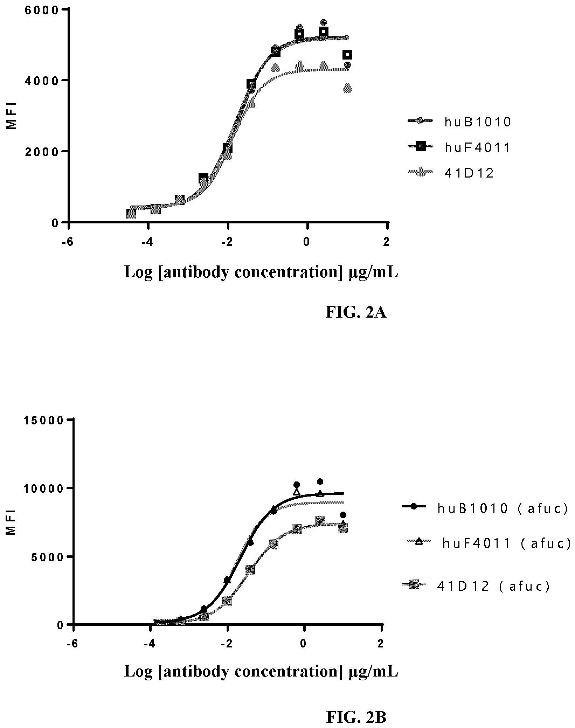

The activity of anti-CD70 antibodies for binding to CD70-positive cells was assayed by flow cytometry. The procedures are as follows:
786-O cells (ATCC, CRL-1932) or Raji cells (ATCC, CCL-86) at $1 \times 10^6$ cells/mL were blocked with 1% BSAPBS buffer, followed by the addition of diluted anti-CD70 antibody samples at different concentrations were added, and the mixture was incubated for 1 h. After the plate was washed twice, Alexa Fluor 488-goat anti-human (H + L) antibody (Invitrogen, CAT# A11013) was added, and the mixture was incubated for 1 h. After the plate was washed twice, fluorescence signals were read using a flow cytometer.The experimental results are shown in FIGs. 1A, 1B, 2A and 2B.

The experimental results showed that the anti-CD70 antibodies of the present disclosure were all able to efficiently bind to CD70-positive cells, and huB7002 and its non-fucosylated antibody huB7002 (afuc) and huB1010 and its non-fucosylated antibody huB1010 (afuc) both had stronger binding ability than the positive control.

**Test Example 3: Experiment on Binding of Anti-CD70 Antibodies to Human, Monkey and Mouse CD70 Proteins by ELISA**

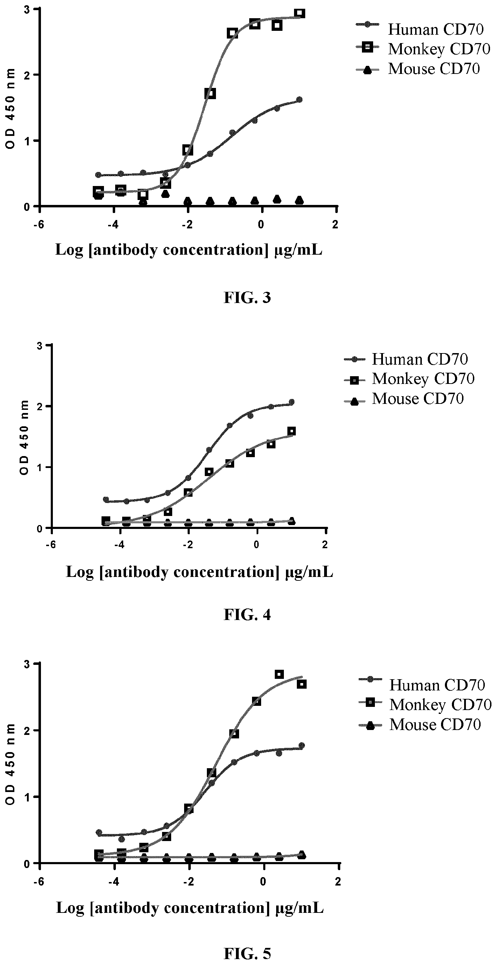

The binding ability of the anti-CD70 antibodies was determined by the amount of the antibodies bound to the CD70 antigen protein immobilized on the ELISA plate. 1 μg/mL human CD70-Fc (set forth in SEQ ID NO: 2), monkey CD70-Fc (set forth in SEQ ID NO: 82) and mouse CD70-Fc (set forth in SEQ ID NO: 83) were coated, incubated and blocked. After the plate was washed, diluted anti-CD70 antibodies at different concentrations were added, then horseradish peroxidase-goat anti-human F(ab')$_2$ antibody (Jackson, CAT# 109-036-097) was added after the plate was washed again, and a tetramethylbenzidine solution was added after the plate was washed again, finally, a stop solution was added. OD450 values were measured on a microplate reader.The experimental results are shown in FIGs 3, 4 and 5.
The experimental results showed that the anti-CD70 antibodies of the present disclosure were all able to bind to the human CD70 antigen and the monkey CD70 antigen, but not the mouse CD70 antigen.

**Test Example 4: Experiment on Blocking of Binding of CD27 to CD70-Positive Cells by Anti-CD70 Antibodies**

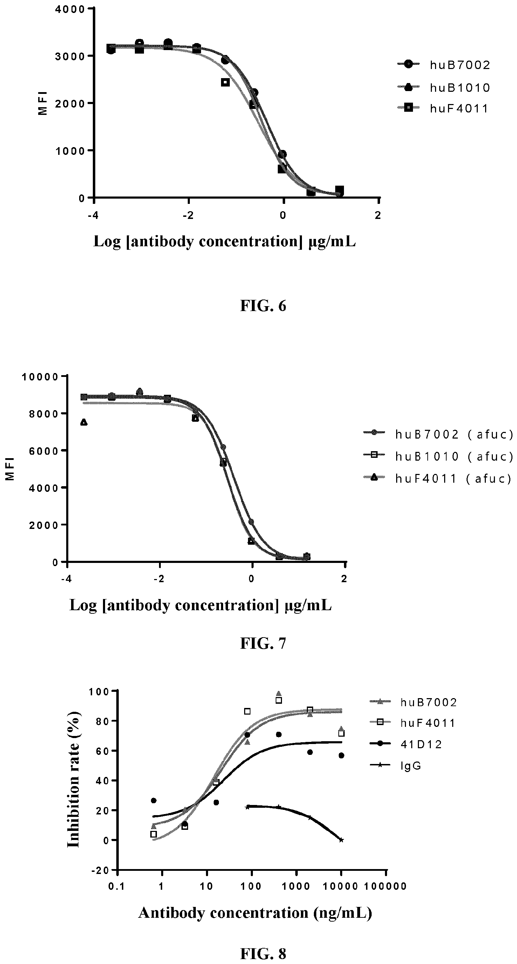

The cell-blocking activity of the anti-CD70 antibodies was assayed by flow cytometry. CHO-S cells expressing human full-length CD70 (Invitrogen, R80007) at $1\times10^6$ cells/mL were blocked with 1% BSAPBS buffer, followed by the addition with diluted anti-CD70 antibody samples at different concentrations and biotin-labeled CD27-Fc (set forth in SEQ ID NO: 84), and the mixture was incubated for 1 h. After the plate was washed twice, Alexa Fluor 488-streptavidin (Invitrogen, CAT# S11223) was added, and the mixture was incubated for 1 h. After the plate was washed twice, fluorescence signals were read using a flow cytometer. The experimental results are shown in FIGs. 6 and 7.
The experimental results showed that the anti-CD70 antibodies of the present disclosure had blocking ability against the binding of CD27 to CD70-positive cells.

**Test Example 5: Reporter System Experiment on Inhibition of IL-8 Secretion Mediated by CD70/CD27 Binding by Anti-CD70 Antibodies**

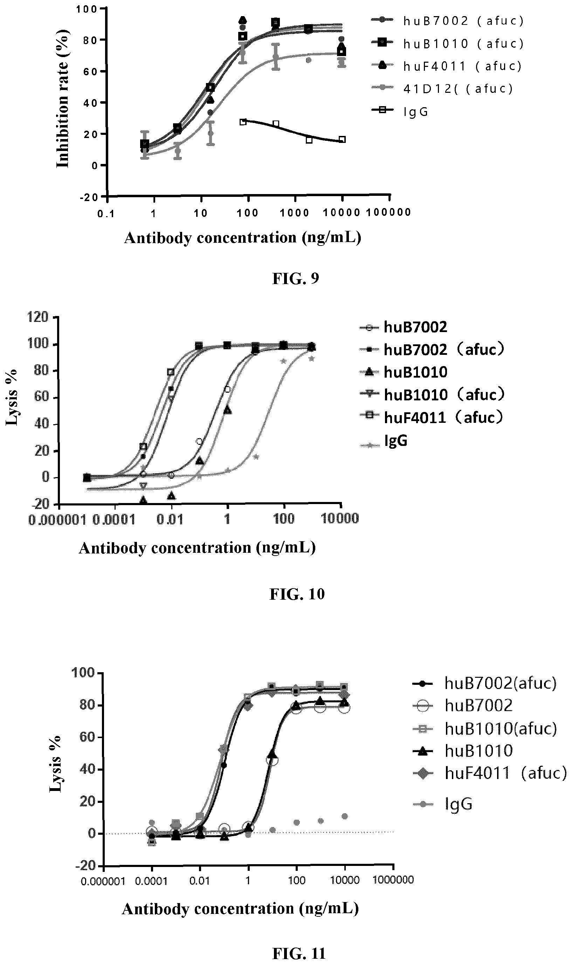

After CD70 binds to CD27, CD27 cells will secrete IL-8. In this experiment, the effect of the anti-CD70 antibodies on the level of CD70-induced CD27 signaling was assayed by determining IL-8 secretion from CD27-expressing cells.
U266 cells (ATCC, TIB-196) were collected, resuspended in RPMI1640 (Gibco, CAT# 11875119) containing 10% FBS (Gibco, CAT# 10099-141), and diluted to $1\times10^6$ cells/mL. HT1080/CD27 cells (HT1080 cells expressing human full-length CD27 (ATCC, CCL-121)) were collected, resuspended in RPMI1640 containing 10% FBS, and diluted to $2\times10^6$ cells/mL.
U266 cells and antibodies at different concentrations (the negative control was an IgG protein unrelated to the CD70 antigen) were added to a 96-well plate (Corning, CAT# 3599) at a ratio of 1:1 (50 μL of each) and incubated for 60 min (37 °C, 5% $CO_2$), followed by the addition of 50 μL of HT1080/CD27 cells.
After 18 h of incubation (37 °C, 5% $CO_2$), the supernatants were collected and 10-fold diluted before Elisa assay with an IL-8 Elisa kit (NEOBIOSCIENCE, CAT# EHC007.96), and OD450 values were measured on a microplate reader. The experimental results are shown in FIGs 8 and 9 and Table 14.

Table 14. Experiment on inhibition of IL-8 secretion mediated by CD70/CD27 binding by CD70 antibodies

| Antibody | IC50 (ng/mL) | Imax (%) |
|---|---|---|
| huB7002 (afuc) | 19.8 | 91.7 |
| huF4011 (afuc) | 13.0 | 92.3 |
| huB7002 | 21.0 | 98.7 |
| huF4011 | 14.2 | 93.8 |
| 41D12 | 23.3 | 70.9 |
| 41D12 (afuc) | 24.2 | 71.1 |
| Note: Imax is the maximum inhibition rate of the anti-CD70 antibodies for inhibiting IL-8 secretion from CD27 cells | | |

The experimental results showed that the anti-CD70 antibodies of the present disclosure had better inhibition ability to IL-8 secretion from HT1080/CD27 cells than the control antibodies 41D12 and 41D12 (afuc). This suggests that the anti-CD70 antibodies of the present disclosure are effective in inhibiting CD70-induced CD27 signaling by blocking CD70/CD27 binding.

EP 4 173 638 A1

**Test Example 6: Experiment on *In Vitro* ADCC of Anti-CD70 Antibodies for 786-O Cells**

786-O-Luc cells (luciferase-expressing 786-O cells (ATCC, CCL-86)) were collected, resuspended in Assay Buffer (an MEMα (Gibco, CAT# 12561-056) basic medium (containing 2 mM L-Glutamine) supplemented with 12.5% fetal bovine serum (Gibco, CAT# 10099-141), 12.5% horse serum (Beyotime, CAT# C0262), 0.2 mM inositol (SIGMA, CAT# I7508), 0.02 mM folic acid (SIGMA, CAT# F8758), 0.1 mM 2-mercaptoethanol (MERCK, CAT# M6250-10ML) and 200 U/mL recombinant human IL-2 (Peprotech, CAT# 200-02-100)), and diluted to $2\times10^5$ cells/mL.

NK92 cells (Nanjing Cobioer, CBP60980) were collected, resuspended in Assay buffer, and diluted to $1\times10^6$ cells/mL.

786-O-Luc and antibodies at different concentrations were added to a 96-well plate (Corning, CAT# 3903) at a ratio of 1:1 (25 μL of each) and incubated for 30 min (37 °C, 5% $CO_2$), followed by the addition of 25 μL of effector cells (NK92). After 4 h of incubation (37 °C, 5% $CO_2$), 50 μL of One-Glo reagent (Promega, CAT# E6120) was added to each well and the mixture was incubated at room temperature for 10 min. The luminescence intensity (luminescence) was measured with a microplate reader. The results are shown in FIG. 10 and Table 15.

Table 15. Experiment on *in vitro* ADCC of CD70 antibodies for 786-O cells

| Antibody | huB7002 (afuc) | huB7002 | huB1010 (afuc) | huB1010 | huF4011 (afuc) |
|---|---|---|---|---|---|
| EC50 (ng/mL) | 0.0071 | 0.3816 | 0.0028 | 0.7088 | 0.0047 |
| Maximum lysis (%) | 96.02 | 95.81 | 97.00 | 97.28 | 98.49 |

786-O-Luc cells were collected, resuspended in an RPMI 1640 medium (Gibco, CAT# 11875119) containing 10% ultra-low IgG fetal bovine serum (Gibco, CAT# 1921005PJ), and diluted to $1\times10^5$ cells/mL.

Peripheral blood mononuclear cells (PBMCs) were isolated from fresh human blood using Ficoll (GE, CAT# 17-5442-02), resuspended in an RPMI 1640 medium, and diluted to $2\times10^6$ cells/mL.

786-O-Luc and antibodies at different concentrations were added to a 96-well plate (Corning, CAT# 3903) at a ratio of 1: 1 (50 μL of each) and incubated for 30 min (37 °C, 5% $CO_2$), followed by the addition of 50 μL of effector cells (PBMCs). After 4 h of incubation (37 °C, 5% $CO_2$), 50 μL of One-Glo reagent (Promega, CAT# E6120) was added to each well and the mixture was incubated at room temperature for 10 min. The luminescence intensity was measured with a microplate reader. The experimental results are shown in FIG. 11 and Table 16.

Table 16. Experiment on *in vitro* ADCC of CD70 antibodies for 786-O cells

| | huB7002 (afuc) | huB7002 | huB1010 (afuc) | huB 1010 | huF4011 (afuc) |
|---|---|---|---|---|---|
| EC50 (ng/mL) | 0.11 | 8.32 | 0.07 | 7.21 | 0.08 |
| Maximum lysis (%) | 89.79 | 78.10 | 92.03 | 82.03 | 90.29 |

The experimental results showed that the anti-CD70 antibodies of the present disclosure had strong *in vitro* ADCC effect for 786-O cells, and the afucosylated humanized antibodies significantly improved the ADCC effect.

**Test Example 7: Experiment on *In Vitro* CDC of Anti-CD70 Antibodies on Raji Cells**

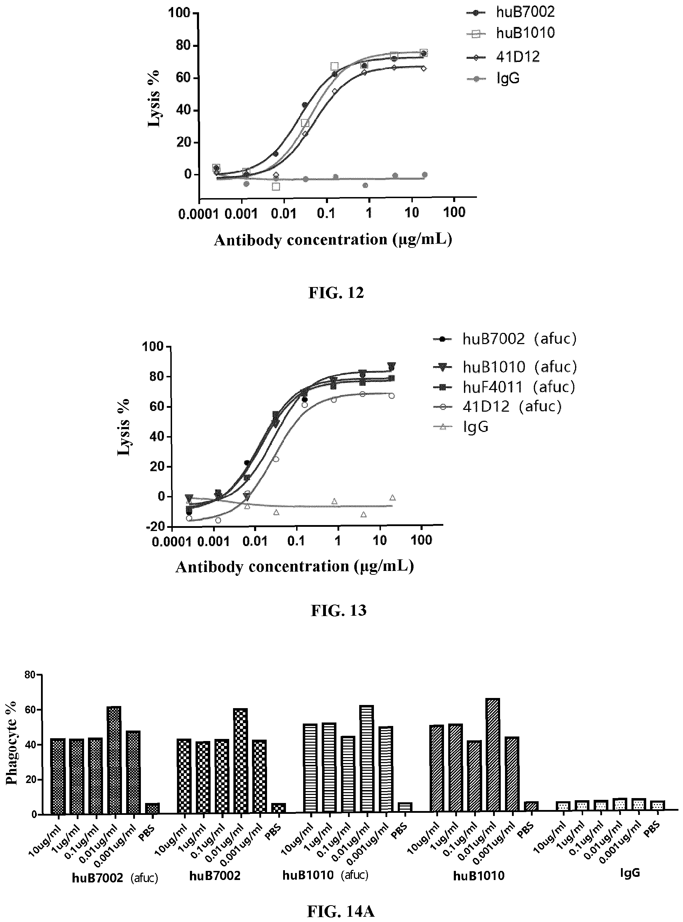

Raji cells (ATCC, CCL-86) were collected and resuspended at $1\times10^6$ cells/mL in a phenol red-free RPMI 1640 medium (Gibco, CAT# 11835-030) containing 10% ultra-low IgG fetal bovine serum (Gibco, CAT# 1921005PJ). Subsequently, the cells were seeded in a 96-well plate (Corning, CAT# 3903) at $5\times10^4$ cells/well (50 μL/well). Then, 50 μL of antibodies at different concentrations were added. After 30 min of incubation (37 °C, 5% $CO_2$), 50 μL of human serum (freshly extracted) was added to each well. After 2 h of incubation (37 °C, 5% $CO_2$), 16.6 μL of Alamar Blue reagent (Thermo, CAT# DAL1025) was added to each well, and the mixture was incubated for 20 h (37 °C, 5% $CO_2$). The emission wavelength 585 nm (excitation wavelength 570 nm) was detected with FlexStation 3 (Molecular Devices). The experimental results are shown in Table 17 and FIGs. 12 and 13.

Table 17. Experimental results *of in vitro* CDC of anti-CD70 antibodies on Raji cells

| | huB7002 (afuc) | huB1010 (afuc) | huF4011 (afuc) | 41D12 (afuc) | huB7002 | huB1010 | 41D12 |
|---|---|---|---|---|---|---|---|
| EC50 (μg/mL) | 0.013 | 0.028 | 0.014 | 0.028 | 0.024 | 0.042 | 0.052 |

(continued)

| | huB7002 (afuc) | huB1010 (afuc) | huF4011 (afuc) | 41D12 (afuc) | huB7002 | huB1010 | 41D12 |
|---|---|---|---|---|---|---|---|
| Maximum lysis (%) | 84.9 | 86.0 | 78.1 | 67.8 | 74.3 | 74.7 | 65.5 |

The experimental results showed that the anti-CD70 antibodies involved in the present disclosure had a stronger *in vitro* CDC effect on Raji cells than the control antibody 41D12.

**Test Example 8: Experiment on *In Vitro* ADCP of Anti-CD70 Antibodies on 786-O and Raji Cells**

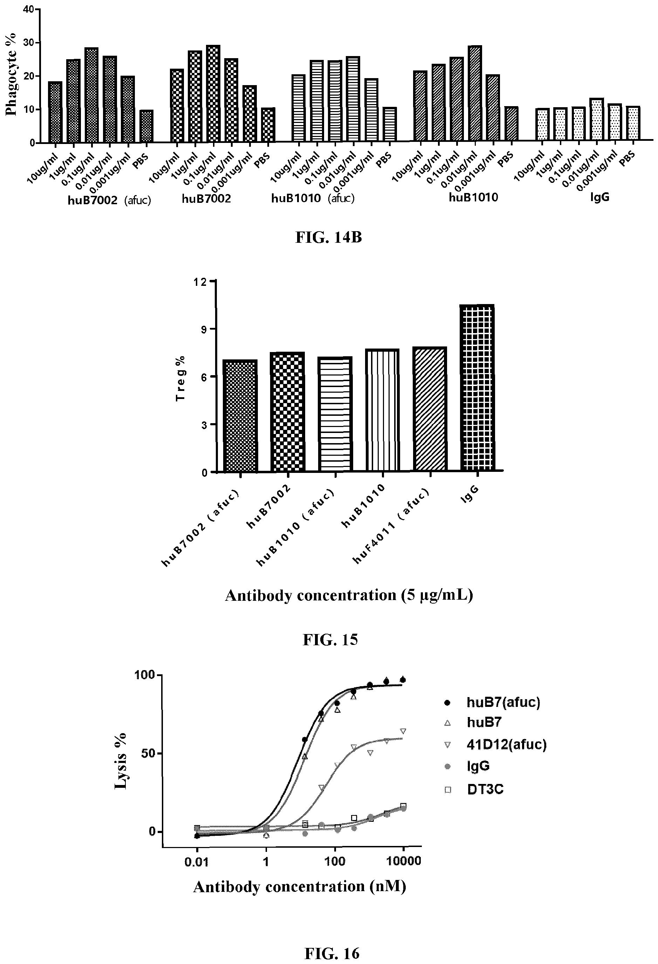

PBMCs were isolated from human blood using Ficoll (GE, CAT# 17-5442-02), from which CD14+ monocytes were sorted using CD14 magnetic beads (Miltenyi Biotec, CAT# 130-050-201). Monocytes were differentiated into macrophages by culturing for 7 days in an RPMI 1640 medium (Gibco, CAT# 11875119) containing 10% FBS (Gibco, CAT# 10091148) and 50 ng/mL M-CSF (Peprotech, CAT# 300-25). On the day of the experiment, macrophages were scraped off with a scraper and collected. 786-O cells (ATCC, CRL-1932) or Raji cells (ATCC, CCL-86) labeled with 0.1 μM CFSE (carboxyfluorescein diacetate succinimidyl ester, BD, CAT# 565082) were added at an E:T ratio of 1:4 after resuspension in 1% BSAPBS buffer, and diluted anti-CD70 antibody samples at different concentrations were added. Phagocytosis of target cells was performed for 1.5 h. After the phagocytosis was completed, the cells were washed twice with PBS, anti-human CD14 antibody conjugated to APC (Ebioscience, CAT# 17-0149-42) was added, and after 30 min of incubation on ice, the cells were washed twice with PBS. Finally, analysis was performed by flow cytometry. Phagocytosis was determined by assessing the percentage of CFSE+ positive cells on the CD14+ live cell gate. The experimental results are shown in FIGs. 14A and 14B.

The experimental results showed that the anti-CD70 antibodies of the present disclosure had good *in vitro* ADCP effects on 786-O cells and Raji cells.

**Test Example 9: Experiment *of In Vitro* Inhibition of Treg Cells by Anti-CD70 Antibodies**

U266 cells (ATCC, TIB-196) were collected, resuspended in RPMI1640 (Gibco, CAT # 11875119) containing 10% FBS (Gibco, CAT# 10099-141), and diluted to $2 \times 10^6$ cells/mL.

Peripheral blood mononuclear cells (PBMCs) were isolated from fresh human blood using Ficoll (GE, CAT# 17-5442-02), resuspended in an RPMI 1640 medium, and diluted to $2 \times 10^6$ cells/mL.

U266 cells and PBMCs were added to a 96-well plate (U-plate, corning, CAT# 3788) at a ratio of 1: 1 (50 μL of each), followed by the addition of 25 μL of antibodies at different concentrations and 25 μL of anti-CD3 (ebioscience, CAT# 16-0037-85) and anti-CD28 (ebioscience, CAT# 16-0289-85) antibodies at a final concentration of 3 μg/mL, and the mixture was incubated for 48 h (37 °C, 5% CO$_2$). The cells were collected in a 1.5 mL ep tube (Axygen, CAT# MCT-150-C-S) and washed once with 500 μL flow buffer, and PerCP-Cy™5.5 Mouse anti-CD4 (BD Pharmingen™, CAT# 560650), CD25 monoclonal antibody, PE (eBioscience, CAT# 12-0257-42) and CD127 monoclonal antibody were added. Alexa Fluor 647 (eBioscience, CAT# 51-1278-42) flow cytometry staining (4 °C, 20 min) was performed, and the proportion of Treg cells in CD4+CD25+CD127low was analyzed using a BD FACSVerseTM flow cytometer (BD Biosciences, 651154). The experimental results are shown in FIG. 15.

The experimental results showed that the anti-CD70 antibodies of the present disclosure had good *in vitro* inhibition ability to Treg cells.

**Test Example 10: Experiment on Internalization of 786-O Cells on Anti-CD70 Antibodies**

786-O-Luc cells (luciferase-expressing 786-O cells (ATCC, CRL-1932)) were collected, resuspended in an RPMI 1640 medium (Gibco, CAT# 11875119) containing 10% ultra-low IgG fetal bovine serum (Gibco, CAT# 1921005PJ), and diluted to $2 \times 10^4$ cells/mL. Subsequently, the cells were seeded in a 96-well plate (Corning, CAT# 3903) at 1000 cells/well (50 μL/well), and incubated for 16 h (37 °C, 5% CO$_2$).

DT3C (formed by fusion of Fragment A of diphtheria toxin and 3C fragment of group G Streptococcus, with a molar concentration 6 times the molar concentration of the antibody) at 4-fold concentration was prepared using an RPMI 1640 medium containing 10% ultra-low IgG fetal bovine serum. The antibody at 4-fold concentration was prepared with the same culture medium, and DT3C and the antibody were mixed according to a volume ratio of 1:1. The mixture was left to stand and incubated at room temperature for 30 min. Then, concentration gradient dilution was performed. The diluted antibodies were added to the cells according to a ratio of 1:1, at 50 μL/well, and incubated for 3 days (37

°C, 5% $CO_2$). 50 $\mu$L of CellTiter-Glo Luminescent Cell Viability Assay (Promega, CAT# G7573) was added to each well, and the mixture was incubated at room temperature for 10 min. The luminescence intensity was measured with a microplate reader. The experiment results are shown in FIG. 16 and Table 18.

Table 18. Experimental results of internalization of 786-O cells on CD70 antibodies

| Antibody | huB7002 (afuc) | huB7002 | 41D12 (afuc) |
|---|---|---|---|
| EC50 (nM) | 8.65 | 13.26 | 55.46 |
| Maximum lysis % | 96.45 | 97.18 | 63.59 |

The experimental results showed that the anti-CD70 antibodies of the present disclosure were able to be internalized by 786-O cells with a maximum lysis rate of cell internalization exceeding 96%.

**Test Example 11: Experiment on _In Vivo_ Pharmacodynamics of Anti-CD70 Antibodies in Mouse Raji Model**

**1. Experiment on _in vitro_ pharmacodynamics of anti-CD70 antibodies in mouse Raji model**

200 $\mu$L of Luc-Raji cells (luciferase-expressing Raji cells (ATCC, CCL-86)) ($1 \times 10^6$ cells) were injected into CB 17 SCID mice (purchased from Vital River) via the tail vein. 7 days after the inoculation, each mouse was intraperitoneally injected with a bioluminescent substrate (15 mg/mL) in a volume of 10 mL/kg, anesthetized by isoflurane, and photographed by a small animal imaging system 10 min after injection. The mice were randomly divided into 4 groups according to bioluminescence signals by removing those with too great and too small values of body weight and bioluminescence signal (Total Flux), including negative control IgG (an IgG protein unrelated to the CD70 antigen, administered at a dose of 30 mg/kg) group, positive control 41D12 (administered at a dose of 10 mg/kg) group, huB7002 (administered at a dose of 10 mg/kg) group, huF4011-10 (administered at a dose of 10 mg/kg) group, with 8 mice in each group. The administration of antibody by intraperitoneal injection was started on the day of grouping, 2 times per week for 2 weeks. The mice were photographed twice a week and weighed, and data were recorded. The data were recorded using Excel statistical software, wherein the bioluminescence signal value is Total Flux (p/s), and the average value was calculated as avg; the SD value was calculated as STDEV; the SEM value was calculated as STDEV/SQRT (number of animals per group). GraphPad Prism software was used for plotting, and Two-way ANOVA was used for statistical analysis of the data.

Relative tumor proliferation rate T/C (%) = (T - T0)/(C - C0) $\times$ 100, where T and C are the number of tumor photons for the treatment group and control group at the end of the experiment, respectively; and T0 and C0 are the number of tumor photons at the beginning of the experiment.

$$\text{Tumor growth inhibition (TGI) (\%)} = 100 - \text{T/C (\%)}.$$

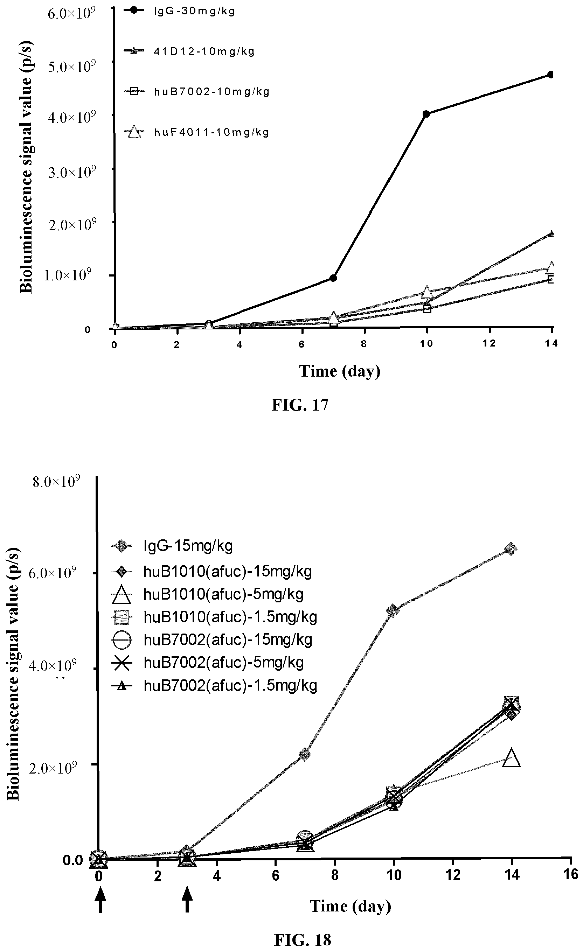

The experimental results are shown in Table 19 and FIG. 17.

Table 19. Experiment on _in vitro_ pharmacodynamics of anti-CD70 antibodies in mouse

| Raji model | | | | | | |
|---|---|---|---|---|---|---|
| Group | Total Flux (p/s) | | | | Tumor growth inhibition TGI (%) | p |
| | D0 | SEM | D14 | SEM | | (vs blank) |
| IgG-30mg/kg | 1.33E+07 | 2.00E+06 | 4.73E+09 | 6.64E+08 | - | - |
| 41D12-10mg/kg | 1.33E+07 | 2.05E+06 | 1.75E+09 | 3.81E+08 | 63% | p<0.001 |
| huB7002-10mg/kg | 1.34E+07 | 2.07E+06 | 8.92E+08 | 2.32E+08 | 81% | p<0.001 |
| huF4011-10mg/kg | 1.33E+07 | 2.25E+06 | 1.11E+09 | 4.28E+08 | 77% | p<0.001 |

The experimental results showed that compared with negative control IgG, the anti-CD70 antibody at the dose of 10 mg/kg was able to significantly inhibit the growth of tumor cells in a Luc-Raji tumor model (p < 0.001), wherein the tumor growth inhibition of huB7002 was up to 81%, and the tumor growth inhibition of positive control 41D12 was only 63%.

**2. Experiment on *in vivo* pharmacodynamics of non-fucosylated anti-CD70 antibodies in mouse Raji model**

200 μL of Luc-Raji cells (luciferase-expressing Raji cells (ATCC, CCL-86)) ($1 \times 10^6$ cells) were injected into CB 17 SCID mice (purchased from Vital River) via the tail vein. 7 days after the inoculation, each mouse was intraperitoneally injected with a bioluminescent substrate (15 mg/mL) in a volume of 10 mL/kg, anesthetized by isoflurane, and photographed by a small animal imaging system 10 min after injection. The mice were randomly grouped according to bioluminescence signals by removing those with too great and too small values of body weight and bioluminescence signal (Total Flux), with 8 mice in each group. The administration of antibody by intraperitoneal injection was started on the day of grouping (the negative control was an IgG protein unrelated to the target), for a total of 2 times (on day 1 and day 3). The mice were photographed on day 7 and day 14 and weighed, and data were recorded. The experimental results are shown in FIG. 18.

The experimental results showed that compared with the negative control IgG group, the antibodies in the non-fucosylated antibody group were all able to significantly inhibit the tumor growth ($p < 0.001$). On day 7 after the administration, the tumor growth inhibition rates of huB1010 (afuc) at the doses of 1.5 mg/kg, 5 mg/kg and 15 mg/kg were 82%, 85% and 84%, respectively, and the tumor growth inhibition rates of huB7002 (afuc) at the doses of 1.5 mg/kg, 5 mg/kg and 15 mg/kg were 87%, 85% and 82%, respectively. On day 14 after the administration, all administration groups still had high tumor inhibition activity, for example, on day 14, the tumor growth inhibition rate of huB1010 (afuc) 5 mg/kg dose group still achieved 68% compared with that of the negative control IgG group. All the dose groups showed no dose effect, suggesting that even a dose of 1.5 mg/kg is sufficient to adequately inhibit the tumor growth.

**Test Example 12: Experiment on *In Vivo* Pharmacokinetics of Anti-CD70 Antibodies**

Male SD rats (purchased from Vital River) were grouped (3 rats/group) and administered by intravenous injection at a dose of 3 mpk. For the administration group, 0.15 mL of whole blood was collected before the administration and 5 min (min for minute), 8 h (h for hour), 1 d (d for day), 2 d, 4 d, 7 d, 10 d, 14 d, 21 d and 28d after the administration. Without anticoagulation, the collected blood was left to stand at 4 °C for 30 min, and centrifuged at 1000 g for 15 min. The supernatant (serum) was placed in an EP tube and stored at -80 °C. The data obtained by plotting standard curves for different samples according to the method used in the ELISA assay on the binding of CD70 antibodies to the CD70 protein in Test Example 3 and converting the CD70 antibody concentrations in serum at different time points from the OD450 values were analyzed by Phoenix WinNonlin software, so as to calculate related pharmacokinetic parameters. The experimental results are shown in Table 20.

Table 20. Experimental results *of in vivo* pharmacokinetics of the anti-CD70 antibodies

|  | huB7002 | huB1010 | 41D12 |
|---|---|---|---|
| t1/2 (d) | 17.2 | 16.2 | 13.3 |
| Cmax (ug/mL) | 58.4 | 60.6 | 57.6 |
| AUC $_{0-t}$ (ug/mL*h) | 7568 | 9444 | 7005 |
| AUC $_{0-\infty}$ (ug/mL*h) | 10788 | 13401 | 8851 |
| CL (mL/day/kg) | 6.7 | 5.4 | 8.2 |
| MRT $_{0-\infty}$ (h) | 549.1 | 542.3 | 413.3 |

The experimental results showed that huB7002 and huB1010 both had good pharmacokinetic performance in rats: their average t1/2 values were 17.2 d and 16.2 d, respectively, which were better than that (13.3 d) of the positive control 41D12, suggesting that the antibodies have good stability in rats.

**Claims**

**1.** An anti-CD70 antibody comprising a heavy chain variable region and a light chain variable region, wherein:

    i) the heavy chain variable region comprises an HCDR1 and an HCDR3 set forth in SEQ ID NO: 15 and SEQ ID NO: 17, respectively, and an HCDR2 set forth in SEQ ID NO: 54 or SEQ ID NO: 16; and the light chain variable region comprises an LCDR1, an LCDR2 and an LCDR3 set forth in SEQ ID NO 18, SEQ ID NO 19 and SEQ ID NO 20, respectively;

ii) the heavy chain variable region comprises an HCDR1 and an HCDR3 set forth in SEQ ID NO: 9 and SEQ ID NO: 11, respectively, and an HCDR2 set forth in SEQ ID NO: 10 or SEQ ID NO: 42; and the light chain variable region comprises an LCDR1 and an LCDR3 set forth in SEQ ID NO: 12 and SEQ ID NO: 14, respectively, and an LCDR2 set forth in SEQ ID NO: 13 or SEQ ID NO: 43; or
iii) the heavy chain variable region comprises an HCDR1 and an HCDR3 set forth in SEQ ID NO: 21 and SEQ ID NO: 23, respectively, and an HCDR2 set forth in SEQ ID NO: 22 or SEQ ID NO: 71; and the light chain variable region comprises an LCDR1 and an LCDR3 set forth in SEQ ID NO: 24 and SEQ ID NO: 25, respectively, and an LCDR2 set forth in SEQ ID NO: 13 or SEQ ID NO: 43;

preferably,

i) the heavy chain variable region comprises an HCDR1, an HCDR2 and an HCDR3 set forth in SEQ ID NO: 15, SEQ ID NO: 54 and SEQ ID NO: 17, respectively; and the light chain variable region comprises an LCDR1, an LCDR2 and an LCDR3 set forth in SEQ ID NO: 18, SEQ ID NO: 19 and SEQ ID NO: 20, respectively;
ii) the heavy chain variable region comprises an HCDR1, an HCDR2 and an HCDR3 set forth in SEQ ID NO: 9, SEQ ID NO: 42 and SEQ ID NO: 11, respectively; and the light chain variable region comprises an LCDR1, an LCDR2 and an LCDR3 set forth in SEQ ID NO: 12, SEQ ID NO: 43 and SEQ ID NO: 14, respectively; or
iii) the heavy chain variable region comprises an HCDR1, an HCDR2 and an HCDR3 set forth in SEQ ID NO: 21, SEQ ID NO: 71 and SEQ ID NO: 23, respectively; and the light chain variable region comprises an LCDR1, an LCDR2 and an LCDR3 set forth in SEQ ID NO: 24, SEQ ID NO: 43 and SEQ ID NO: 25, respectively.

2. The anti-CD70 antibody according to claim 1, being a murine antibody, a chimeric antibody or a humanized antibody.

3. The anti-CD70 antibody according to claim 1 or 2, being a humanized antibody, wherein the humanized antibody comprises a framework region or a framework region variant of a human antibody, wherein the framework region variant has up to 11 amino acid back mutations relative to a light chain framework region and/or a heavy chain framework region of the human antibody;
preferably, the humanized antibody comprises:

a) a heavy chain variable region comprising an HCDR1 and an HCDR3 set forth in SEQ ID NO: 15 and SEQ ID NO: 17, respectively, and an HCDR2 set forth in SEQ ID NO: 54 or SEQ ID NO: 16; and a light chain variable region comprising an LCDR1, an LCDR2 and an LCDR3 set forth in SEQ ID NO: 18, SEQ ID NO: 19 and SEQ ID NO: 20, respectively;
wherein the light chain variable region comprises a light chain framework region variant of the human antibody comprising one or more amino acid back mutations selected from the group consisting of 38R, 43S, 69R, 70Q and 71Y relative to the light chain framework region of the human antibody, and/or the heavy chain variable region comprises a heavy chain framework region variant of the human antibody comprising one or more amino acid back mutations selected from the group consisting of 2I, 24T, 46K, 72E and 82a N relative to the heavy chain framework region of the human antibody; or
b) a heavy chain variable region comprising an HCDR1 and an HCDR3 set forth in SEQ ID NO: 9 and SEQ ID NO: 11, respectively, and an HCDR2 set forth in SEQ ID NO: 10 or SEQ ID NO: 42; and a light chain variable region comprising an LCDR1 and an LCDR3 set forth in SEQ ID NO: 12 and SEQ ID NO: 14, respectively, and an LCDR2 set forth in SEQ ID NO: 13 or SEQ ID NO: 43;
wherein the heavy chain variable region comprises a heavy chain framework region variant of the human antibody comprising one or more amino acid back mutations selected from the group consisting of 4M, 37I, 38K, 48I, 67A, 69L, 71A, 73R, 78A, 80L and 94T relative to the heavy chain framework region of the human antibody; and/or the light chain variable region comprises a light chain framework region variant of the human antibody comprising one or more amino acid back mutations selected from the group consisting of 5S and 70N relative to the light chain framework region of the human antibody; or
c) a heavy chain variable region comprising an HCDR1 and an HCDR3 set forth in SEQ ID NO: 21 and SEQ ID NO: 23, respectively, and an HCDR2 set forth in SEQ ID NO: 22 or SEQ ID NO: 71; and a light chain variable region comprising an LCDR1 and an LCDR3 set forth in SEQ ID NO: 24 and SEQ ID NO: 25, respectively, and an LCDR2 set forth in SEQ ID NO: 13 or SEQ ID NO: 43;
wherein the heavy chain variable region comprises a heavy chain framework region variant of the human antibody comprising one or more amino acid back mutations selected from the group consisting of 27D, 30P, 37L, 38K, 48I, 66K, 67A, 69L and 82a N relative to the heavy chain framework region of the human antibody, and/or the light chain variable region comprises a light chain framework region variant of the human antibody comprising a 49S amino acid back mutation relative to the light chain framework region of the human antibody.

4. The anti-CD70 antibody according to claim 1 or 2, comprising a heavy chain variable region and a light chain variable region, wherein:

d) the heavy chain variable region comprises an amino acid sequence having at least 90% sequence identity to SEQ ID NO: 5, 44, 45, 46, 51, 52 or 53, and/or the light chain variable region comprises an amino acid sequence having at least 90% sequence identity to SEQ ID NO: 6, 47, 48, 49 or 50; or

e) the heavy chain variable region comprises an amino acid sequence having at least 90% sequence identity to SEQ ID NO: 3, 26, 27, 28, 29, 30, 31, 34, 35, 36, 37, 38 or 39, and/or the light chain variable region comprises an amino acid sequence having at least 90% sequence identity to SEQ ID NO: 4, 32, 33, 40, or 41; or

f) the heavy chain variable region comprises an amino acid sequence having at least 90% sequence identity to SEQ ID NO: 7, 55, 56, 57, 58, 59, 60, 63, 64, 65, 66, 67 or 68, and/or the light chain variable region comprises an amino acid sequence having at least 90% sequence identity to SEQ ID NO: 8, 61, 62, 69 or 70;

preferably, the anti-CD70 antibody comprises a heavy chain variable region and a light chain variable region, wherein:

(g) the heavy chain variable region is set forth in SEQ ID NO: 44, 45, 46, 51, 52 or 53; and the light chain variable region is set forth in SEQ ID NO: 47, 48, 49 or 50; or

h) the heavy chain variable region is set forth in SEQ ID NO: 26, 27, 28, 29, 30, 31, 34, 35, 36, 37, 38 or 39; and the light chain variable region is set forth in SEQ ID NO: 32, 33, 40 or 41; or

(i) the heavy chain variable region is set forth in SEQ ID NO: 5; and the light chain variable region is set forth in SEQ ID NO: 6; or

(j) the heavy chain variable region is set forth in SEQ ID NO: 3; and the light chain variable region is set forth in SEQ ID NO: 4; or

(k) the heavy chain variable region is set forth in SEQ ID NO: 7; and the light chain variable region is set forth in SEQ ID NO: 8; or

l) the heavy chain variable region is set forth in SEQ ID NO: 55, 56, 57, 58, 59, 60, 63, 64, 65, 66, 67 or 68; and the light chain variable region is set forth in SEQ ID NO: 61, 62, 69 or 70; and

more preferably, the anti-CD70 antibody comprises a heavy chain variable region and a light chain variable region as shown below, wherein:

(n) the heavy chain variable region is set forth in SEQ ID NO: 51; and the light chain variable region is set forth in SEQ ID NO: 47; or

(o) the heavy chain variable region is set forth in SEQ ID NO: 35; and the light chain variable region is set forth in SEQ ID NO: 40; or

(p) the heavy chain variable region is set forth in SEQ ID NO: 65; and the light chain variable region is set forth in SEQ ID NO: 70.

5. The anti-CD70 antibody according to any one of claims 1 to 4, comprising a heavy chain constant region and a light chain constant region of the antibody; wherein preferably, the heavy chain constant region is selected from the group consisting of constant regions of human IgG1, IgG2, IgG3 and IgG4 and conventional variants thereof, and the light chain constant region is selected from the group consisting of constant regions of human antibody x and λ chains and conventional variants thereof; and more preferably, the antibody comprises a heavy chain constant region set forth in SEQ ID NO: 72 and a light chain constant region set forth in SEQ ID NO: 73.

6. The anti-CD70 antibody according to any one of claims 1 to 5, comprising:

q) a heavy chain having at least 85% sequence identity to SEQ ID NO: 74, and/or a light chain having at least 85% sequence identity to SEQ ID NO: 75; or

r) a heavy chain having at least 85% sequence identity to SEQ ID NO: 76 and/or a light chain having at least 85% identity to SEQ ID NO: 77; or

s) a heavy chain having at least 85% sequence identity to SEQ ID NO: 78, and/or a light chain having at least 85% identity to SEQ ID NO: 79;

wherein preferably, the anti-CD70 antibody comprises:

t) a heavy chain set forth in SEQ ID NO: 74 and a light chain set forth in SEQ ID NO: 75; or

u) a heavy chain set forth in SEQ ID NO: 76 and a light chain set forth in SEQ ID NO: 77; or

v) a heavy chain set forth in SEQ ID NO: 78 and a light chain set forth in SEQ ID NO: 79.

7. An isolated anti-CD70 antibody, wherein the anti-CD70 antibody competes for binding to human CD70 or monkey CD70 with the anti-CD70 antibody according to any one of claims 1 to 6.

8. The anti-CD70 antibody according to any one of claims 1 to 7, being a low-fucosylated antibody; wherein preferably, the low-fucosylated antibody is an antibody with at least 80%, 85%, 90%, 95% or 100% of heavy chain not modified by fucosylation; and more preferably, the antibody is an IgG1 antibody with 100% of heavy chain not modified by fucosylation.

9. The anti-CD70 antibody according to any one of claims 1 to 8, having at least one of the following properties:

   A. binding to human CD70 with a KD value of less than $1\times10^{-8}$ M, preferably less than $1\times10^{-9}$ M, or less than $1\times10^{-10}$ M, or less than $6\times10^{-11}$ M, wherein the KD value is determined by surface plasmon resonance technology;
   B. being able to bind to both a human CD70 antigen and a monkey CD70 antigen, but not a mouse CD70 antigen;
   C. being able to inhibit CD70-induced CD27 signaling, wherein preferably, the anti-CD70 antibody has maximum percentage inhibition of greater than 72%, 90%, 91%, 93% or 98% for inhibiting IL-8 secretion from human CD27-expressing cells;
   D. having one or more of the following effector functions: antibody-dependent cellmediated cytotoxicity, complement-dependent cytotoxicity and antibody-dependent cellmediated phagocytosis for human CD70-expressing cells; or
   E. being able to be internalized by human CD70-expressing cells.

10. A nucleic acid molecule encoding the anti-CD70 antibody according to any one of claims 1 to 9.

11. A host cell comprising the nucleic acid molecule according to claim 10, wherein preferably, the host cell is a microbial, plant or animal cell host cell; and more preferably, the host cell is a host cell with *Glut* and *Fut8* genes knocked out.

12. A pharmaceutical composition comprising a therapeutically effective amount of the anti-CD70 antibody according to any one of claims 1 to 9, or the nucleic acid molecule according to claim 10, and one or more pharmaceutically acceptable carriers, diluents or excipients.

13. An immunoconjugate, comprising: the anti-CD70 antibody according to any one of claims 1 to 9 and an effector molecule, wherein the effector molecule is conjugated to the anti-CD70 antibody; preferably, the effector molecule is selected from one or more of a radioisotope, an anti-tumor agent, an immunomodulator, a biological response modifier, a lectin, a cytotoxic drug, a chromophore, a fluorophore, a chemiluminescent compound, an enzymes, and a metal ion.

14. A method for immunodetection or determination of CD70, comprising the step of making the anti-CD70 antibody according to any one of claims 1 to 9 in contact with a subject or a sample from the subject.

15. A kit comprising the anti-CD70 antibody according to any one of claims 1 to 9 or the immunoconjugate according to claim 13.

16. A method for preventing or treating a disease or disorder, comprising administering to a subject a therapeutically effective amount of the following:

   A. the anti-CD70 antibody according to any one of claims 1 to 9,
   B. the nucleic acid molecule according to claim 10,
   C. the pharmaceutical composition according to claim 12, or
   D. the immunoconjugate according to claim 13,

   wherein the disease or disorder is preferably a tumor, an autoimmune disease or an infectious disease; preferably, the disease or disorder is a disease or disorder related to CD70.

FIG. 1A

FIG. 1B

**FIG. 2A**

**FIG. 2B**

**FIG. 3**

**FIG. 4**

**FIG. 5**

FIG. 6

FIG. 7

FIG. 8

FIG. 9

FIG. 10

FIG. 11

FIG. 12

FIG. 13

FIG. 14A

FIG. 14B

Antibody concentration (5 μg/mL)

FIG. 15

FIG. 16

FIG. 17

FIG. 18

## INTERNATIONAL SEARCH REPORT

International application No.

**PCT/CN2021/102998**

**A.    CLASSIFICATION OF SUBJECT MATTER**

A61K 39/395(2006.01)i;  C07K 16/28(2006.01)i

According to International Patent Classification (IPC) or to both national classification and IPC

**B.    FIELDS SEARCHED**

Minimum documentation searched (classification system followed by classification symbols)

A61K; C07K

Documentation searched other than minimum documentation to the extent that such documents are included in the fields searched

Electronic data base consulted during the international search (name of data base and, where practicable, search terms used)

CNABS, DWPI, SIPOABS, CNTXT, WOTXT, EPTXT, USTXT, JPTXT, KRTXT, CNKI, 万方数据资源系统, PubMed, ScienceDirect, GenBank, EBI-EMBL, STN, China Patent Biological Sequence Search System: applicant/inventor, 人CD70, CD27L, TNFSF7, 猴CD70, 抗体, CD70 antibody, 岩藻糖基化, fucosylation, 人源化, humanized, +胞外+, ECD, 重链和轻链可变区, CDR, 恒定区序列检索, 免疫缀合物, 免疫偶联物, ADC

**C.    DOCUMENTS CONSIDERED TO BE RELEVANT**

| Category* | Citation of document, with indication, where appropriate, of the relevant passages | Relevant to claim No. |
|---|---|---|
| X | CN 103596979 A (ARGEN-X BV) 19 February 2014 (2014-02-19)<br>claims 16-18, embodiments 17, 19-21, table 30 | 7-16 |
| X | CN 101370830 A (MEDAREX, INC.) 18 February 2009 (2009-02-18)<br>embodiments 13-14, 17, 20-21, claims 1-59 | 7-16 |
| A | US 2019106498 A1 (DE HAARD HANS et al.) 11 April 2019 (2019-04-11)<br>entire document | 1-16 |
| A | US 2018325955 A1 (CRISPR THERAPEUTICS, AG.) 15 November 2018 (2018-11-15)<br>entire document | 1-16 |
| A | EP 2511299 A1 (SEATTLE GENETICS, INC.) 17 October 2012 (2012-10-17)<br>entire document | 1-16 |
| A | CN 110699327 A (ZHEJIANG BLUESHIELD MEDICINE CO., LTD.) 17 January 2020<br>(2020-01-17)<br>entire document | 1-16 |

☐ Further documents are listed in the continuation of Box C.       ☑ See patent family annex.

| | |
|---|---|
| *    Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **07 September 2021** | **28 September 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2021/102998** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

**INTERNATIONAL SEARCH REPORT**

International application No.

**PCT/CN2021/102998**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **16**
because they relate to subject matter not required to be searched by this Authority, namely:

[1] Claim 16 relates to a method for preventing or treating a disease or disorder, and pertains to methods for treating diseases, and thus to a method for treating diseases pursuant to PCT Rule 39.1 (iv). The present report is made based on the amendment of claim 16 to be a preparation of a drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2021/102998**

| Patent document cited in search report | | | Publication date (day/month/year) | Patent family member(s) | | | Publication date (day/month/year) |
|---|---|---|---|---|---|---|---|
| CN | 103596979 | A | 19 February 2014 | PT | 2686347 | T | 05 July 2018 |
| | | | | DK | 2686347 | T3 | 25 June 2018 |
| | | | | EP | 2686347 | B1 | 02 May 2018 |
| | | | | BR | 112013021562 | A2 | 08 November 2016 |
| | | | | SI | 2686347 | T1 | 31 August 2018 |
| | | | | US | 8834882 | B2 | 16 September 2014 |
| | | | | JP | 6261651 | B2 | 17 January 2018 |
| | | | | US | 2014141016 | A1 | 22 May 2014 |
| | | | | US | 2014235843 | A1 | 21 August 2014 |
| | | | | TR | 201807202 | T4 | 21 June 2018 |
| | | | | US | 2017369581 | A9 | 28 December 2017 |
| | | | | JP | 2014509861 | A | 24 April 2014 |
| | | | | ES | 2670874 | T3 | 01 June 2018 |
| | | | | IL | 228001 | D0 | 30 September 2013 |
| | | | | JP | 2016196471 | A | 24 November 2016 |
| | | | | WO | 2012123586 | A1 | 20 September 2012 |
| | | | | JP | 5982409 | B2 | 07 September 2016 |
| | | | | AU | 2012228194 | A1 | 12 September 2013 |
| | | | | EP | 2686347 | A1 | 22 January 2014 |
| | | | | US | 2014147450 | A1 | 29 May 2014 |
| | | | | RU | 2013146106 | A | 27 April 2015 |
| | | | | IL | 228001 | A | 28 June 2018 |
| | | | | US | 2015266963 | A1 | 24 September 2015 |
| | | | | CN | 103596979 | B | 26 January 2018 |
| | | | | AU | 2012228194 | B2 | 01 June 2017 |
| | | | | PL | 2686347 | T3 | 31 October 2018 |
| | | | | HU | E039849 | T2 | 28 February 2019 |
| | | | | CY | 1120471 | T1 | 10 July 2019 |
| | | | | RU | 2604196 | C2 | 10 December 2016 |
| | | | | US | 9765148 | B2 | 19 September 2017 |
| | | | | US | 2019270823 | A1 | 05 September 2019 |
| | | | | CA | 2828753 | A1 | 20 September 2012 |
| | | | | US | 9765149 | B2 | 19 September 2017 |
| CN | 101370830 | A | 18 February 2009 | ZA | 200802641 | A | 25 February 2009 |
| | | | | ZA | 200802641 | B | 25 February 2009 |
| US | 2019106498 | A1 | 11 April 2019 | GB | 2567613 | A | 24 April 2019 |
| | | | | JP | 2020528044 | A | 17 September 2020 |
| | | | | AU | 2018285731 | A1 | 28 November 2019 |
| | | | | IL | 271266 | D0 | 30 January 2020 |
| | | | | CN | 110730789 | A | 24 January 2020 |
| | | | | EA | 202090061 | A1 | 13 April 2020 |
| | | | | WO | 2018229303 | A1 | 20 December 2018 |
| | | | | GB | 201709677 | D0 | 02 August 2017 |
| | | | | BR | 112019026795 | A2 | 30 June 2020 |
| | | | | CA | 3063694 | A1 | 20 December 2018 |
| | | | | JO | P20190285 | A1 | 16 December 2018 |
| | | | | EP | 3638696 | A1 | 22 April 2020 |
| | | | | PH | 12019502477 | A1 | 20 July 2020 |
| | | | | KR | 20200024823 | A | 09 March 2020 |
| | | | | GB | 201712273 | D0 | 13 September 2017 |

Form PCT/ISA/210 (patent family annex) (January 2015)

INTERNATIONAL SEARCH REPORT
Information on patent family members

International application No.

**PCT/CN2021/102998**

| Patent document cited in search report | | | Publication date (day/month/year) | Patent family member(s) | | | Publication date (day/month/year) |
|---|---|---|---|---|---|---|---|
| US | 2018325955 | A1 | 15 November 2018 | US | 10736919 | B2 | 11 August 2020 |
| | | | | CO | 2019013821 | A2 | 31 January 2020 |
| | | | | PH | 12019502529 | A1 | 13 July 2020 |
| | | | | US | 2019314413 | A1 | 17 October 2019 |
| | | | | CN | 110914289 | A | 24 March 2020 |
| | | | | US | 2019365809 | A1 | 05 December 2019 |
| | | | | US | 10729725 | B2 | 04 August 2020 |
| | | | | US | 2019365808 | A1 | 05 December 2019 |
| | | | | US | 2020405764 | A1 | 31 December 2020 |
| | | | | US | 2020330518 | A1 | 22 October 2020 |
| | | | | ZA | 201906923 | B | 27 January 2021 |
| | | | | KR | 20200005596 | A | 15 January 2020 |
| | | | | US | 2021108174 | A1 | 15 April 2021 |
| | | | | US | 10857184 | B2 | 08 December 2020 |
| | | | | CA | 3062506 | A1 | 23 May 2019 |
| | | | | EP | 3621981 | A2 | 18 March 2020 |
| | | | | JP | 2020519277 | A | 02 July 2020 |
| | | | | US | 10881689 | B2 | 05 January 2021 |
| | | | | BR | 112019023608 | A2 | 26 May 2020 |
| | | | | US | 2021085718 | A1 | 25 March 2021 |
| | | | | WO | 2019097305 | A2 | 23 May 2019 |
| | | | | AU | 2018367896 | A1 | 31 October 2019 |
| | | | | WO | 2019097305 | A3 | 01 August 2019 |
| | | | | MX | 2019013514 | A | 20 January 2020 |
| | | | | US | 2019314414 | A1 | 17 October 2019 |
| EP | 2511299 | A1 | 17 October 2012 | CN | 101203241 | A | 18 June 2008 |
| | | | | US | 8067546 | B2 | 29 November 2011 |
| | | | | EP | 1871418 | A2 | 02 January 2008 |
| | | | | US | 8562987 | B2 | 22 October 2013 |
| | | | | US | 2014178936 | A1 | 26 June 2014 |
| | | | | US | 2009148942 | A1 | 11 June 2009 |
| | | | | CN | 101203241 | B | 22 February 2012 |
| | | | | EP | 1871418 | A4 | 06 January 2010 |
| | | | | CA | 2605507 | C | 28 June 2016 |
| | | | | JP | 2012205596 | A | 25 October 2012 |
| | | | | WO | 2006113909 | A2 | 26 October 2006 |
| | | | | AU | 2006236225 | B2 | 10 May 2012 |
| | | | | US | 2021002380 | A1 | 07 January 2021 |
| | | | | WO | 2006113909 | A3 | 31 January 2008 |
| | | | | ES | 2477765 | T3 | 17 July 2014 |
| | | | | CA | 2605507 | A1 | 26 October 2006 |
| | | | | US | 2017022282 | A1 | 26 January 2017 |
| | | | | EP | 1871418 | B1 | 19 March 2014 |
| | | | | DK | 1871418 | T3 | 10 June 2014 |
| | | | | US | 2012045436 | A1 | 23 February 2012 |
| | | | | US | 9428585 | B2 | 30 August 2016 |
| | | | | JP | 2008538292 | A | 23 October 2008 |
| | | | | US | 2017342157 | A1 | 30 November 2017 |
| | | | | AU | 2006236225 | A1 | 26 October 2006 |
| | | | | JP | 5122441 | B2 | 16 January 2013 |

Form PCT/ISA/210 (patent family annex) (January 2015)

**INTERNATIONAL SEARCH REPORT**
Information on patent family members

International application No.

**PCT/CN2021/102998**

| Patent document cited in search report | | | Publication date (day/month/year) | Patent family member(s) | | | Publication date (day/month/year) |
|---|---|---|---|---|---|---|---|
| | | | | AU | 2006236225 | C1 | 02 May 2013 |
| | | | | US | 9701752 | B2 | 11 July 2017 |
| CN | 110699327 | A | 17 January 2020 | CN | 110699327 | B | 13 April 2021 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012123586 A1
- WO 2006044643 A3
- WO 2007038637 A3
- WO 2017138471 A1
- US 20040110704 A
- WO 03035835 A
- EP 1176195 A
- US 20120276086 A
- US 8642292 B
- US 5500362 A
- US 5821337 A
- CN 101573384
- WO 2016127790 A1
- US 4683195 A
- WO 2012123586 A

**Non-patent literature cited in the description**

- *J. biol. chem,* 1968, vol. 243, 3558
- **KABAT, E. A. et al.** Sequences of Proteins of Immunological Interest. 1991
- **BIRD et al.** *Science,* 1988, vol. 242, 423-426
- **HUSTON et al.** *Proc. Natl. Acad. Sci USA,* 1988, vol. 85, 5879-5883
- **KABAT et al.** Sequences of Proteins of Immunological Interest. Public Health Service, National Institutes of Health, 1991
- **AL-LAZIKANI et al.** *JMB,* 1997, vol. 273, 927-948
- **LEFRANC M. P.** *Immunologist,* 1999, vol. 7, 132-136
- **LEFRANC, M. P. et al.** *Dev. Comp. Immunol.,* 2003, vol. 27, 55-77
- Epitope Mapping Protocols in Methods in Molecular Biology. 1996, vol. 66
- **STAHLI et al.** *Methods in Enzymology,* 1983, vol. 9, 242-253
- **KIRKLAND et al.** *J. Immunol.,* 1986, vol. 137, 3614-3619
- **HARLOW ; LANE.** Antibodies: A Laboratory Manual. Cold Spring Harbor Press, 1988
- **MOREL et al.** *Molec. Immunol.,* 1988, vol. 25, 7-15
- **CHEUNG et al.** *Virology,* 1990, vol. 176, 546-552
- **MOLDENHAUER et al.** *Scand. J. Immunol.,* 1990, vol. 32, 77-82
- **YAMANE-OHNUKI et al.** Establishment of FUT8 knockout Chinese hamster ovary cells: an ideal host cell line for producing completely defucosylated antibodies with enhanced antibody-dependent cellular cytotoxicity. *BIOTECHNOLOGY AND BIOENGINEERING,* 2004, 614-622
- **TARENTINO et al.** *Biochem.,* 1975, vol. 14, 5516
- **NATSUME et al.** *Drug Des. Devel. Ther.,* 2009, vol. 3, 7
- **HSE et al.** *J. Biol. Chem.,* 1997, vol. 272, 9062-9070
- **YANG et al.** *Nature Biotechnology,* 2015, vol. 33, 842-844
- **YAMANE-OHNUKI et al.** *Biotechnol. Bioeng.,* 2004, vol. 87, 614
- **CLYNES et al.** *PNAS USA,* 1998, vol. 95, 652-656
- **VAN BIJ et al.** *Journal of Hepatology,* 04 October 2010, vol. 53, 677-685
- **ROMEUF et al.** *Br J Haematol.,* March 2008, vol. 140 (6), 635-43
- Antibodies: A Laboratory Manual. Cold Spring Harbor Press
- *immunoglobulin journal,* 2001, ISBN 012441351
- **WATSON et al.** Molecular Biology of the Gene. The Benj amin/Cummings Pub. Co, 1987, 224
- **ALTSCHUL, S.F. et al.** *J. Mol. Biol.,* 1990, vol. 215, 403-410
- **GISH, W. et al.** *Nature Genet.,* 1993, vol. 3, 266-272
- **MADDEN, T.L. et al.** *Meth. Enzymol.,* 1996, vol. 266, 131-141
- **ALTSCHUL, S.F. et al.** *Nucleic Acids Res.,* 1997, vol. 25, 3389-3402
- **ZHANG, J. et al.** *Genome Res.,* 1997, vol. 7, 649-656
- **MULLIS et al.** *Cold Spring Harbor Symp. Quant. Biol.,* 1987, vol. 51, 263
- PCR TECHNOLOGY. Stockton Press, 1989
- Antibodies: A Laboratory Manual and Molecular Cloning: A Laboratory Manual. Cold Spring Harbor Laboratory